(19) Europäisches Patentamt / European Patent Office / Office européen des brevets

(11) EP 4 772 199 A1

(12) # EUROPEAN PATENT APPLICATION

(43) Date of publication:
**08.07.2026 Bulletin 2026/28**

(21) Application number: **25225667.2**

(22) Date of filing: **19.12.2025**

(51) International Patent Classification (IPC):
***A61K 51/04*** *(2006.01)* ***A61K 103/30*** *(2006.01)*
***C07C 275/00*** *(2006.01)*

(52) Cooperative Patent Classification (CPC):
**A61K 51/0402; A61K 51/0497; C07C 275/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **06.01.2025 CN 202510025633**

(71) Applicant: **Mednovo Group Co. Ltd**
**Suzhou, Jiangsu 215163 (CN)**

(72) Inventors:
- **LI, Peishang**
  **Jiangsu, 215163 (CN)**
- **KONG, Xiangsheng**
  **Jiangsu, 215163 (CN)**
- **CAI, Chenchen**
  **Jiangsu, 215163 (CN)**
- **WANG, Zhiqian**
  **Jiangsu, 215163 (CN)**
- **WANG, Xue**
  **Jiangsu, 215163 (CN)**
- **ZHANG, Hongzhang**
  **Jiangsu, 215163 (CN)**

(74) Representative: **Plougmann Vingtoft a/s**
**Strandvejen 70**
**2900 Hellerup (DK)**

(54) **PSMA-TARGETING LIGAND COMPOUND, AND CHELATE AND USE THEREOF**

(57) A prostate-specific membrane antigen (PSMA)-targeting ligand compound and a chelate thereof and use thereof are provided. The PSMA-targeting ligand compound has a structure as shown in formula I, where $R_1$, $R_2$ and $R_4$ are each independently selected from the group consisting of H and optionally substituted linear or branched C1-C5 alkyl; $R_3$ is selected from the group consisting of optionally substituted aryl and optionally substituted heteroaryl; X is a sulfur atom or an oxygen atom; Y is selected from the group consisting of a chemical bond and -NH-$(CH_2)$m-, where m is an integer selected from 0-18; and Z is a radioactive metal ion chelating group.

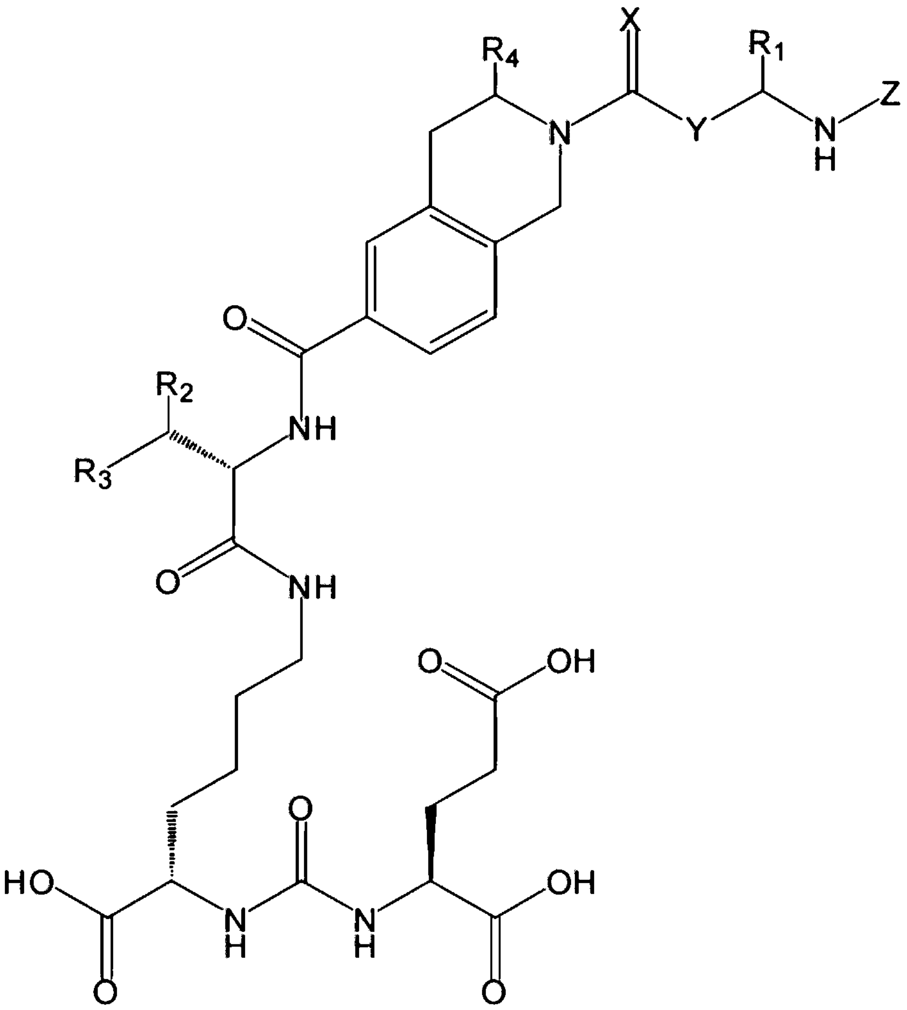

formula I

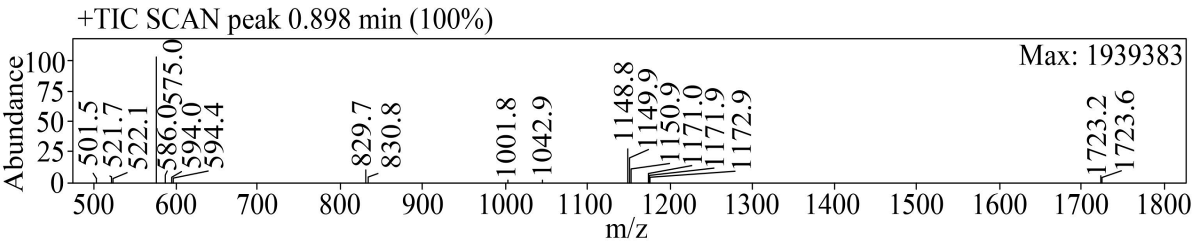


DRAWINGS

**FIG. 1**

## Description

### TECHNICAL FIELD

The present disclosure belongs to the technical field of pharmaceuticals. In particular, the present disclosure relates to a PSMA-targeting ligand compound, and a chelate thereof and use thereof.

### BACKGROUND

Prostate cancer is one of the most common malignant tumors in the male urogenital or reproductive system. In 2020, approximately 1,414,259 new cases of prostate cancer were diagnosed worldwide, accounting for 7.3% of all malignant tumors, while the disease resulted in 375,304 deaths, constituting 3.8% of total malignant tumor mortality.

Prostate-specific membrane antigen (PSMA) is a transmembrane glycoprotein that is over-expressed in most prostate cancer cells and their metastases. It has emerged as a critical therapeutic target for metastatic prostate cancer (mPCa).

PSMA-617, also known as vipivotide tetraxetan, is a highly effective inhibitor of prostate-specific membrane antigen (PSMA) with an inhibition constant (Ki) of 0.37 nM. PSMA-617 consists of three components: a targeting group, glutamate-urea-lysine; DOTA capable of chelating $^{68}$Ga or $^{177}$Lu; and a linker connecting these two components. Glutamate-urea-lysine serves as the targeting group that selectively binds to PSMA. PSMA-617, when conjugated with the radioactive isotope Lutetium-177 ($^{177}$Lu), forms $^{177}$Lu-PSMA-6l7 (see the formula below for the structure). $^{177}$Lu-PSMA-617 has been developed as a radionuclide drug conjugate (RDC) for use in PSMA-targeted radioligand therapy. This therapy localizes prostate cancer cell populations by utilizing the PSMA molecules and delivers the radiation source precisely to these cells for targeted irradiation while sparing surrounding cells. $^{177}$Lu-PSMA-617 has been approved for marketing by the FDA under the trade name Pluvicto, and is used for the treatment of PSMA-positive metastatic castration-resistant prostate cancer (mCRPC) in patients. In the clinical phase III VISION trial, $^{177}$Lu-PSMA-617 significantly improved the survival in patients with metastatic castration-resistant prostate cancer. Furthermore, PSMA-617 is also used for PSMA PET/CT imaging, a diagnostic scan used to assess PSMA expression levels, which aids in enhancing the sensitivity and accuracy of prostate cancer diagnosis.

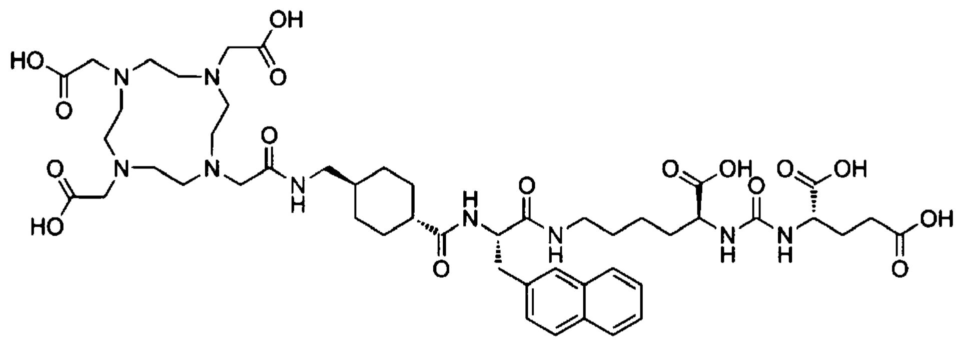

However, clinical applications have revealed deficiencies in the pharmacokinetic profile of PSMA-617, such as suboptimal parameters in terms of absorption, distribution, and metabolism, which limit the therapeutic efficacy of the radionuclide. Consequently, a pressing need remains widely recognized in the pharmaceutical field for improvements to prostate-specific membrane antigen-targeted radionuclide-conjugated ligand to better meet requirements in clinical practice.

### SUMMARY

In view of the above problems, an object of the present disclosure is to provide a PSMA-targeting ligand compound, a chelate thereof, a preparation method therefor and use thereof. The present inventors have found through researches that a new class of PSMA-targeting ligand compounds with markedly improved pharmacokinetic profiles can be obtained by modifying the chemical structure of PSMA-617 and screening for related biological activity. The ligand compounds exhibit more efficient absorption and good stability in the human body, and are fully taken up and internalized by cancerous cells. Therefore, conjugating the ligand compound with a radionuclide can yield significantly improved anti-cancer drugs or diagnostic agents with better imaging performance.

The above-mentioned object of the present disclosure is achieved by the following technical solutions provided herein:

In the first aspect, the present disclosure provides a PSMA-targeting ligand compound having a structure as shown in formula I, or a stereoisomer, a tautomer, a hydrate, a solvate, a pharmaceutically acceptable salt or ester thereof,

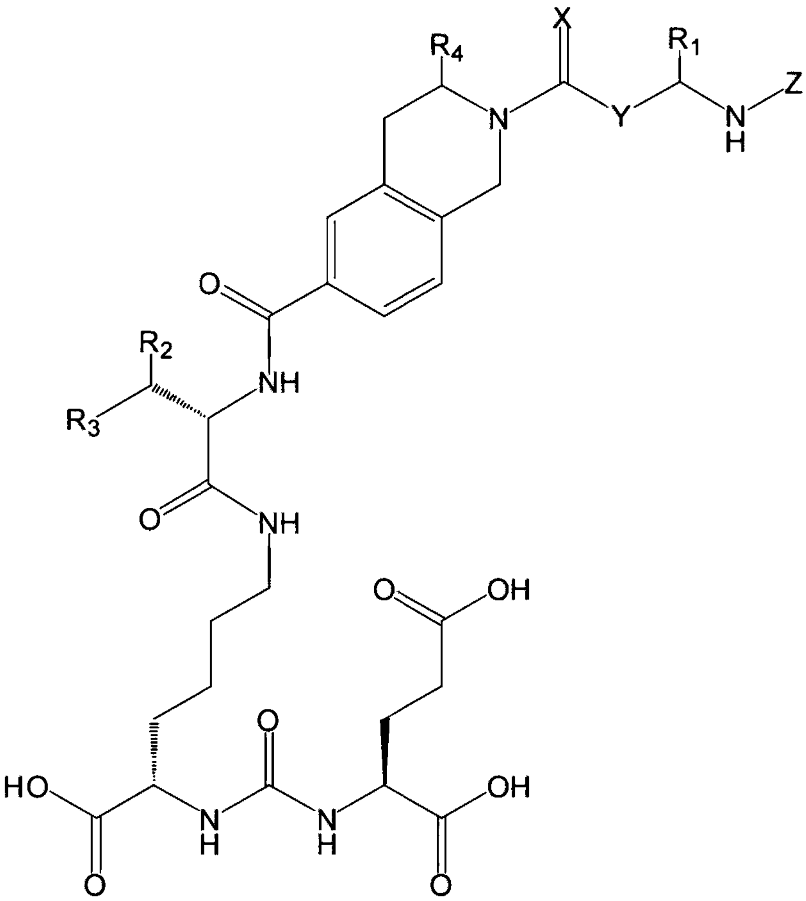

I

wherein

$R_1$, $R_2$ and $R_4$ are each independently selected from H and optionally substituted linear or branched C1-C5 alkyl, wherein a substituent in the optionally substituted linear or branched C1-C5 alkyl is selected from deuterium, carboxyl, linear or branched C1-C5 alkylacyl, linear or branched C1-C5 alkyl ester group, halogen, hydroxyl, linear or branched C1-C5 alkylthio, cyano, linear or branched C1-C5 alkoxy, amino, nitro, phenylsulfonamido, linear or branched C1-C5 alkylamido, 6- to 14-membered aryl, 5- to 14-membered heteroaryl, 6- to 14-membered aryloxy, 5- to 14-membered heteroaryloxy, linear or branched C2-C6 alkenyl, and linear or branched C2-C6 alkynyl;

$R_3$ is selected from optionally substituted 6- to 14-membered aryl and optionally substituted 5- to 14-membered heteroaryl, wherein the aryl or heteroaryl is substituted with a substituent in the optionally substituted 6- to 14-membered aryl or the optionally substituted 5- to 14-membered heteroaryl is selected from deuterium, carboxyl, halogen, linear or branched C1-C5 alkyl substituted with halogen, linear or branched C1-C5 alkylacyl, linear or branched C1-C5 alkyl ester group, hydroxyl, linear or branched C1-C5 alkylthio, cyano, linear or branched C1-C5 alkoxy, amino, nitro, phenylsulfonamido, linear or branched C1-C5 alkylamido, linear or branched C2-C6 alkenyl, and linear or branched C2-C6 alkynyl;

X is a sulfur or oxygen atom;

Y is selected from a chemical bond and $-NH-(CH_2)_m-$, wherein m is an integer selected from 0-18;

and Z is a radioactive metal ion chelating group.

According to some embodiments of the present disclosure, $R_1$ is H, carboxyl-substituted methyl or carboxyl-substituted ethyl.

According to some embodiments of the present disclosure, $R_2$ is H, methyl or ethyl.

According to some embodiments of the present disclosure, $R_3$ is selected from optionally substituted phenyl and

optionally substituted naphthyl, wherein a substituent in the optionally substituted phenyl or the optionally substituted naphthyl is selected from halogen, such as F-, Cl- or Br-, trifluoromethyl, difluoromethyl, and monofluoromethyl.

According to some embodiments of the present disclosure, $R_4$ is H, methyl or ethyl.

According to some embodiments of the present disclosure, Y is a chemical bond or $-NH-CH_2-$.

According to some embodiments of the present disclosure, m is 0, 1 or 2.

According to some embodiments of the present disclosure, Z is a radioactive metal ion chelating group formed by the following chelating agents: 1,4,7,10-tetraazacyclododecane-1,4,7,10-tetraacetic acid (DOTA), 1,4,7-triazacyclononane-1,4,7 -triacetic acid (NOTA), 1,4,7-triazacyclononane triacetic acid with amino acid modifications (NOTA-AA), diethylenetriamine pentaacetic acid (DTPA), 1,4,7-triazacyclononane-1,4-diacetic acid (NODA), 2-(4,7-bis(carboxymethyl)-1,4,7-triazonon-1-yl)glutaric acid (NODAGA), 1,4,7,10-tetraazacyclododecane-1,4,7,10-tetramethylene phosphonic acid (DOTP), 1,4,8,11-tetraazacyclotetradecane-1,4,8,11-tetraacetic acid (TETA), diacetylbis(N4-methyl-3-thiosemicarbazone) (ATSM), pyruvaldehyde bis(N4,N4-dimethylthiosemicarbazone) (PTSM), ethylenediamine tetraacetic acid (EDTA), trisodium ethylenediamine disuccinate (EC), N,N'-bis(2-hydroxybenzyl)-ethylenediamine-N,N-diacetic acid (HBED), N,N'-bis(2-hydroxybenzyl)ethylenediamine-N,N'-diacetic acid ketone (HBEDCC), monobenzylamino diacetic acid (SBAD), N,N'-bis(3-aminopropyl)ethylenediamine (BAPEN), deferoxamine mesylate (Df), N'-{5-[acetyl(hydroxyl) amino]pentyl}-N-[5-({4-[5-aminopentyl)(hydroxyl)amino]-4-oxobutano yl}amino)pentyl]-N-hydroxylsuccinamide (DFO), 1,4,7-triazacyclononane (TACN), 1,4,7-triazacyclononane-1,7-diacetic acid/1,4,7-triazacyclononane-1,4,7-triacetic acid maleimide (NO2A/NOTAM), 1,4,7,10-tetraazacyclododecane-1,7-diacetic acid (CB-DO2A), 1,4,7,10-tetraazacyclododecane (Cyclen), sodium 2,2',2"-(1,4,7,10-tetraazacyclododecane-1,4,7-triyl)triacetate (DO3A), 1,4,7,10-tetraazacyclododecane-1,4,7-triacetic acid-10-methylphosphonic acid (DO3AP), 6-hydrazinonicotinoyl succinimide ester hydrochloride (HYNIC), S-acetylmercaptoacetyltriserine (MAS3), mercaptoacetyltriglycine (MAG3), isonitrile, and derivatives thereof.

According to some embodiments of the present disclosure, $R_3$ is selected from the following groups:

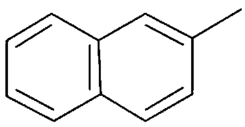 , 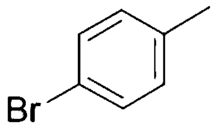 , and 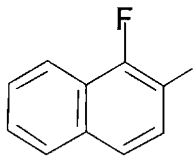 .

According to some embodiments of the present disclosure, Z is

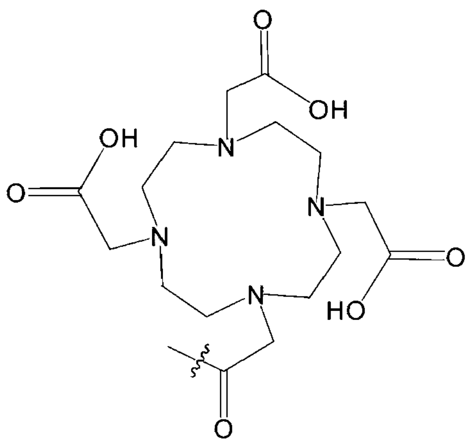 .

According to some specific embodiments of the present disclosure, the PSMA-targeting ligand compound or the stereoisomer thereof is selected from the following compounds:

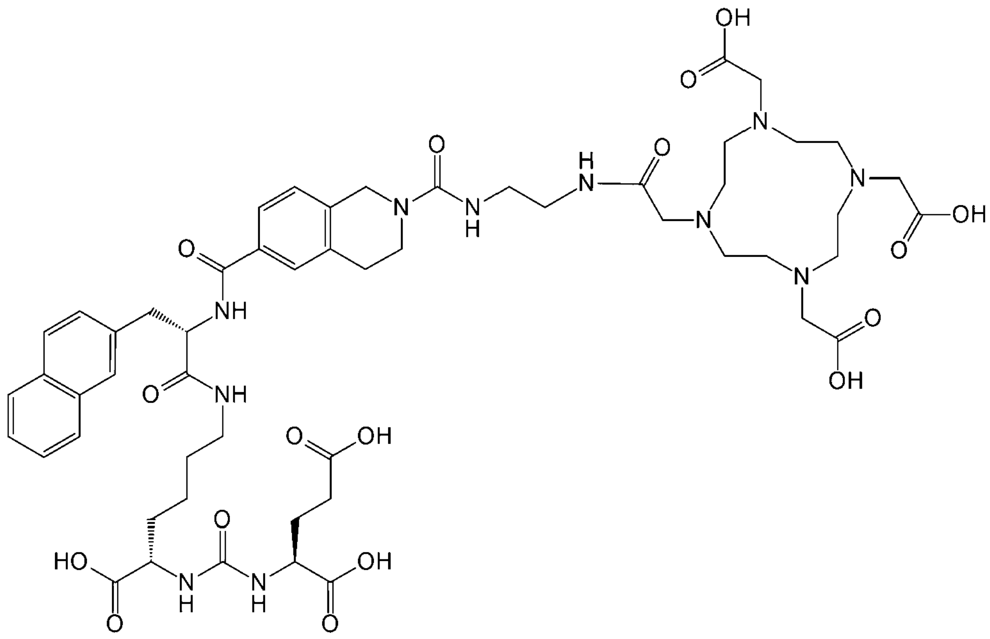

m3-12;

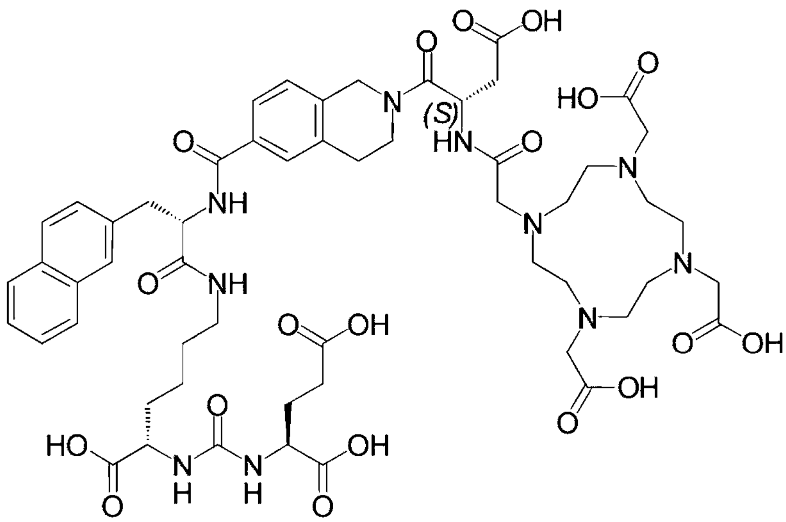

m3-18;

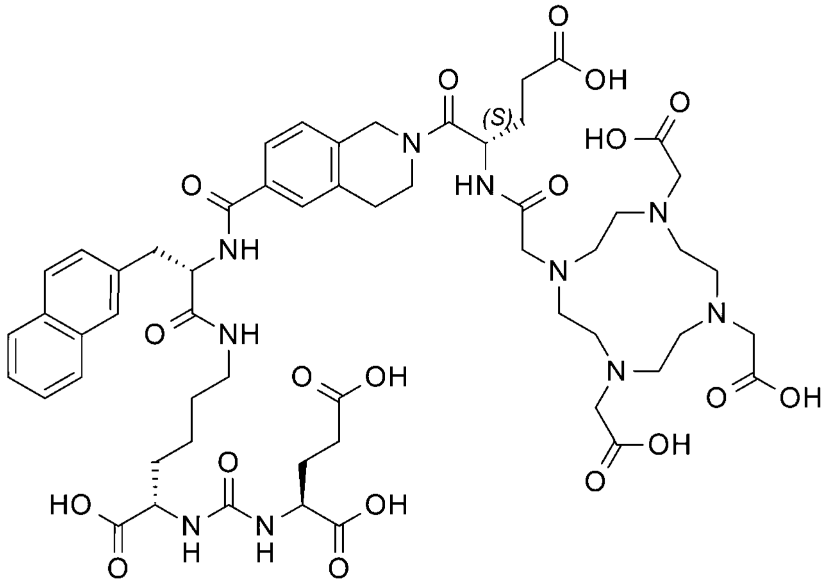

m3-19;

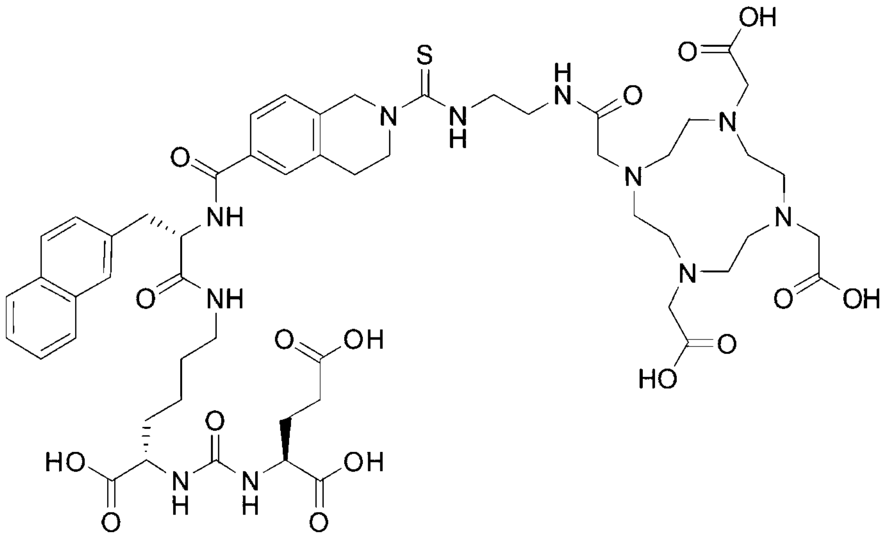

m3-12-1;

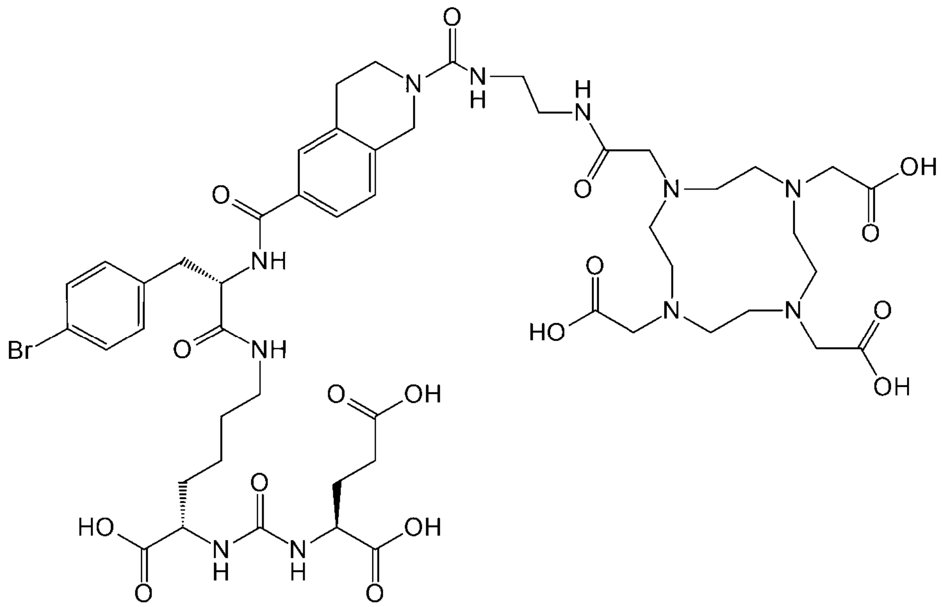

m3-12-3;

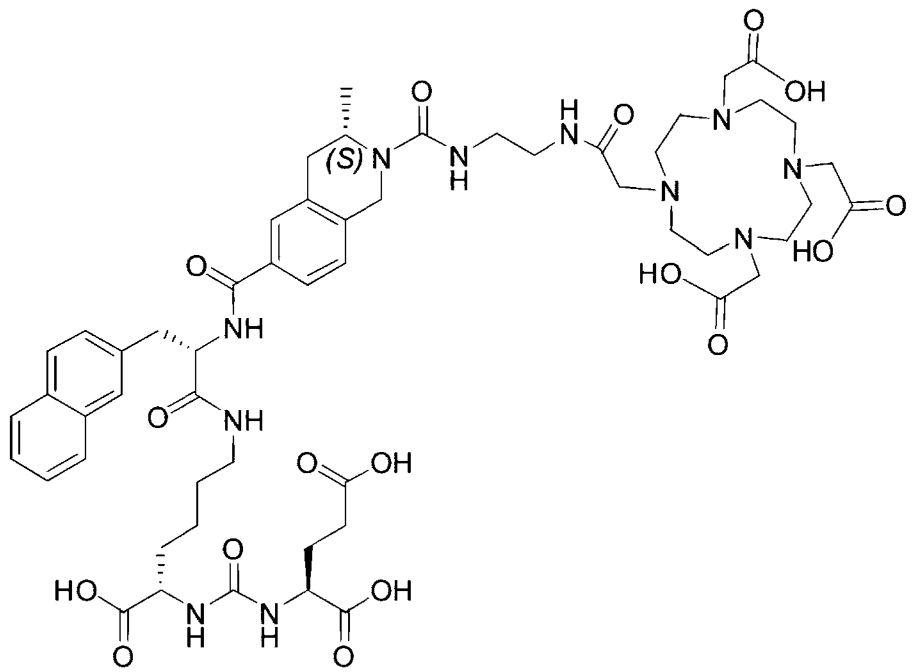

m3-12-a;

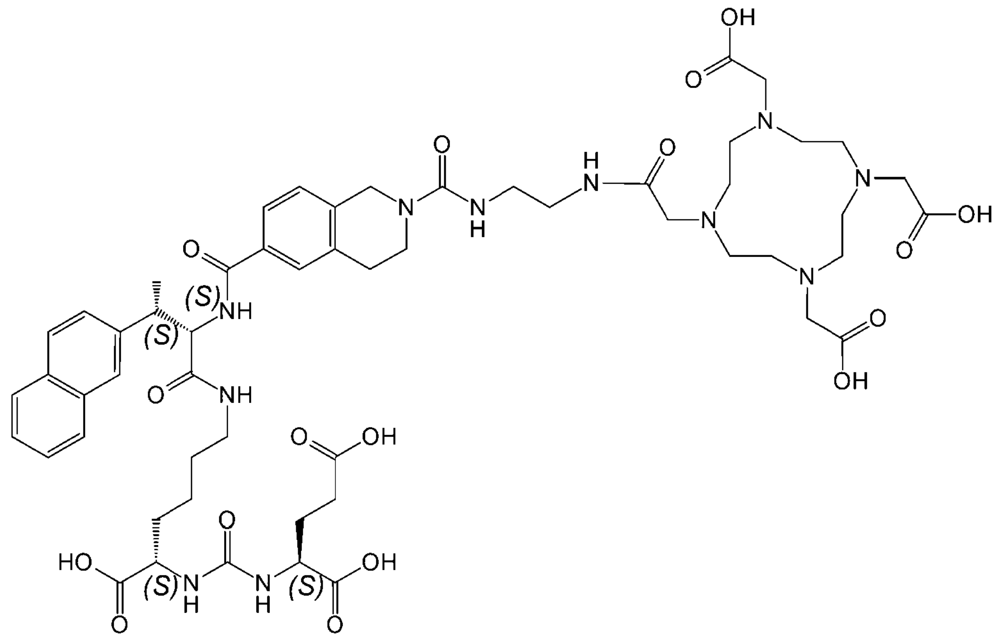

m3-12-2;

and

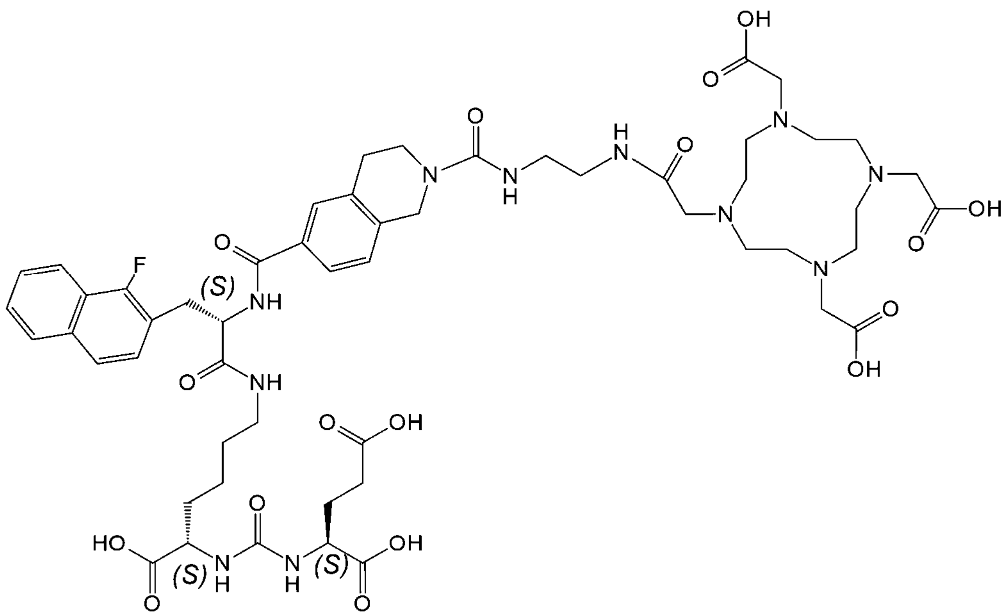

m3-12-5.

In the second aspect, the present disclosure provides a method for preparing the PSMA-targeting ligand compound or the stereoisomer, the tautomer, the hydrate, the solvate, the pharmaceutically acceptable salt or ester thereof according to the first aspect of the present disclosure, the method including:

(1) reacting a compound as shown in formula II with a compound as shown in formula III in an organic solvent containing a condensing reagent and an organic amine to produce a compound as shown in formula IV;

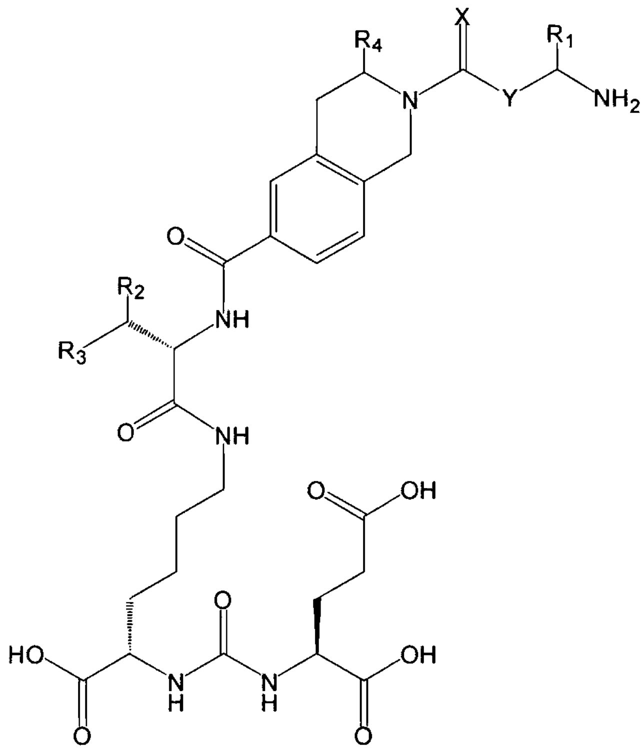

II

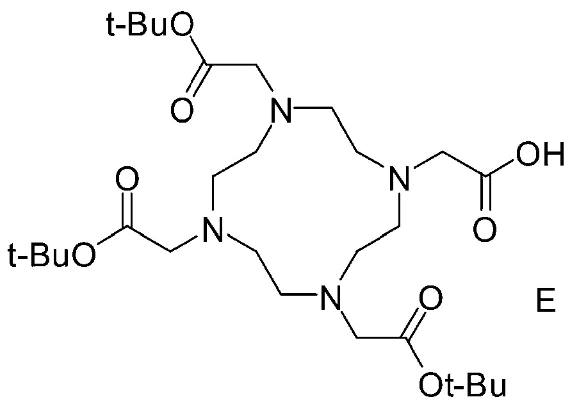

E

III

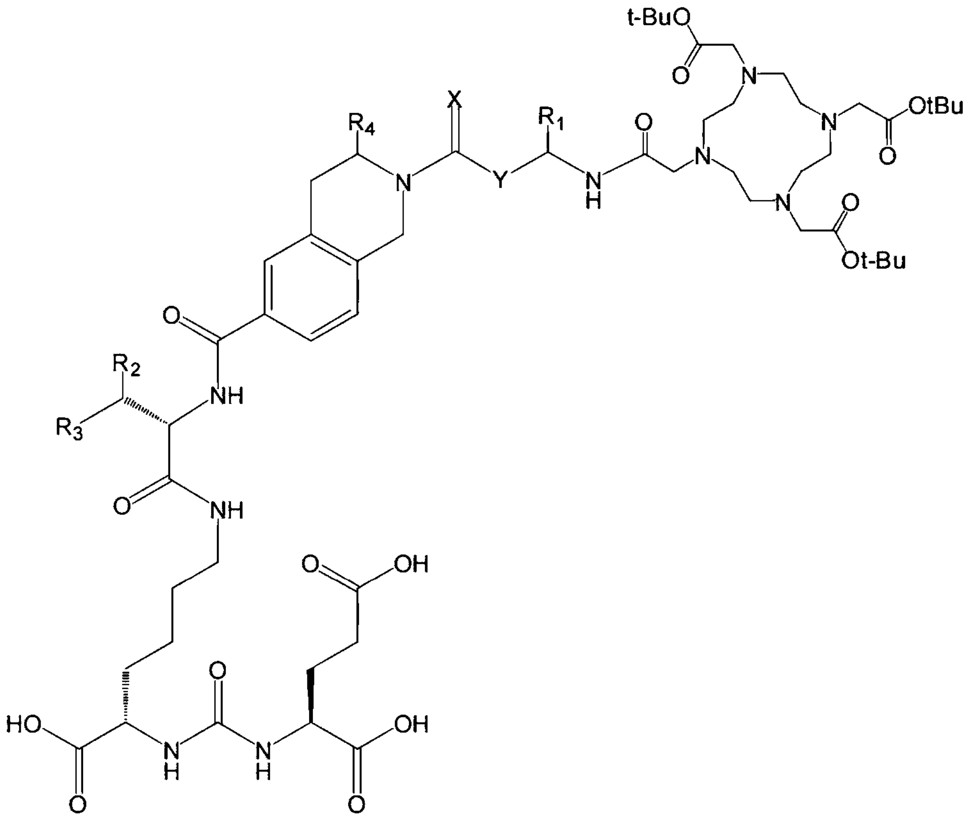

IV

(2) reacting the compound as shown in formula IV in an organic solvent containing a deprotection reagent to produce the PSMA-targeting ligand compound as shown in formula I.

According to some embodiments of the present disclosure, the condensing reagent is at least one selected from the group consisting of 2-(7-azobenzotriazole)-N,N,N',N'-tetramethyluronium hexafluorophosphate (HATU), N,N'-dicyclohexylcarbodiimide (DCC), 1-(3-dimethylaminopropyl)-3-ethylcarbodiimide (EDCI), (benzotriazol-1-yloxy)tripyrrolidylphosphonium hexafluorophosphate (ByBOP) and 1-propylphosphonic acid cyclic anhydride (T3P).

According to some embodiments of the present disclosure, the organic amine is at least one selected from the group consisting of N,N-diisopropylethylamine (DIEA) and triethylamine ($Et_3N$).

According to some embodiments of the present disclosure, the deprotection reagent is at least one selected from the group consisting of trifluoroacetic acid (TFA), a solution of hydrogen chloride in dioxane, a solution of hydrogen chloride in methanol, and a solution of hydrogen chloride in ethyl acetate.

According to some embodiments of the present disclosure, the organic solvent is at least one selected from the group consisting of N,N-dimethylformamide (DMF), tetrahydrofuran (THF) and dichloromethane (DCM).

In the third aspect, the present disclosure provides a chelate including the PSMA-targeting ligand compound or the stereoisomer, the tautomer, the hydrate, the solvate, the pharmaceutically acceptable salt or ester thereof according to the first aspect of the present disclosure, and a radioactive metal ion chelated thereto.

According to some embodiments of the present disclosure, the radioactive metal is one or more selected from the group consisting of $^{177}Lu$, $^{125}I$, $^{131}I$, $^{211}At$, $^{111}In$, $^{153}Sm$, $^{186}Re$, $^{188}Re$, $^{67}Cu$, $^{212}Pb$, $^{225}Ac$, $^{213}Bi$, $^{212}Bi$, $^{212}Pb$ and $^{68}Ga$.

According to some embodiments of the present disclosure, the radioactive metal is $^{177}Lu$.

In a fourth aspect, the present disclosure provides a pharmaceutical composition for use in the treatment of a tumor, the pharmaceutical composition including the chelate according to the third aspect of the present disclosure, and optionally, at least one pharmaceutically acceptable carrier.

In a fifth aspect, the present disclosure provides a reagent composition for use in the diagnosis of a tumor, the reagent composition including the chelate according to the third aspect of the present disclosure, and optionally, at least one diagnostically acceptable carrier.

In a sixth aspect, the present disclosure provides use of the PSMA-targeting ligand compound or the stereoisomer, the tautomer, the hydrate, the solvate, the pharmaceutically acceptable salt or ester thereof according to the first aspect of the present disclosure in preparation of a PSMA inhibitor.

Correspondingly, the present disclosure provides a method for inhibiting PSMA, including: administering an effective amount of the PSMA-targeting ligand compound or the stereoisomer, the tautomer, the hydrate, the solvate, the pharmaceutically acceptable salt or ester thereof according to the first aspect of the present disclosure to a subject.

In a seventh aspect, the present disclosure provides use of the chelate according to the third aspect of the present disclosure in preparation of a medicament for the treatment of a tumor.

Correspondingly, the present disclosure provides a method for treating a tumor, including: administering a therapeutically effective amount of the chelate according to the third aspect of the present disclosure to a subject.

In the eighth aspect, the present disclosure provides use of the chelate according to the third aspect of the present disclosure in preparation of a reagent for the diagnosis of a tumor.

According to some embodiments of the present disclosure, the reagent for the diagnosis of a tumor is a tumor imaging agent.

Correspondingly, the present disclosure provides a method for imaging a tumor, including: administering an effective amount of the chelate according to the third aspect of the present disclosure to a subject, and performing imaging.

According to some embodiments of the present disclosure, the tumor is at least one selected from the group consisting of prostate cancer, renal carcinoma, and gastric cancer.

According to some embodiments of the present disclosure, the tumor is prostate cancer. In particular, the prostate cancer is at least one selected from the group consisting of castration-resistant prostate cancer, metastatic castration-resistant prostate cancer, and PSMA-positive prostate cancer.

As used herein, the term "subject" means a human or animal. Generally, the animal is a vertebrate such as a primate, rodent, domestic animal, or game animal. Primates include chimpanzees, cynomolgus monkeys, spider monkeys, and macaques, e.g., Rhesus monkey. Rodents include mice, rats, marmots, ferrets, rabbits and hamsters. Domestic and game animals include cows, horses, pigs, deer, bison, buffalo, felines, e.g., domestic cat, and canines, e.g., dog, fox, wolf. In some embodiments, the subject is a mammal. The mammal can be a human, non-human primate, mouse, rat, dog, cat, horse, or cow, but are not limited to these examples. In addition, the methods described herein can be used to treat domesticated animals and/or pets. The term does not denote a particular age or gender. Thus, adult and newborn subjects, as well as fetuses, whether male or female, are intended to be included within the scope of this term.

As used herein, the term "administer" involves any route for delivering the active substance, i.e., the PSMA-targeting ligand compound or the stereoisomer, the tautomer, the hydrate, the solvate, the pharmaceutically acceptable salt or ester, chelate or composition thereof to a subject. In some embodiments, the active substance described herein is administered enterally to the small intestine. Routes of delivery may include non-invasive oral (through the mouth), topical (skin), transmucosal (nasal, buccal/sublingual, vaginal, ocular and rectal) and inhalation routes, as well as parenteral routes, and other methods known in the art. Parenteral administration refers to a route of delivery that is generally associated with injection, including intraorbital, infusion, intraarterial, intracarotid, intracapsular, intracardiac, intradermal, intramuscular, intraperitoneal, intrapulmonary, intraspinal, intrasternal, intrathecal, intrauterine, intravenous, subarachnoid, subcapsular, subcutaneous, transmucosal, or transtracheal routes. When administered via the parenteral route, the composition may be in the form of a solution or a suspension for infusion or for injection, or as lyophilized powders.

As used herein, the term "effective amount" refers to the amount of the active substance to decrease at least one or more symptoms of a disease or disorder, and relates to a sufficient amount of the active substance to provide the desired effect. The phrase "therapeutically effective amount" as used herein means a sufficient amount of the active substance to treat a disorder, at a reasonable benefit/risk ratio applicable to any medical treatment.

As used herein, the term "treatment" refers to therapeutic treatment, wherein the purpose thereof is to reverse, alleviate, ameliorate, inhibit, slow down or stop the progression or severity of a condition associated with, a disease or disorder. The term "treating" includes reducing or alleviating at least one adverse effect or symptom of a condition, disease or disorder. Treatment is generally "effective" if one or more symptoms or clinical markers are reduced. Alternatively, treatment is "effective" if the progression of a disease is reduced or halted. That is to say, "treatment" includes not just the improvement of symptoms or markers, but also a cessation or at least slowing of the progression or worsening of symptoms that would be expected in absence of the treatment. Beneficial or desired clinical results include, but are not limited to, alleviation of one or more symptom(s), reduction in the extent of the disease, stabilized (i.e., not worsening) state of disease, delay or slowing of disease progression, amelioration or alleviation of the disease state, and remission (whether partial or total), whether detectable or undetectable. The term "treatment" of a disease also includes achieving relief from the symptoms or adverse effect of the disease (including palliative treatment).

A therapeutically or prophylactically significant reduction in a symptom is defined as, e.g. at least about 10%, at least about 20%, at least about 30%, at least about 40%, at least about 50%, at least about 60%, at least about 70%, at least about 80%, at least about 90%, at least about 100%, at least about 125%, at least about 150% or more in a measured parameter as compared with the status of a control or non-treated subject prior to the administration of the active substance described herein. Measured or measurable parameters include clinically detectable markers of disease, for example, elevated or depressed levels of a biological marker, as well as parameters related to a clinically accepted scale of symptoms or markers for the diseases. It will be understood, however, that the total daily administration amount of the

active substance disclosed herein is decided by the attending physician within the scope of sound medical judgment. The exact administration amount required varies depending on factors such as the type of disease being treated, and gender, age, and weight of the subject.

As used herein, the term "optionally" may be taken to mean that the subsequently described structure, event or circumstance may or may not occur, and that the description includes both instances where the event occurs and instances where it does not occur.

Embodiments of the present disclosure have at least the following beneficial effects:

1. Through molecular structure optimization, the present disclosure skillfully integrates a radionuclide chelating group having a large molecular weight with a PSMA-targeting group, thereby obtaining the PSMA-targeting ligand compound or the stereoisomer, the tautomer, the hydrate, the solvate, the pharmaceutically acceptable salt or ester thereof. According to experimental results, the compounds provided by the present disclosure demonstrate performance in radiodiagnostic applications (PET/CT or SPECT/CT scans) comparable to currently marketed PSMA diagnostic products, showing no significant differences.
2. The compounds provided by the present disclosure exhibit outstanding advantages in clinical diagnostic applications. Following injection of the radiolabeled agent in the patients, PET/CT or SPECT/CT scanning demonstrates a high tumor-to-background ratio (target to background ratio). This enables clinicians to accurately obtain critical diagnostic information, including tumor size, precise location, and malignancy level, from the precise imaging data.
3. Through innovation and optimization of the molecular structure, the present disclosure achieves a groundbreaking multi-functional design within a single molecule. Specifically, the same molecular structure can be flexibly loaded with either diagnostic or therapeutic radionuclides. This enables combined diagnostic and therapeutic or even multiple synergistic functions, genuinely advancing the medical technology innovation of "integrated diagnosis and treatment".
4. Through precise optimization of the molecular structure, the drug molecule of the present disclosure exhibits significantly enhanced tumor cell targeting performance. Its high affinity for tumor cells results in prolonged retention at the lesion site alongside rapid clearance from the systemic circulation. The prolonged half-life *in vivo* enables a higher dose of the drug to accumulate at the tumor site. This allows for reduced and less frequent dosing, which effectively mitigates potential toxicity risks.

## BRIEF DESCRIPTION OF THE DRAWINGS

The embodiments of the present disclosure are described in detail below with reference to the accompanying drawings, in which:

FIG. 1, FIG. 2A, and FIG. 3 show the the liquid chromatography mass spectrometry (LC-MS), high performance liquid chromatography (HPLC), and hydrogen nuclear magnetic resonance ($^1$H-NMR) spectrum, respectively, of compound m3-12, FIG. 2B shows integration results of HPLC in FIG. 2A;
FIG. 4, FIG. 5A, and FIG. 6 show the LC-MS, HPLC, and $^1$H-NMR spectra, respectively, of compound m3-18, FIG. 5B shows integration results of HPLC in FIG. 5A;
FIG. 7, FIG. 8A, and FIG. 9 show the LC-MS, HPLC, and $^1$H-NMR spectra, respectively, of compound m3-19, FIG. 8B shows integration results of HPLC in FIG. 8A;
FIG. 10 , FIG. 11A, and FIG. 12 show the LC-MS, HPLC, and $^1$H-NMR spectra, respectively, of compound m3-12-1, FIG. 11B shows integration results of HPLC in FIG. 11A;
FIG. 13, FIG. 14A, and FIG. 15 show the LC-MS, HPLC, and $^1$H-NMR spectra, respectively, of compound m3-12-3, FIG. 14B shows integration results of HPLC in FIG. 14A; and
FIG. 16, FIG. 17A, and FIG. 18 show the LC-MS, HPLC, and $^1$H-NMR spectra, respectively, of compound m3-12-a, FIG. 17B shows integration results of HPLC in FIG. 17A.

## DETAILED DESCRIPTION OF THE EMBODIMENTS

The present disclosure is further described in detail below in conjunction with specific examples, and the examples given are only used to illustrate the present disclosure, but are not to limit the scope of the present disclosure.

Unless otherwise specified, the experimental methods used in the Examples are conventional methods; unless otherwise specified, all chemical reagents and biological products used are commercially available.

### Example 1 Synthesis of compound m3-12

Step 1: Synthesis of compound 2

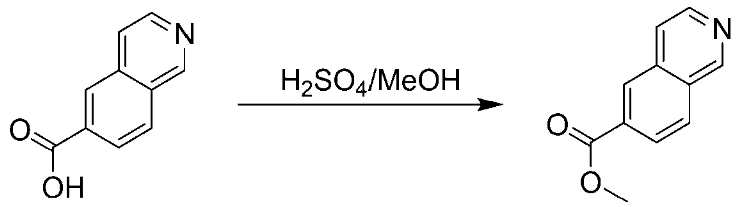


1 2

At room temperature, to a 250 mL three-necked flask equipped with a magnetic stirrer, compound 1 (10.0 g, 57.1 mol, 1.0 eq.) was added, which was then dissolved in MeOH (70 mL). Concentrated $H_2SO_4$ (41.6 mL, 782.0 mmol, 13.5 eq.) was slowly added thereto. A resulting mixture was stirred for 5 min to yield a black solution. The black solution was heated to 80°C and reacted overnight under reflux. The reaction was monitored by TLC, and when it showed complete consumption of raw materials and the formation of a major product spot, a resulting reaction solution was subjected to rotary evaporation. A resulting residue was stirred with water and ethyl acetate for liquid layering, and a resulting organic phase was washed with 0.5 mol/L aqueous NaOH solution to reach pH = 8-9. A resulting organic phase was then washed with saturated brine, separated, and concentrated to dryness to yield a product, i.e., compound 2 (8 g, with a yield of 74%) as an ash black solid, which was used in the next step.

Step 2: Synthesis of compound 3

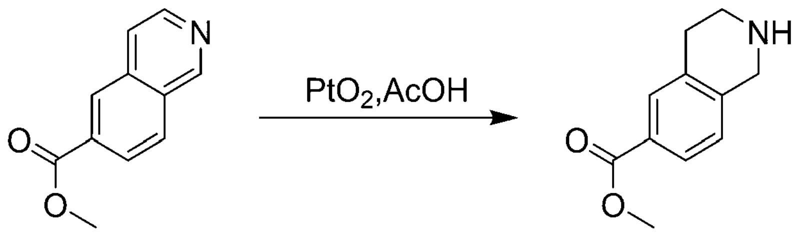


2 3

At room temperature, to a 250 mL single-neck flask, raw material 2 (8.0 g, 42.7 mmol, 1.0 eq.) was added, followed by adding MeOH (160 mL) and acetic acid (7.33 mL). A resulting mixture was stirred for dissolution, and $PtO_2$ (0.97 g, 12%wt) was then added. Atmosphere in the single-neck flask was replaced with nitrogen three times and then hydrogen twice using hydrogen balloon. A resulting mixture was reacted with stirring at 25°C for 48 h, during which hydrogen gas were replaced and replenished multiple times. Upon complete depletion of raw materials monitored by TLC, a resulting reaction mixture was subjected to suction filtration using a Buchner funnel loaded with diatomaceous earth. A resulting filter cake was washed three times with MeOH (30 mL) and the filtrate was subjected to rotary evaporation. A resulting crude product was extracted with ethyl acetate (EA), followed by washing with saturated aqueous $NaHCO_3$ solution and extraction. A resulting organic phase was concentrated to dryness to yield brownish black compound 3 (9.0 g), which was used in the next step;

Step 3: Synthesis of compound 4

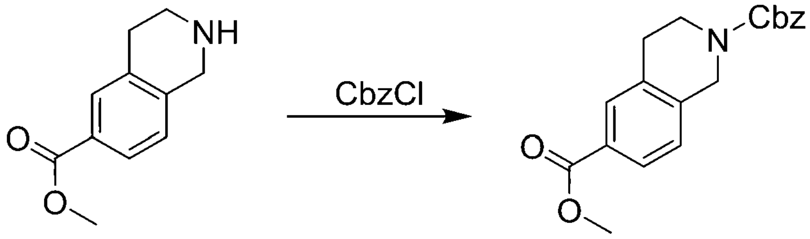


3 4

At room temperature, to a 250 mL single-neck flask filled with raw material 3 (8.0 g, 41.8 mmol, 1.0 eq.) dissolved in dichloromethane (DCM) (100 mL), triethylamine ($Et_3N$) (21.2 g, 209.1 mmol, 5.0 eq.) was added, and then benzyl chloroformate (CbzCl) (10.7 g, 62.8 mmol, 1.5 eq.) was slowly added. A resulting mixture was stirred at room temperature overnight. Upon product formation as monitored by liquid chromatography-mass spectrometry (LC-MS), the reaction was stopped. Water and ethyl acetate were added thereto and a resulting mixture was stirred for liquid layering. A resulting organic phase was washed with saturated brine, separated and concentrated to yield a crude product. The crude product was purified by silica gel column chromatography (petroleum ether (PE) : ethyl acetate (EtOAc) (volume ratio) = 20 : 1 to 10 : 1) to yield product 4 (7.7 g, with a yield of 100%, and a purity of 86.75%) as a yellow liquid, which was directly used in the next step.

Step 4: Synthesis of compound 5

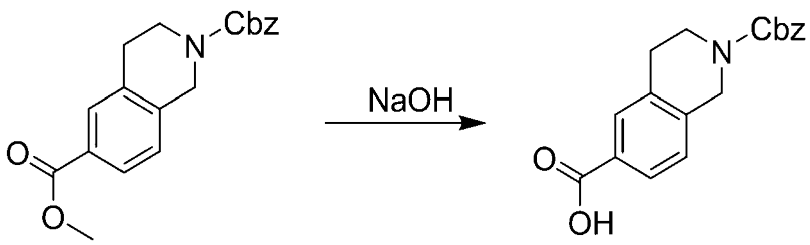


4 5

At room temperature, to a 500 mL single-neck flask, raw material 4 (7.0 g, 21.5 mmol, 1.0 eq.) was added, followed by adding MeOH (350 mL), and a resulting mixture was stirred for dissolution. An aqueous NaOH solution (35.1 g, 878.5 mmol, 40 eq.) was then added and a resulting mixture was stirred at 25°C for 2 hours. Upon new product spot formation and complete consumption of the raw materials as monitored by LC-MS, the reaction was stopped. A resulting reaction solution was concentrated and methanol therein was removed. The resulting reaction solution was adjusted with 4 N aqueous hydrochloric acid solution to pH = 2-3. Water and ethyl acetate were then added thereto and a resulting mixture was stirred for liquid layering. A resulting organic phases were combined and concentrated to dryness to yield a crude product, i.e., product 5 (4.8 g, with a yield of 71.7%) as a yellow solid.

Step 5: Synthesis of compound 6

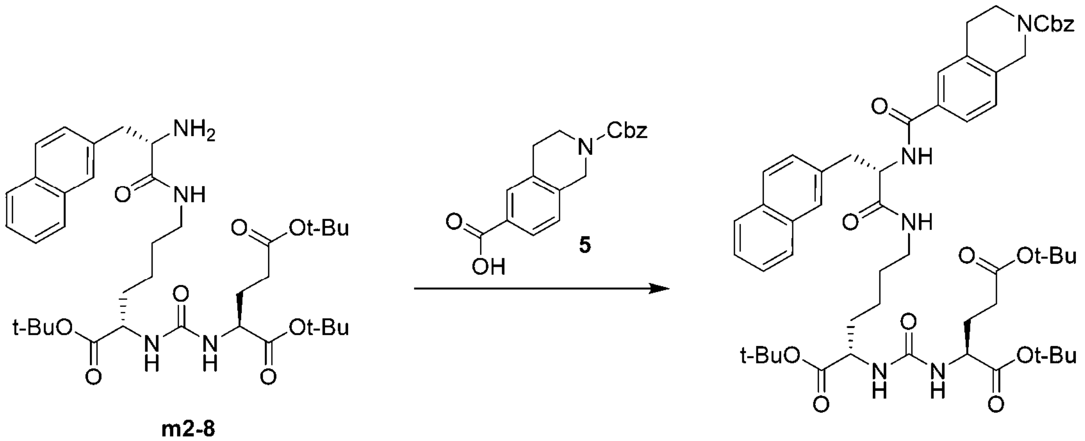

m2-8 5 6

At room temperature, to a 200 mL single-neck flask filled with raw material m2-8 (7.84 g, 11.4 mmol, 1.0 eq.) dissolved in DMF (100 mL), raw material 5 (4.63 g, 14.8 mmol, 1.3 eq.), N,N-diisopropylethylamine (DIEA) (3.78 mL, 22.9 mmol, 2.0 eq.) and 2-(7-azabenzotriazole)-N,N,N',N'-tetramethyluronium hexafluorophosphate (HATU) (6.53 g, 17.2 mmol, 1.5 eq.) were separately added. A resulting mixture was stirred at room temperature overnight. Upon target product formation and reaction completion as detected by LC-MS, saturated brine and ethyl acetate were added thereto and a resulting mixture was stirred for liquid layering. A resulting organic phase was concentrated to yield a crude target product. The crude product was purified by a silica gel column chromatography (PE : EtOAc = 2 : 1 to 1 : 1) to yield a product (11.0 g, with a purity of 98.2%, and a yield of 77%) as a yellow viscous oil.

Step 6: Synthesis of compound 7

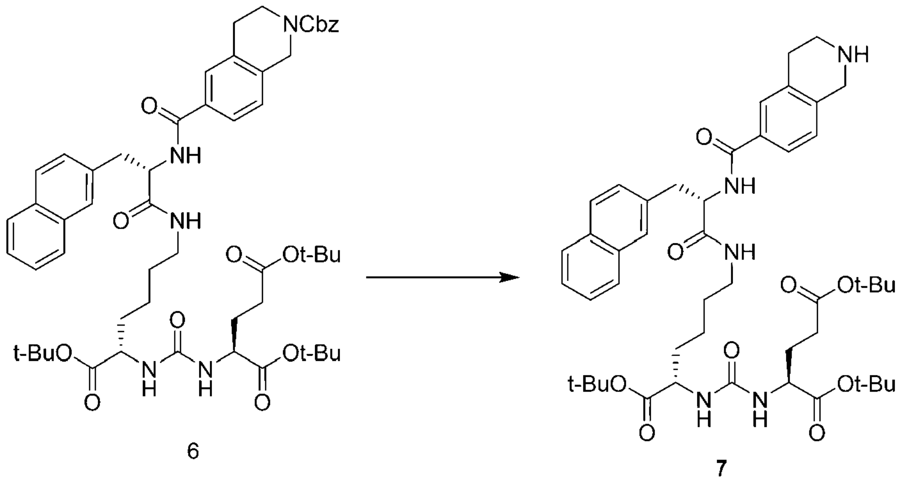

6 7

At room temperature, to a 250 mL single-neck flask, raw material 6 (10.8 g, 7.64 mmol, 1.0 eq.) was added, followed by adding MeOH (100 mL). A resulting mixture was stirred for dissolution. Wet Pd/C (1.1 g, 10% wt) was added thereto. Atmosphere in the single-neck flask was replaced with nitrogen three times and then hydrogen twice using a hydrogen gas balloon. A resulting mixture was then stirred at 25°C for 16 hours. Upon new product spot formation and complete consumption of the raw materials as monitored by TLC, the reaction was stopped. A resulting reaction mixture was subjected to suction filtration using a Buchner funnel loaded with diatomaceous earth. A resulting filter cake was washed with MeOH three times (30 mL × 3), a resulting filtrate was subjected to rotary evaporation, and the resulting reaction solution was concentrated and the solvent MeOH therein was removed. Water and ethyl acetate were then added thereto and a resulting mixture was stirred for liquid layering. Resulting organic phases were combined and concentrated to dryness to obtain a crude product. The crude product was concentrated to yield compound 7 (9.5 g, with a yield of 99.8%) as a brownish black viscous solid, which was directly used in the next reaction.

Step 7: Synthesis of compound 8

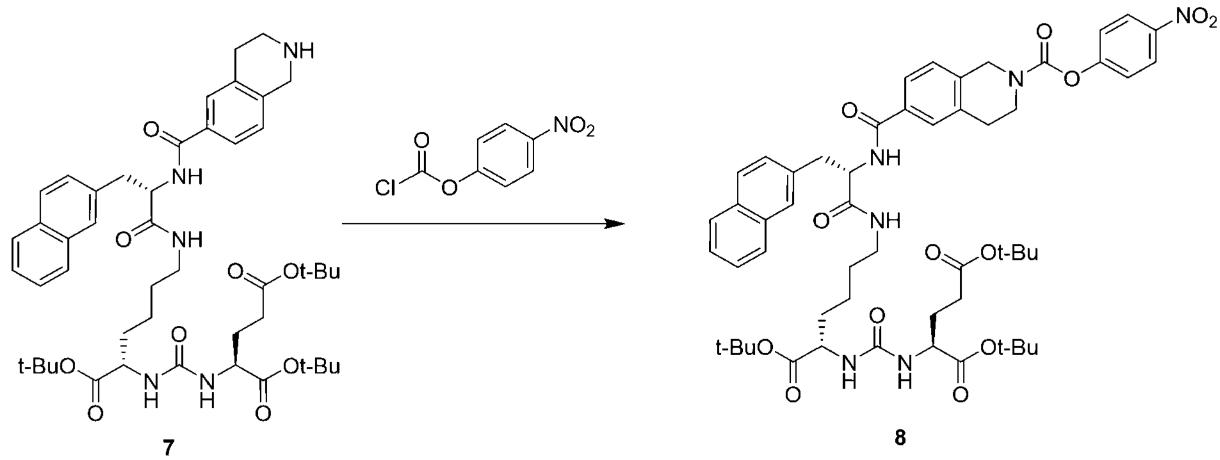


Under ice bath conditions, to a 100 mL single-neck flask filled with raw material 7 (4.6 g, 5.45 mmol, 1.0 eq.) dissolved in DCM (15 mL), p-nitrophenyl chloroformate (1.12 g, 5.6 mmol, 1.2 eq.) and DIEA (2.70 mL, 16.3 mmol, 3.0 eq.) were separately added. A resulting mixture was stirred at room temperature for 2 h. Upon product formation as monitored by LC-MS, the reaction was stopped. Water and ethyl acetate were added thereto, and a resulting mixture was subjected to extraction and liquid layering. A resulting organic phase was washed with saturated brine, separated and directly concentrated to remove the solvent therein. A resulting residue was purified by silica gel column chromatography (an eluent of DCM and MeOH (volume ratio = 50 : 1 to 20 : 1)) to yield the target product, i.e., compound 8 (4.2 g, with a yield of 76.4%) as a yellow oily foamy solid, which was directly used in the next reaction.

Step 8: Synthesis of compound 9

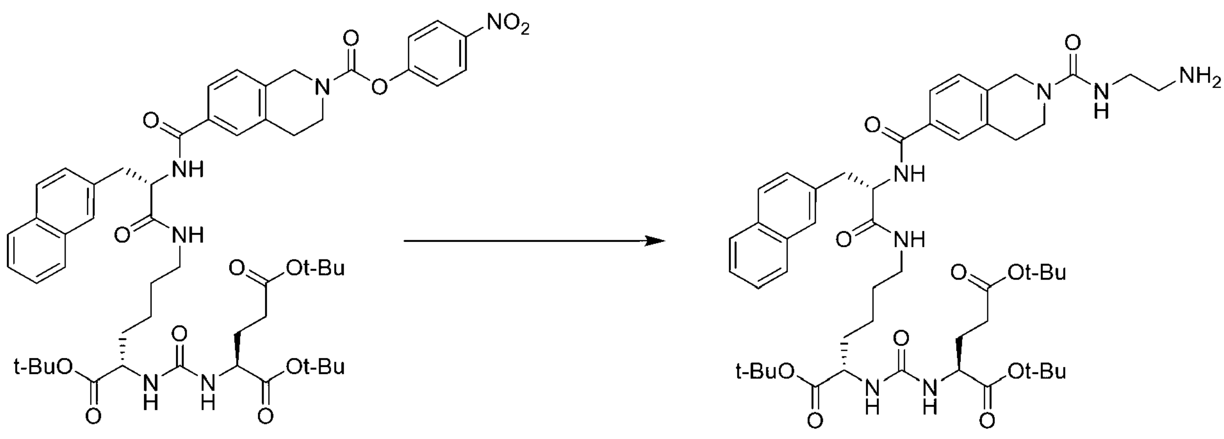


At room temperature, raw material 8 (4.6 g, 4.56 mmol, 1.0 eq.) and N,N-dimethylformamide (DMF) (40 mL) were added to a 100 mL single-neck flask, and a resulting mixture was stirred for dissolution. DIEA (2.26 mL, 13.7 mmol, 3.0 eq.) was added thereto, followed by adding ethylenediamine (3 mL, 44.9 mmol, 9.87 eq.), and a resulting mixture was stirred at room temperature overnight. Upon product formation and complete consumption of the raw materials as monitored by LC-MS, the reaction was stopped. Water and ethyl acetate were added thereto and a resulting mixture was stirred for liquid layering. Resulting organic phases were combined and concentrated to dryness to yield a crude product, i.e., product 9 (3.9 g) as a pale yellow viscous liquid.

Step 9: Synthesis of compound 10

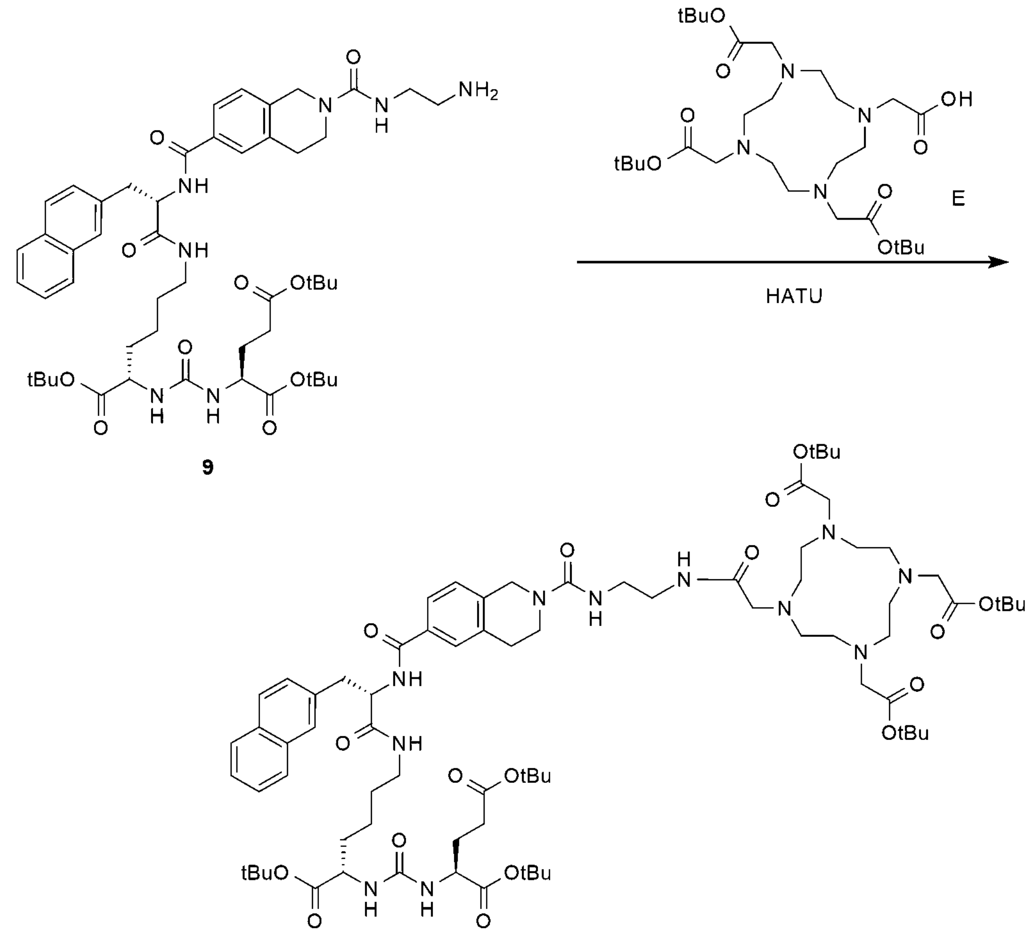


9

10

At room temperature, to a 100 mL single-neck flask filled with raw material 9 (3.8 g, 4.1 mmol, 1.0 eq.) dissolved in DMF (40 mL), raw material E (3.51 g, 6.1 mmol, 1.5 eq.), DIEA (1.35 mL, 8.2 mmol, 2.0 eq.) and HATU (2.33 g, 6.1 mmol, 1.5 eq.) were separately added, and a resulting mixture was stirred at room temperature overnight. Upon product formation as monitored by LC-MS, the reaction was stopped. Water and ethyl acetate were added thereto and a resulting mixture was subjected to extraction and liquid layering. A resulting organic phase was washed with saturated brine, separated and concentrated to obtain a crude product. The crude product was purified by silica gel column chromatography to yield compound 10 (3.9 g), and then subjected to preparative high-performance liquid phase chromatography (Welch Xtimate C18, 21.2 × 250 mm, 5 μm) to yield a pale yellow foamy solid 10 (2.2 g, with a yield of 36.2%). Step 10: Synthesis of compound m3-12

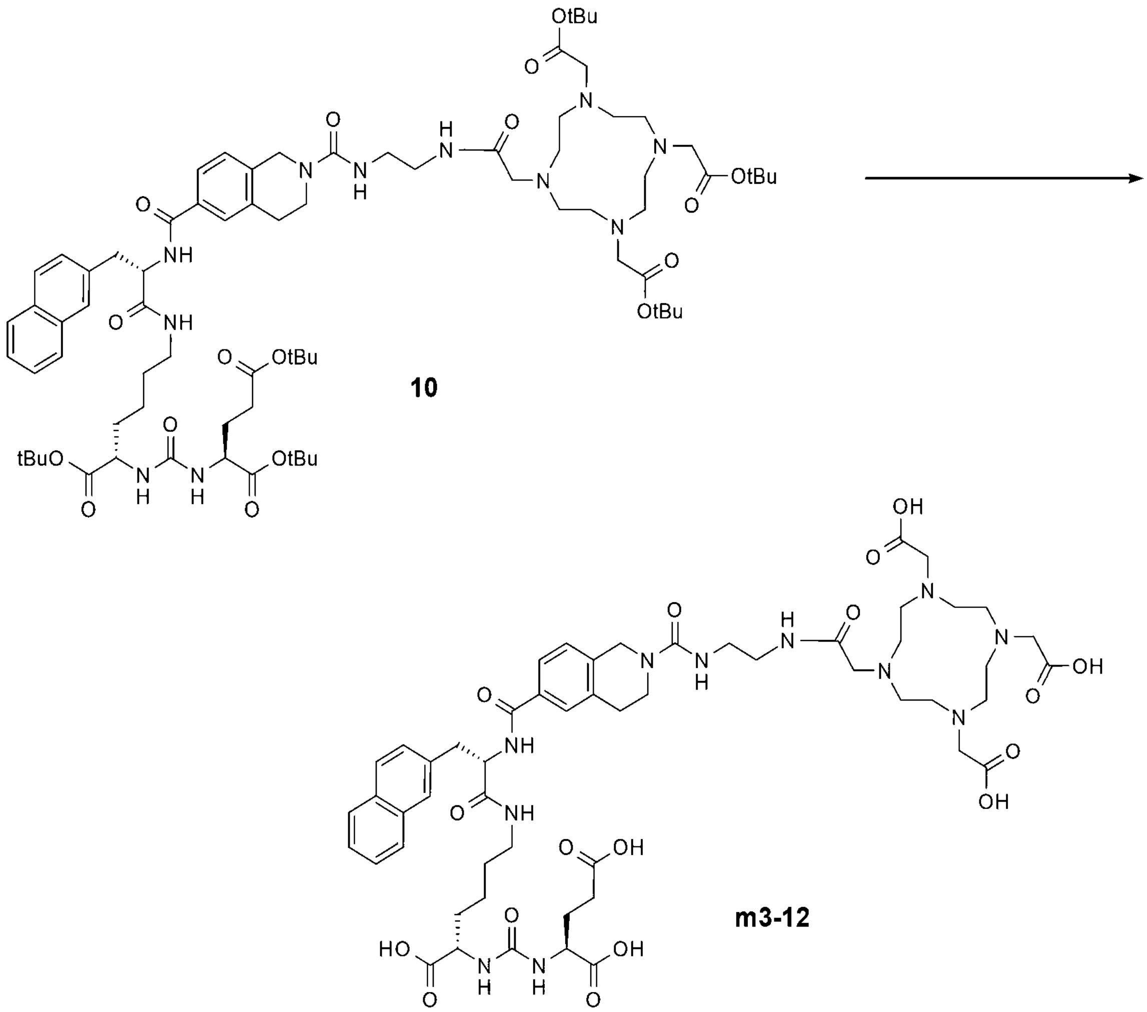

10

m3-12

At room temperature, raw material 10 (0.6 g, 0.41 mmol) and DCM (12 mL) were added to a 50 mL single-neck flask, and a resulting mixture was stirred for dissolution. Trifluoroacetic acid (TFA) (24 mL) was then added thereto and a resulting mixture was reacted at 25°C for 16 h. Upon product formation and complete consumption of the raw materials as monitored by LC-MS, the reaction was stopped. Saturated brine and ethyl acetate were added thereto and a resulting mixture was stirred and subjected to liquid layering. A resulting organic phase was collected, concentrated under reduced pressure and then subjected to preparative high-performance liquid phase chromatography (Welch Xtimate C18, 21.2 × 250 mm, 5 μm) to yield m3-12 (320 mg, with a yield of 69.9%, and a purity of 97.1%) as a white solid. The purity was determined to be up to standard.

The LC-MS, HPLC and $^1$H-NMR spectra of m3-12 are shown in FIG. 1, FIG. 2A, and FIG. 3, respectively.

**Example 2 Synthesis of compound m3-18**

Step 1: Synthesis of compound m3-18-8

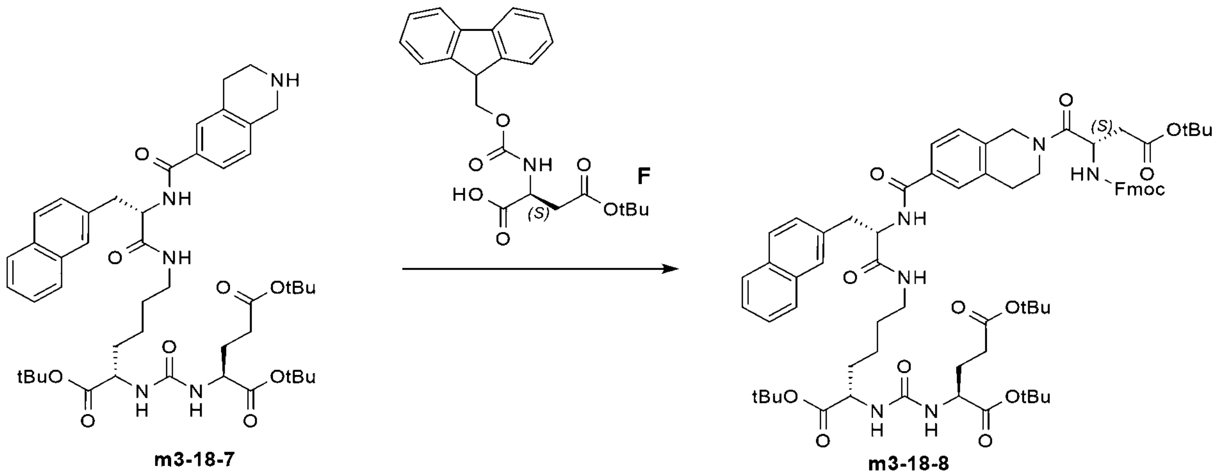


m3-18-7 m3-18-8

At room temperature, to a 100 mL single-neck flask filled with raw material m3-18-7 (4.5 g, 5.3 mmol, 1.0 eq.) dissolved in DMF (40 mL), raw material F (2.85 g, 6.9 mmol, 1.3 eq.), DIEA (1.76 mL, 10.7 mmol, 2.0 eq.) and HATU (3.04 g, 8.0 mmol, 1.5 eq.) were separately added. A resulting mixture was stirred at room temperature overnight. Upon product formation as monitored by LC-MS, the reaction was stopped. Water and ethyl acetate were added thereto and a resulting mixture was subjected to extraction and liquid layering. A resulting organic phase was washed with saturated brine, separated and concentrated to obtain a crude product. The crude product was purified by silica gel column chromatography (DCM : MeOH (volume ratio) = 20 : 1 to 10 : 1) to yield compound m3-18-8 (8.2 g), which was directly used in the next step.

Step 2: Synthesis of compound m3-18-9

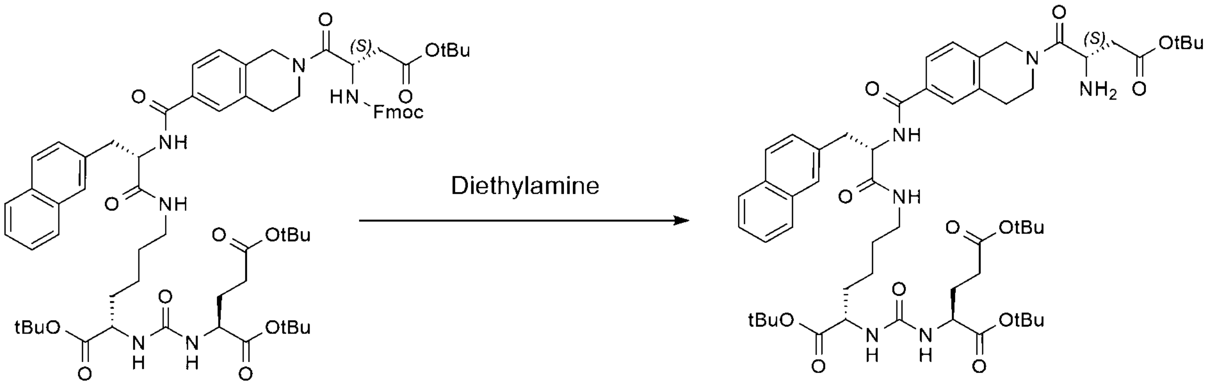


m3-18-8 m3-18-9

At room temperature, raw material m3-18-8 (7.9 g, 3.2 mmol, 1.0 eq.) and DMF (40 mL) were added to a 100 mL single-neck flask, and a resulting mixture was stirred for dissolution. Diethylamine (6.6 mL, 63.8 mmol, 20 eq.) was added thereto and a resulting mixture was stirred at room temperature overnight. When LC-MS showed the clear formation of the target product, the reaction mixture was directly concentrated to remove the solvent therein, and a resulting reside was then purified by silica gel column chromatography (DCM : MEOH (volume ratio) = 50 : 1, 40 : 1, 30 : 1, 20 : 1) to yield the target product, i.e., compound m3-18-9 as a pale yellow solid (2.3 g, with a yield of 70.1%, and a purity of 78.7%).

Step 3: Synthesis of compound m3-18-10

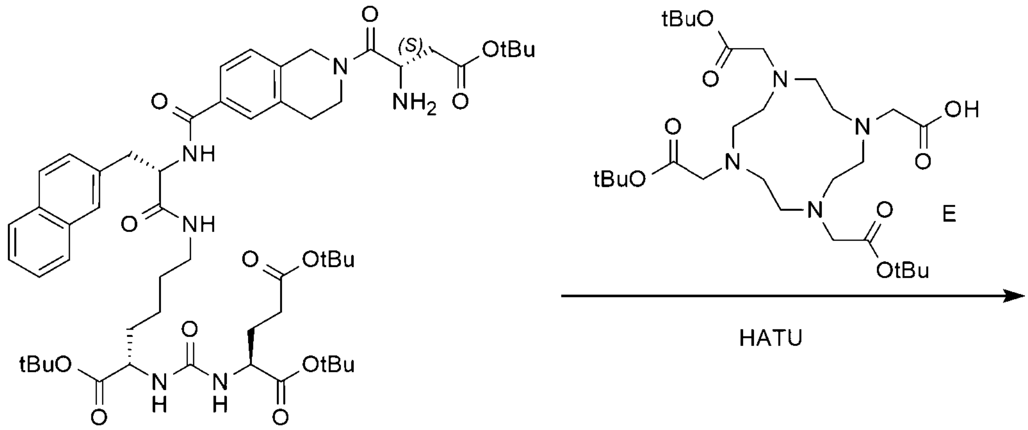


m3-18-9

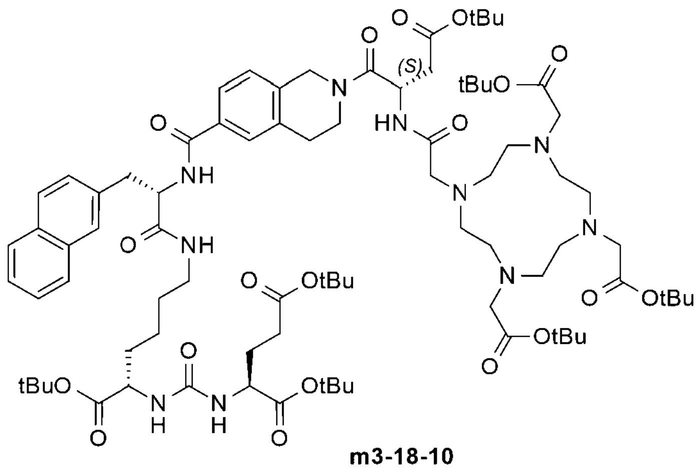

m3-18-10

At room temperature, to a 100 mL single-neck flask filled with raw material m3-18-9 (2.2 g, 2.2 mmol, 1.0 eq.) dissolved in DMF (40 mL), raw material E (1.86 g, 3.3 mmol, 1.5 eq.), DIEA (0.56 g, 4.4 mmol, 2.0 eq.) and HATU (1.24 g, 3.3 mmol, 1.5 eq.) were separately added. A resulting mixture was stirred at room temperature overnight. Upon product formation as monitored by LC-MS, the reaction was stopped. Water and ethyl acetate were added thereto and a resulting mixture was subjected to extraction and liquid layering. A resulting organic phase was washed with saturated brine, separated and concentrated to obtain a crude product. The crude product was purified by silica gel column chromatography to yield crude compound m3-18-10, followed by preparative high-performance liquid phase chromatography (Welch Xtimate C18, 21.2 × 250 mm, 5 μm) to yield a pale yellow foamy solid m3-18-10 (2.8 g, with a yield of 82.3%).

Step 4: Synthesis of compound m3-18

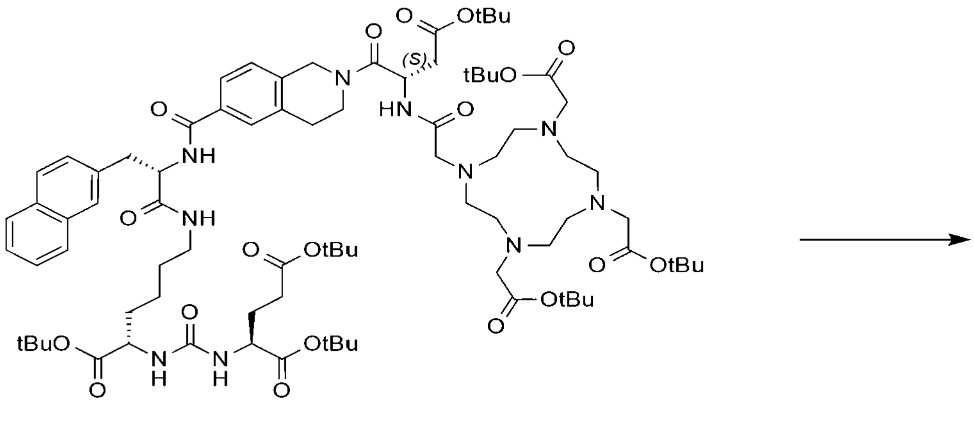

**m3-18-10**

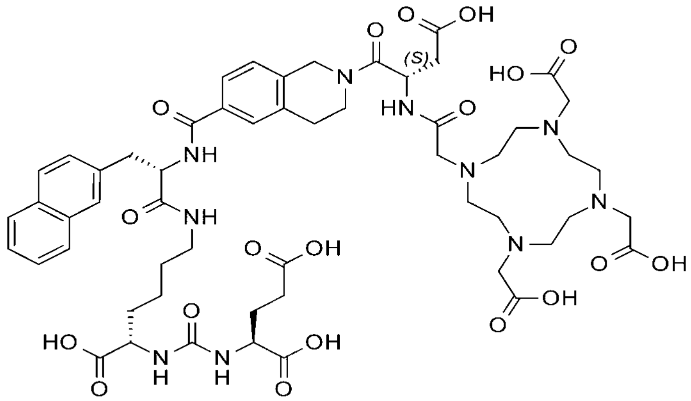

**m3-18**

At room temperature, raw material m3-18-10 (0.3 g, 0.19 mmol) and DCM (20 mL) were added to a 50 mL single-neck flask, a resulting mixture was stirred for dissolution, and a HCl/dioxane solution (0.96 mL, 3.82 mmol, 10 eq.) was added thereto. A resulting mixture was reacted at 25°C for 3 h. Upon product formation and complete consumption of the raw materials as monitored by LC-MS, the reaction was stopped. A resulting reaction mixture was directly concentrated under reduced pressure and then subjected to preparative high-performance liquid phase chromatography (Welch Xtimate C18, 21.2 × 250 mm, 5 μm) to yield m3-18 (23 mg, with a yield of 10%, and a purity of 98.15%) as a white solid. The purity was determined to be up to standard.

The LC-MS, HPLC and $^1$H-NMR spectra of m3-18 are shown in FIG. 4, FIG. 5A, and FIG. 6, respectively.

**Example 3 Synthesis of compound m3-19**

Step 1: Synthesis of compound m3-19-3

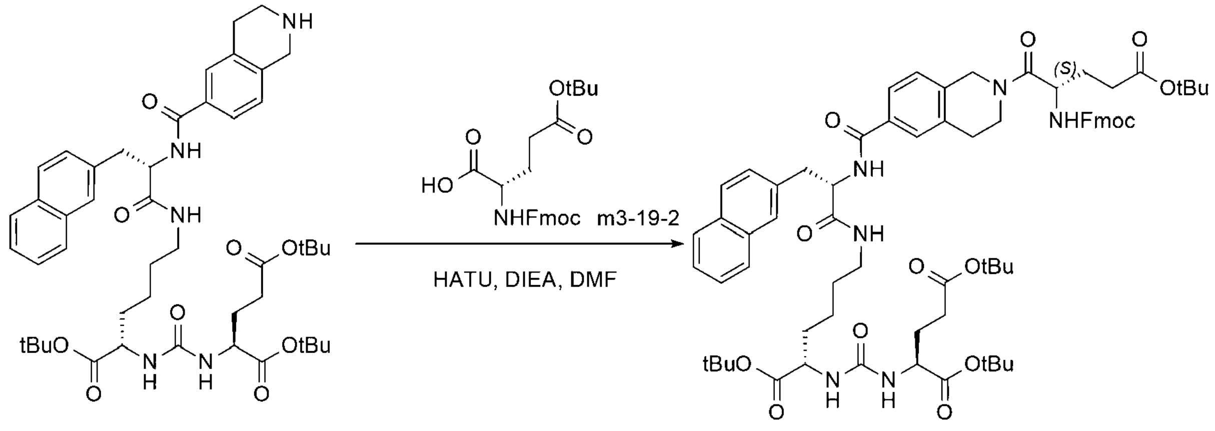


**m3-19-1** **m3-19-3**

Reactant m3-19-1 (2.5 g, 2.98 mmol), HATU (1.47 g, 3.878 mmol) and the solvent DMF (20 mL) were added to 50 mL eggplant-shaped flask to yield a yellow suspension. The reaction system was cooled down to 0°C under nitrogen protection, and reactant m3-19-2 (1.52 g, 3.58 mmol) and DIEA (1.479 mL, 8.948 mmol) were slowly added thereto. A resulting reaction solution was stirred at 25°C under $N_2$ for 2 h. After the reaction was completed, a resulting reaction solution was diluted with 200 mL of ethyl acetate (EA), and washed with 200 mL of saturated brine, saturated $NH_4Cl$ solution and water, respectively. A resulting organic layer was dried over anhydrous $Na_2SO_4$, filtered and concentrated to yield a crude product. The crude product was purified by a silica gel column chromatography (an eluent of DCM and MeOH (volume ratio: 60 : 1)), and a sample after concentration was collected to yield product m3-19-3 (3 g, with a yield of 73%, and a purity of 100%).

Step 2: Synthesis of compound m3-19-4

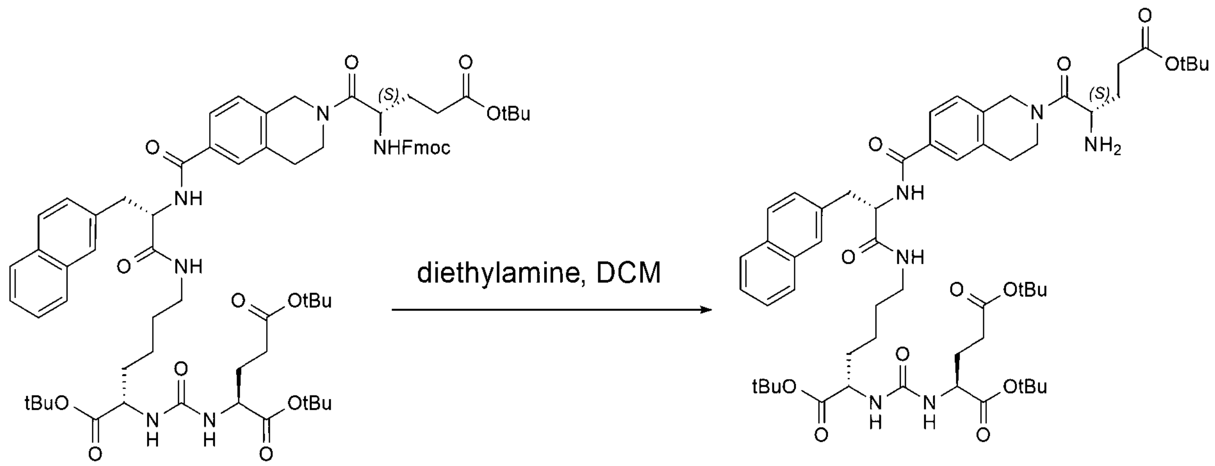


**m3-19-3** **m3-19-4**

Reactant m3-19-3 (3 g, 2.412 mmol) and solvent DCM (10 mL) were added to a 50 mL eggplant-shaped flask to yield a brown solution. Reactant diethylamine (2.496 mL, 24.123 mmol) was slowly added thereto and a resulting reaction solution was stirred at 25°C. After the reaction was completed, a resulting reaction solution was concentrated to yield a crude product, and the crude product was purified by a silica gel column chromatography (an eluent of DCM and MeOH (volume ratio: 50 : 1)). A sample after concentration was collected to yield product m3-19-4 (2.2 g, with a yield of 89.0%, and a purity of 100%).

Step 3: Synthesis of compound m3-19-6

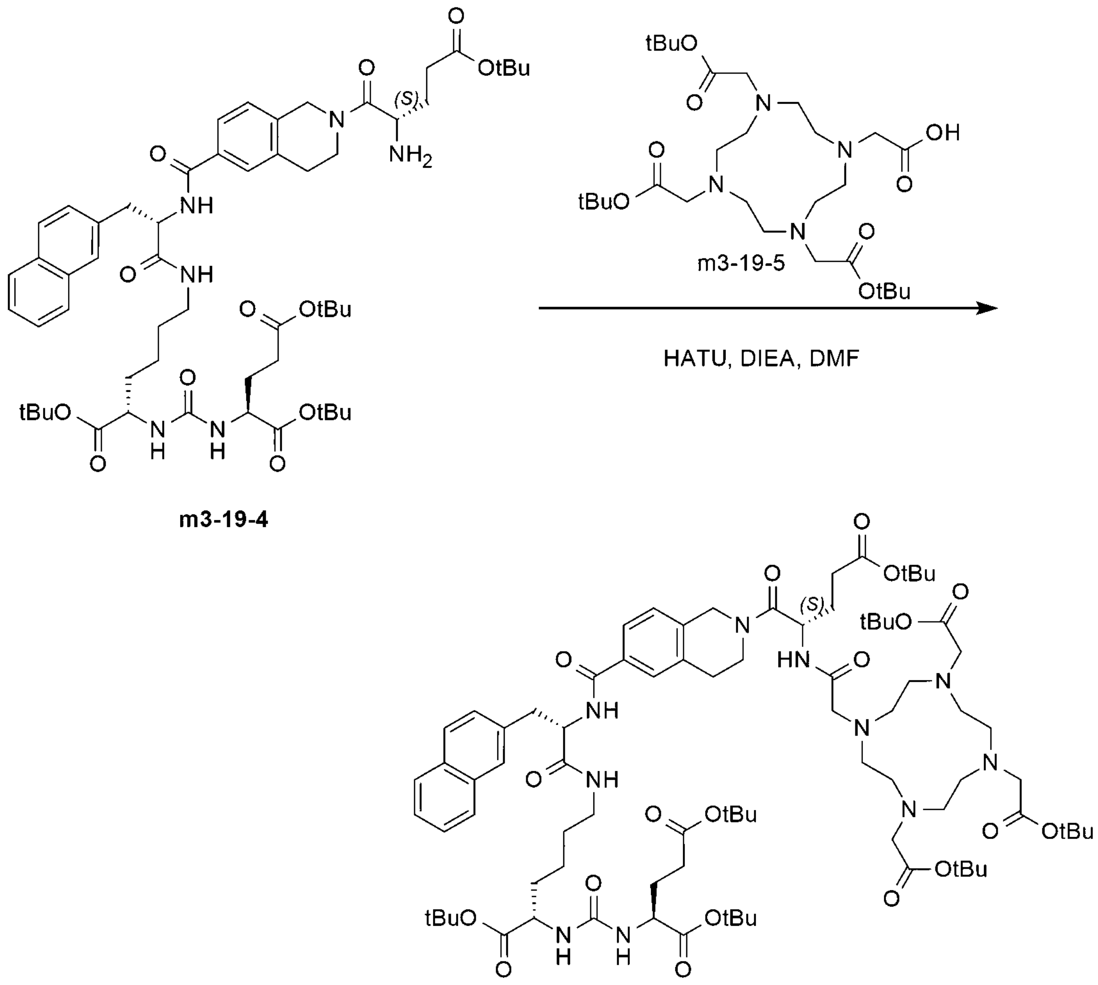


m3-19-4

m3-19-5

m3-19-6

Reactant m3-19-5 (1.47 g, 2.585 mmol), HATU (1.23 g, 3.231 mmol) and solvent DMF (20 mL) were added to a 100 mL three-necked flask to yield a white suspension. Reactant m3-19-4 (2.2 g, 2.154 mmol) dissolved in 5 mL of DMF and DIEA (1.068 mL, 6.462 mmol) were slowly added thereto in sequence. A resulting reaction solution was stirred at 25°C for 2 h. After the reaction was completed, a resulting reaction solution was diluted with 200 mL of EA, and washed with 200 mL of saturated brine and 200 mL of water in sequence. A resulting EA layer was dried over anhydrous $Na_2SO_4$, filtered and concentrated to yield a crude product. The crude product was purified by preparative reversed-phase HPLC (Welch Xtimate C18, 21.2 × 250 mm, 5 μm) to yield product m3-19-6 (1.4 g, 0.847 mmol, with a yield of 39.35%, and a purity of 95%).

Step 4: Synthesis of compound m3-19

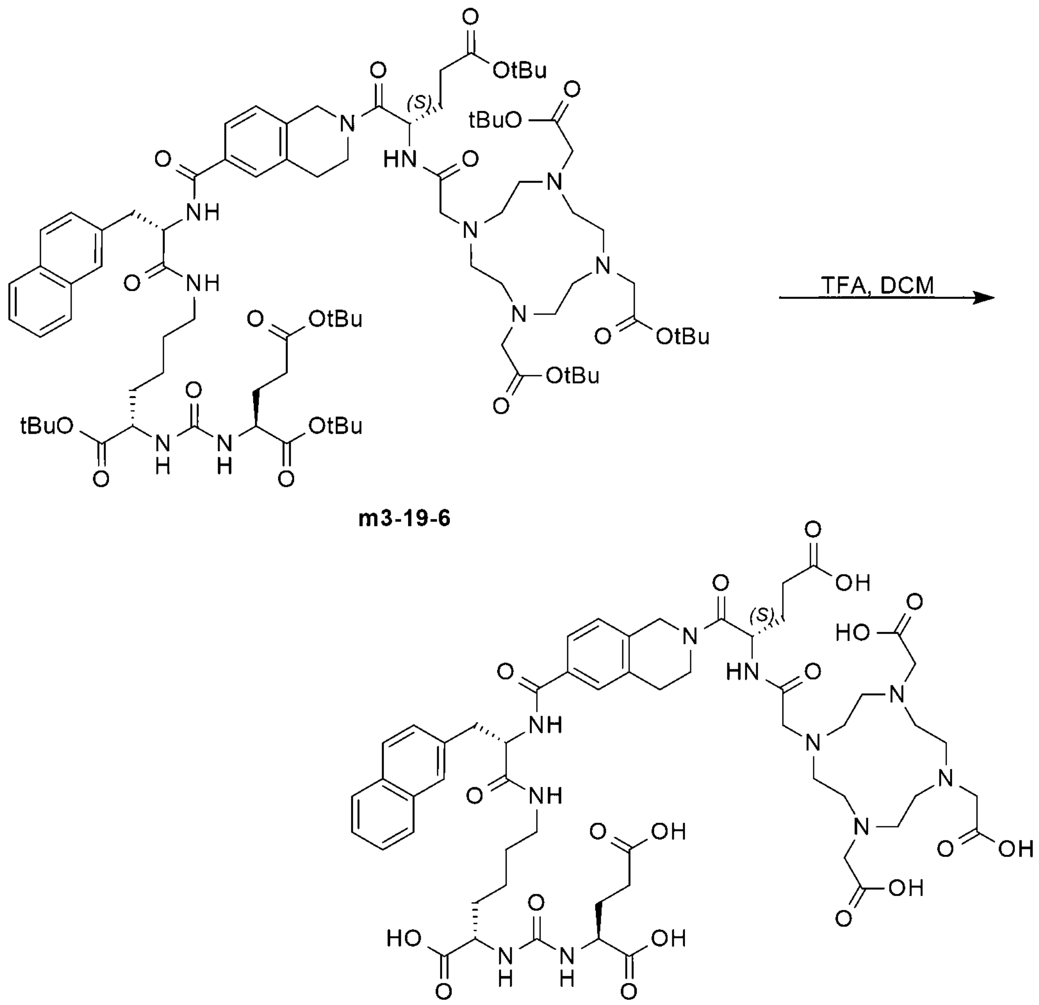


m3-19-6

m3-19

Reactant m3-19-6 (660 mg, 0.420 mmol) and solvent DCM (2 mL) were added to a 25 mL eggplant-shaped flask to yield a yellow solution. Reactant TFA (1437.77 mg, 12.610 mmol) was slowly added dropwise thereto. A resulting reaction solution was stirred at 25°C for 4 h. After the reaction was completed, the resulting reaction solution was concentrated at a low temperature to yield a crude product. The crude product was subjected to separation by preparative HPLC (Welch Xtimate C18, 21.2 × 250 mm, 5 μm) to yield the final product m3-19 (290 mg, 0.233 mmol, with a yield of 55.41%, and a purity of 95.68%). The purity was determined to be up to standard.

The LC-MS, HPLC and $^1$H-NMR spectra of m3-19 are shown in FIG. 7, FIG. 8A, and FIG. 9, respectively.

## Example 4 Synthesis of compound m3-12-1

Step 1: Synthesis of compound m3-12-1-1

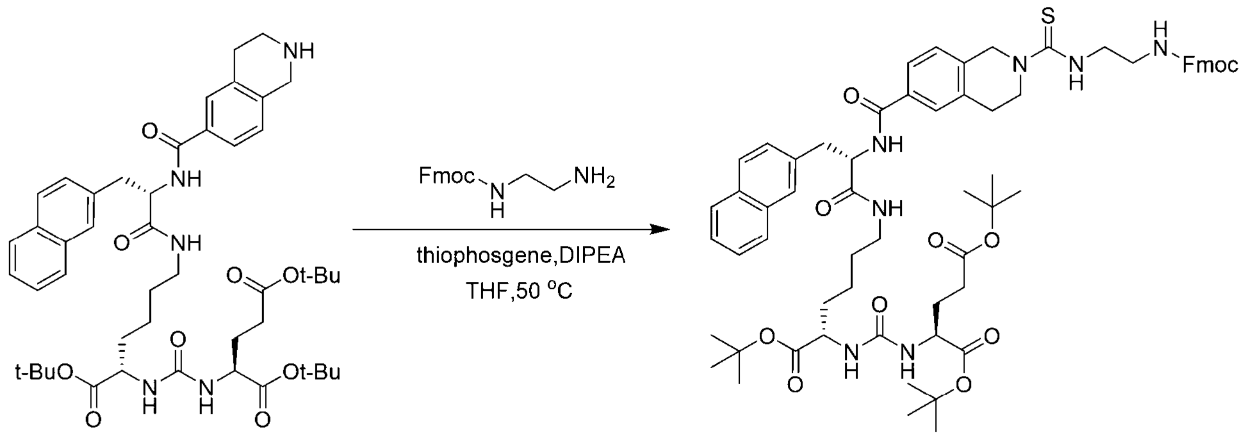


A

m3-12-1-1

To a 100 mL Schlenk tube, N-fluorenylmethoxycarbonylethylenediamine (2 g, 7.084 mmol, 1.0 eq.) was added and dissolved in tetrahydrofuran (THF) (20 mL). Thiophosgene (0.81 g, 7.084 mmol, 1.0 eq.) was added thereto. Under nitrogen protection, N,N-diisopropylethylamine (1.83 g, 14.167 mmol, 2.0 eq.) was added to the above-mentioned reaction solution with a syringe at room temperature. The reaction solution was reacted at 50°C for 2 h. The reaction was monitored by LC-MS until the raw materials were consumed. Post-treatment: A resulting reaction solution was diluted with 20 mL of water, ethyl acetate was then added thereto and a resulting mixture was subjected to extraction and liquid layering. A resulting organic phase was washed with saturated brine, separated and concentrated to yield the corresponding crude isothiocyanate product (1.3 g) as a yellow solid.

To another 100 mL Schlenk tube, compound A (2.2 g, 2.56 mmol, 1.0 eq.) was added and dissolved with THF (10 mL). Isothiocyanate (0.91 g, 2.80 mmol, 1.1 eq.) prepared above was added thereto. Under nitrogen protection, N,N-diisopropylethylamine (0.66 g, 5.10 mmol, 2.0 eq.) was added to the above-mentioned reaction solution with a syringe at room temperature. The reaction solution was reacted at 50°C for 2 h. The reaction was monitored by LC-MS and when no further change in the raw materials was observed, the reaction was stopped. Post-treatment: A resulting reaction solution was diluted by adding water, and ethyl acetate was added thereto, and a resulting mixture was subjected to extraction and liquid layering. A resulting organic phase was washed with saturated brine, separated and concentrated to yield crude target product m3-12-1-1 (2.9 g, with a yield of 97.0%) as a brown oil, which was directly used in the next step without further purification.

Step 2: Synthesis of compound m3-12-1-2

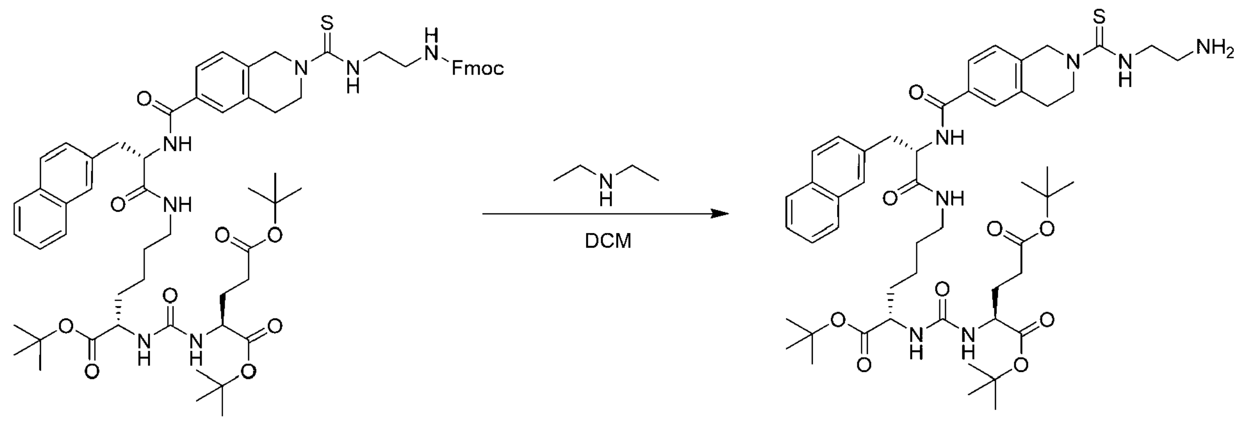


m3-12-1-1

m3-12-1-2

At room temperature, raw material m3-12-1-1 (2.9 g, 2.48 mmol, 1.0 eq.) and DCM (10 mL) were added to a 100 mL single-neck flask, and a resulting mixture was stirred for dissolution. Diethylamine (12.7 mL, 123.29 mmol, 50.0 eq.) was added thereto and a resulting mixture was stirred at 25°C for 2 hours. Upon new product spot formation and complete consumption of the raw materials as monitored by LC-MS, the reaction was stopped. Post-treatment: A resulting reaction

solution was concentrated to remove the solvent DCM and ethylenediamine; water and ethyl acetate were then added, and a resulting mixture was stirred and subjected to liquid layering. Resulting organic phases were combined and concentrated to dryness to yield a crude product. The crude product was purified by silica gel column chromatography (DCM : MeOH (volume ratio) = 20 : 1 to 10 : 1) to yield a rather pure product m3-12-1-2 (1.93 g, with a yield of 82.1%, and a purity of 90%) as a pale yellow viscous liquid.

Step 3: Synthesis of compound m3-12-1-3

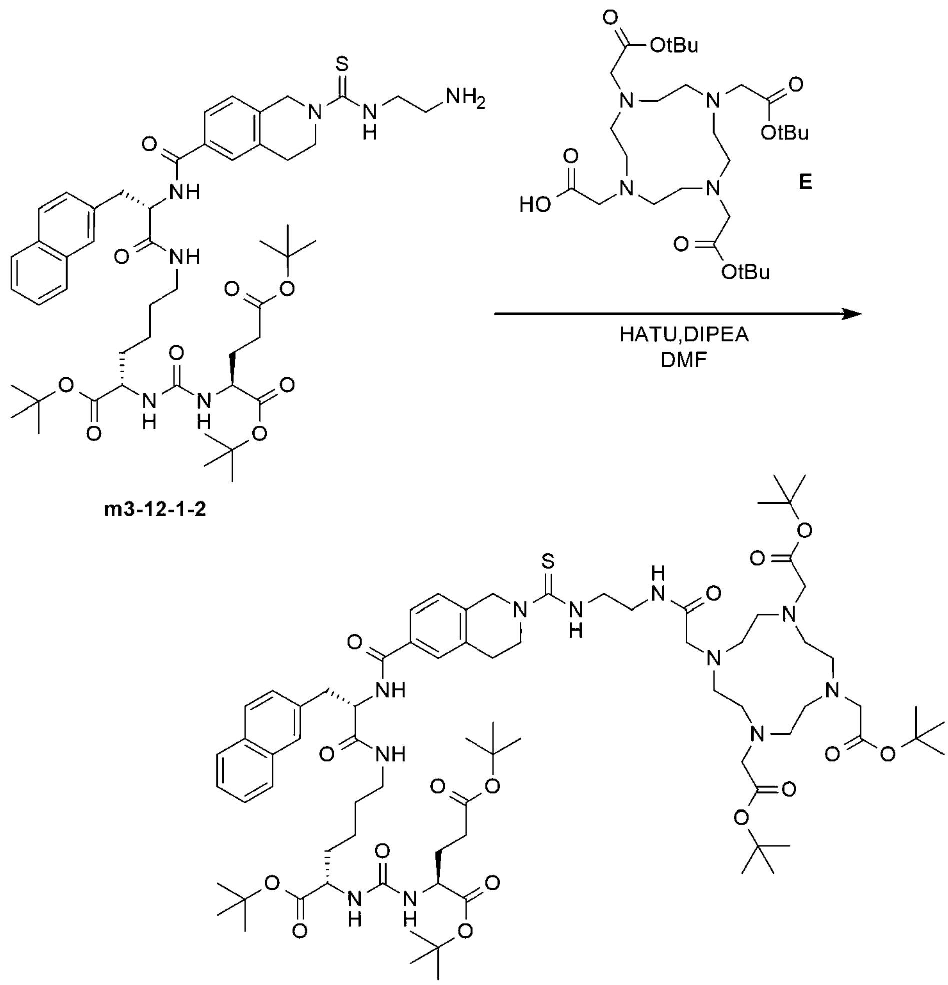


E

m3-12-1-2

m3-12-1-3

At room temperature, raw material m3-12-1-2 (1.93 g, 2.04 mmol, 1.0 eq.) and DMF (20 mL) were added to a 100 mL single-neck flask. A resulting mixture was stirred for dissolution. Raw material E (1.40 g, 2.45 mmol, 1.2 eq.), DIEA (0.53 g, 4.10 mmol, 2.0 eq.) and HATU (1.16 g, 3.05 mmol, 1.5 eq.) were separately added thereto and a resulting mixture was stirred at room temperature overnight. Upon product formation as monitored by LC-MS, the reaction was stopped. Water and ethyl acetate were added thereto and a resulting mixture was subjected to extraction and liquid layering. A resulting organic phase was washed with saturated brine, separated and concentrated to obtain a crude product. The crude product was purified by preparative high-performance liquid phase chromatography (Welch Xtimate C 18, 21.2 × 250 mm, 5 μm) to yield the product m3-12-1-3 (1.66 g, with a yield of 54.2%, and a purity of 95%), which was a white solid after lyophilizing.

Step 4: Synthesis of compound m3-12-1

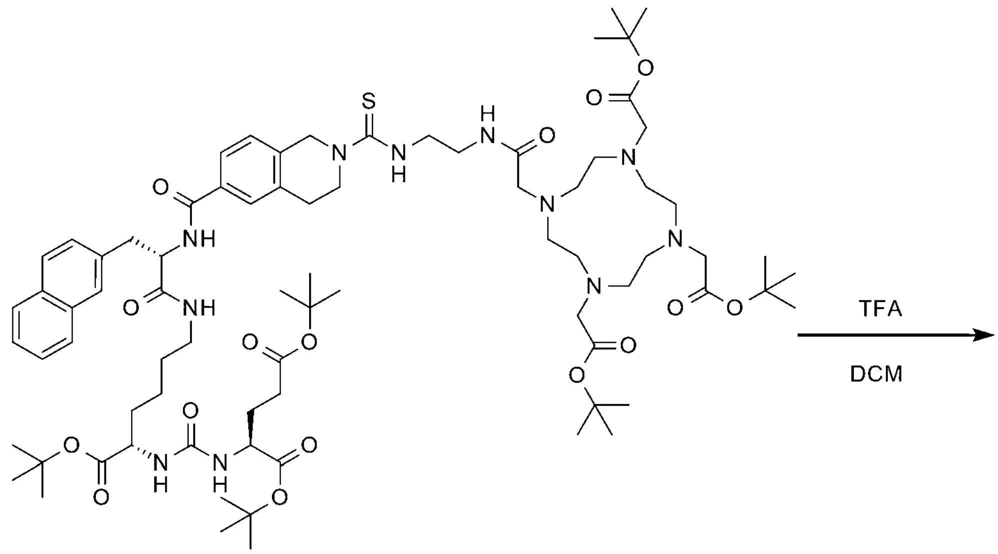


m3-12-1-3

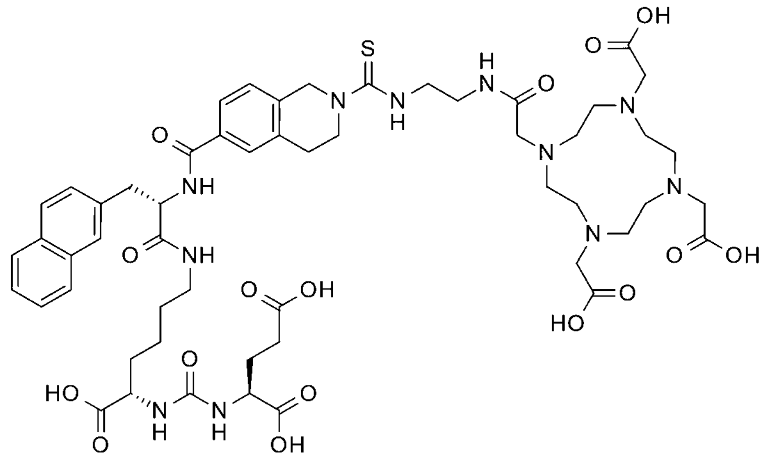

m3-12-1

At room temperature, raw material m3-12-1-3 (0.60 g, 0.40 mmol) and DCM (10 mL) were added to a 50 mL single-neck flask, and a resulting mixture was stirred for dissolution. TFA (15 mL) was then added thereto and a resulting mixture was reacted at 25°C for 2 h. Upon product formation and complete consumption of the raw materials as monitored by LC-MS, the reaction was stopped. Post-treatment: A resulting reaction mixture was extracted with ethyl acetate and the resulting organic phase was concentrated under reduced pressure (below 30°C) and then purified by preparative high-performance liquid phase chromatography (Welch Xtimate C18, 21.2 × 250 mm, 5 μm) to yield a product m3-12-1 (0.1 g, with a yield of 38.7%, and a purity of 92.03%), which was a white solid after lyophilizing.

The LC-MS, HPLC and $^1$H-NMR spectra of m3-12-1 are shown in FIG. 10, FIG. 11A, and FIG. 12, respectively.

**Example 5 Synthesis of compound m3-12-3**

Step 1: Synthesis of compound 1

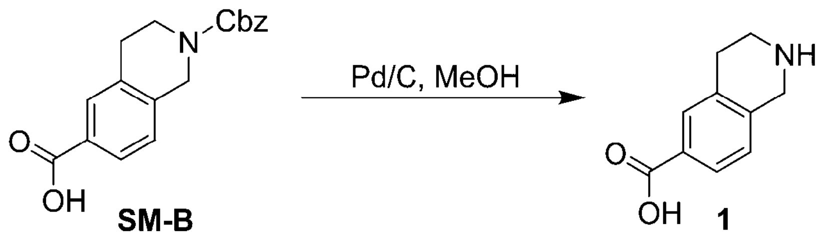

SM-B
1

To a 500 mL eggplant-shaped flask, reactant SM-B (5 g, 16.060 mmol), palladium on carbon containing 10% (mass ratio) Pd (0.85 g, 8.030 mmol) and solvent MeOH (80 mL) were added to yield a black suspension. Atmosphere in the reaction system was replaced with hydrogen and a resulting mixture was stirred at 25°C for 16 h. After the reaction was completed, a resulting reaction solution was concentrated to yield crude product 1 (4 g, 14.673 mmol, with a yield of 91.36%, and a purity of 100%).

Step 2: Synthesis of compound SM-B-Fmoc

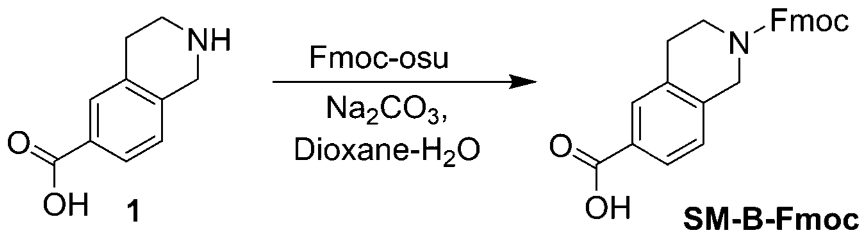

1
SM-B-Fmoc

Reactant 1 (4 g, 14.673 mmol), sodium carbonate (3.11 g, 29.345 mmol) and solvents of dioxane (90 mL) and water (10 mL) were added to a 500 mL eggplant-shaped flask to yield a black suspension. At room temperature, the reactant Fmoc-osu (9-fluorenylmethyl-N-succinimidyl carbonate) (5.44 g, 16.140 mmol) was slowly added thereto. A resulting reaction solution was then stirred at 25°C for 2 h. After the reaction was completed, a resulting reaction solution was diluted with 300 mL of EA, and washed with 200 mL of diluted aqueous HCl solution (pH = 5) three times. A resulting EA layer was dried over anhydrous $Na_2SO_4$, filtered and concentrated. A resulting reaction solution was concentrated to yield a crude product and the crude product was loaded on a silica gel column, followed by eluting with an eluent of DCM and MeOH (volume ratio: 50 : 1). Eluants were collected, combined and concentrated to yield product SM-B-Fmoc (5 g, 11.891 mmol, with a yield of 81.04%, and a purity of 100%).

Step 3: Synthesis of compound m3-12-3-1

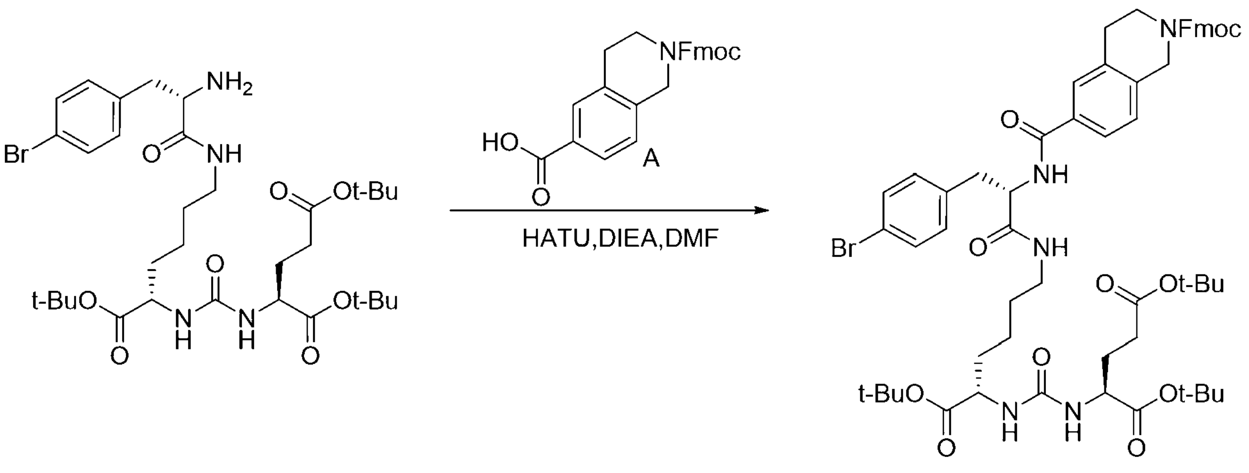

m4-4-2-2
m3-12-3-1

At room temperature, raw material m4-4-2-2 (4.0 g, 5.6 mmol, 1.0 eq.) was dissolved in DMF (30 mL) in a 100 mL single-neck flask, and raw material A (2.7 g, 6.7 mmol, 1.2 eq.), DIEA (2.2 g, 16.8 mmol, 3.0 eq.) and HATU (2.6 g, 6.7 mmol, 1.2 eq.) were then separately added thereto. The mixture was stirred at room temperature overnight. Upon product formation as monitored by LC-MS, the reaction was stopped. Water and ethyl acetate were added thereto and a resulting mixture was subjected to extraction and liquid layering. A resulting organic phase was washed with saturated brine, separated and concentrated to obtain a crude product. The crude product was purified by silica gel column chromatography (with DCM and MeOH (volume ratio = 100 : 1 to 80 : 1)) to yield the product m3-12-3-1 (4.0 g, with a yield of 65%).

Step 4: Synthesis of compound m3-12-3-2

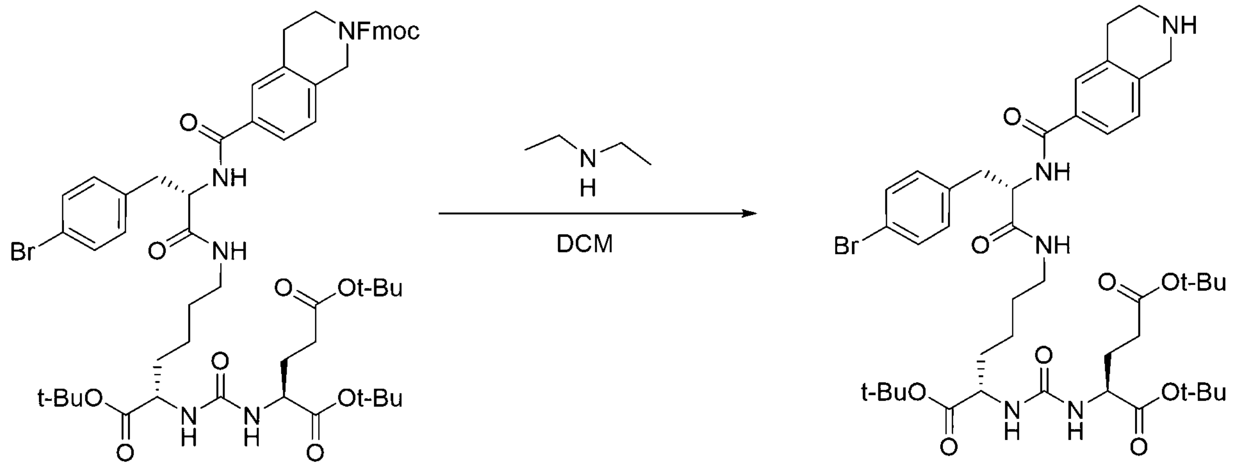


m3-12-3-1

m3-12-3-2

At room temperature, to a 100 mL single-neck flask, raw material m3-12-3-1 (3.9 g, 3.6 mmol, 1.0 eq.) was added, followed by adding DCM (50 mL), and a resulting mixture was stirred for dissolution. Diethylamine (2.6 g, 35.6 mmol, 10.0 eq.) was then added thereto and a resulting mixture was stirred at 25°C overnight. Upon product formation and complete consumption of the raw materials as monitored by LC-MS, the reaction was stopped. A resulting reaction solution was concentrated to dryness to yield a crude product. The crude product was purified by silica gel column chromatography (DCM : MeOH (volume ratio) = 50 : 1 to 10 : 1) to yield the product m3-12-3-2 (2.3 g, with a yield of 74%) as a pale yellow viscous liquid.

Step 5: Synthesis of compound m3-12-3-3

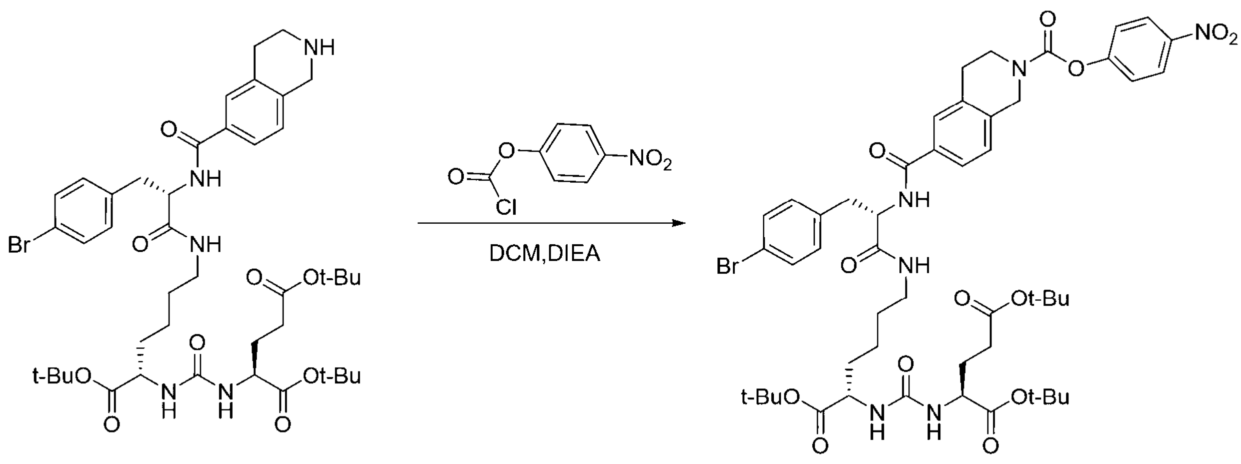


m3-12-3-2

m3-12-3-3

In a 100 mL three-necked flask, raw material m3-12-3-2 (4.5 g, 5.2 mmol, 1.0 eq.) was dissolved in DCM (60 mL). A resulting solution was cooled down to 0°C and p-nitrophenyl chloroformate (1.6 g, 7.8 mmol, 1.5 eq.) was added thereto, and DIEA (3.3 g, 26.0 mmol, 5.0 eq.) was slowly added dropwise thereto. After the addition, a resulting mixture was warmed to room temperature, stirred and reacted for 3 h. Upon product formation as monitored by LC-MS, the reaction was stopped. Water and dichloromethane were then added thereto and a resulting mixture was subjected to extraction and liquid layering. A resulting organic phase was washed with saturated brine, separated and concentrated to obtain a crude product. The crude product was purified by silica gel column chromatography (DCM : MeOH (volume ratio) = 100 : 1 to 20 : 1) to yield the product m3-12-3-3 (5.0 g, with a yield of 92%).

Step 6: Synthesis of compound m3-12-3-4

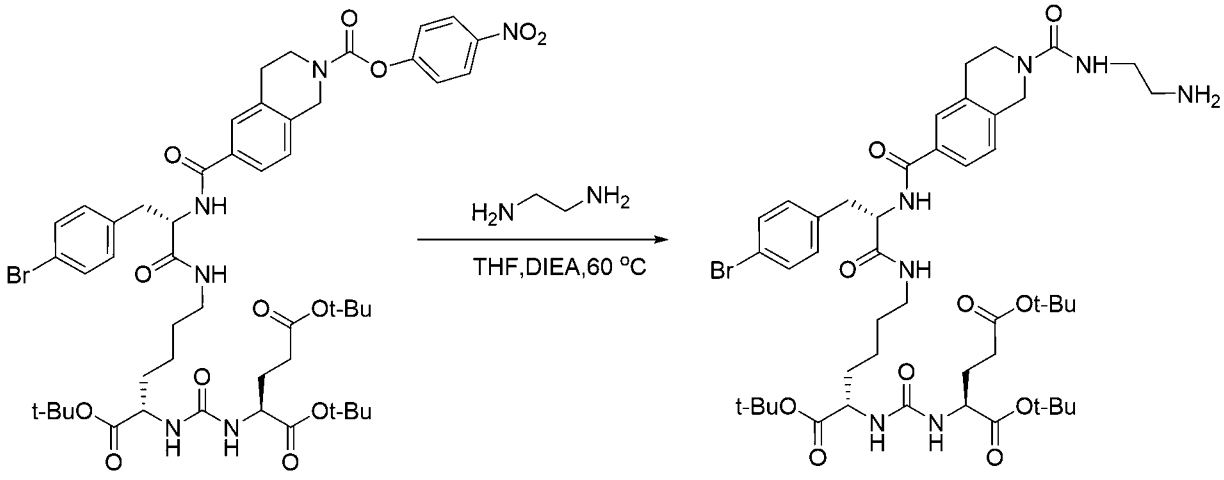

m3-12-3-3 m3-12-3-4

At room temperature, to a 100 mL single-neck flask, raw material m3-12-3-3 (5.0 g, 4.8 mmol, 1.0 eq.) was added, followed by adding THF (60 mL), and a resulting mixture was stirred for dissolution. Ethylenediamine (4.3 g, 72 mmol, 15.0 eq.) was then added thereto and a resulting mixture was warmed to 60°C, stirred and reacted for 3 h. Upon product formation and complete consumption of the raw materials as monitored by LC-MS, the reaction was stopped. A resulting reaction solution was concentrated to dryness to yield a crude product. The crude product was purified by silica gel column chromatography (DCM : MeOH (volume ratio) = 50 : 1 to 5 : 1) to yield the product m3-12-3-4 (3.8 g, with a yield of 83%) as a pale yellow viscous liquid.

Step 7: Synthesis of compound m3-12-3-5

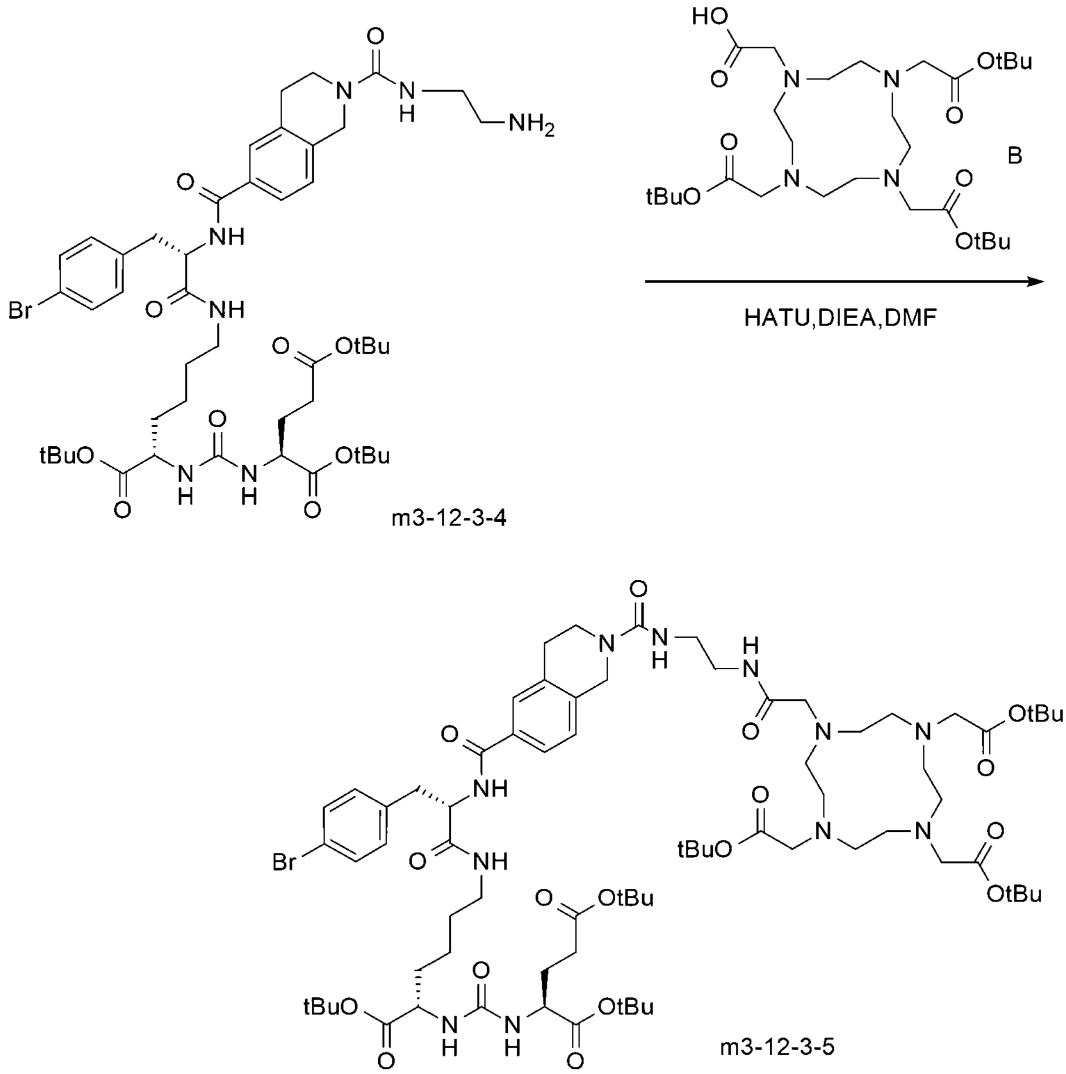


m3-12-3-4

B

m3-12-3-5

At room temperature, raw material m3-12-3-4 (2.0 g, 2.1 mmol, 1.0 eq.) was dissolved in DMF (30 mL) in a 100 mL single-neck flask, and raw material B (1.4 g, 2.5 mmol, 1.2 eq.), DIEA (0.41 g, 3.2 mmol, 1.5 eq.) and HATU (0.95 g, 2.5 mmol, 1.2 eq.) were separately added thereto. A resulting mixture was stirred at room temperature overnight. Upon product formation as monitored by LC-MS, the reaction was stopped. Water and ethyl acetate were then added thereto and a resulting mixture was subjected to extraction and liquid layering. A resulting organic phase was washed with saturated brine, separated and concentrated to obtain a crude product. The crude product was purified through a preparative high-performance liquid phase column (Welch Xtimate C18, 21.2 × 250 mm, 5 μm) to yield the product m3-12-3-5 (1.0 g, with a yield of 31%).

Step 8: Synthesis of compound m3-12-3

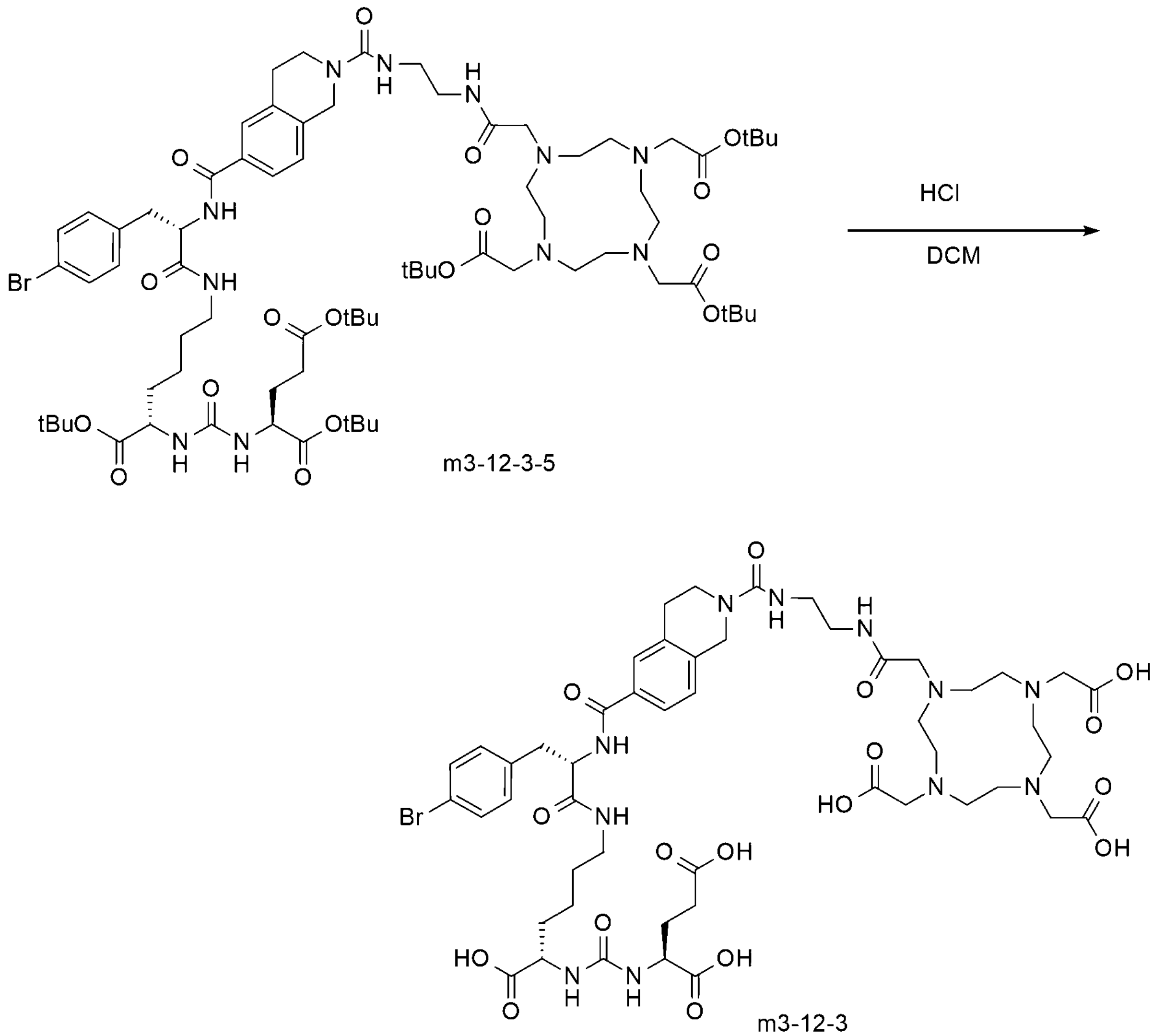


m3-12-3-5

m3-12-3

At room temperature, raw material m3-12-3-5 (240 mg, 0.16 mmol, 1.0 eq.) and DCM (5 mL) were added to a 50 mL single-neck flask, and a resulting mixture was stirred for dissolution. A solution of hydrogen chloride in dioxane (4 M) (12 mL, 48 mmol, 300 eq.) was added thereto and a resulting mixture was stirred at 25°C overnight. Upon product formation and complete consumption of the raw materials as monitored by LC-MS, the reaction was stopped. A resulting reaction solution was concentrated to dryness to yield a crude product. The crude product was purified by a preparative high-performance liquid phase column chromatography (Welch Xtimate C18, 21.2 × 250 mm, 5 μm) to yield the product m3-12-3 (95 mg, with a yield of 51%) as a white solid.

The LC-MS, HPLC and $^{1}$H-NMR spectra of m3-12-3 are shown in FIG. 13, FIG. 14A, and FIG. 15, respectively.

## Example 6 Synthesis of compound m3-12-a

Step 1: Synthesis of compound m3-12a-2

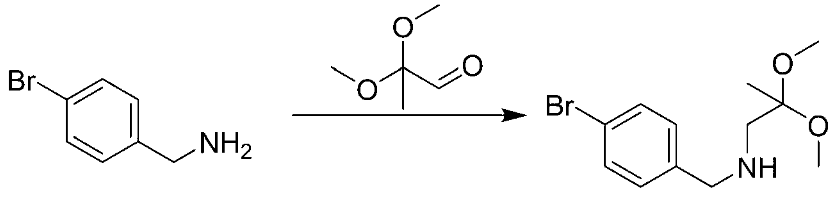

**m3-12a-1** **m3-12a-2**

To a solution of 4-bromobenzylamine (30.0 g, 162 mmol, 1.0 eq) in dichloroethane (DCE) (300 mL), 1,1-dimethoxypropan-2-one (21.0 g, 177 mmol, 1.1 eq) and $MgSO_4$ (60 g) were added. A resulting mixture was stirred at 40°C overnight, and sodium triacetylborohydride (41.2 g, 194.4 mmol, 1.2 eq) was then added thereto, and a resulting mixture was stirred at room temperature for 5 hours. The reaction was quenched by adding saturated aqueous sodium carbonate solution and a resulting reaction mixture was extracted with dichloromethane twice. Resulting organic phases were combined, dried over anhydrous sodium sulfate and subjected to suction filtration. A resulting filtrate was concentrated to yield a yellow oil m3-12a-2 (45 g, with a yield of 96%, and a purity of 80.85%).

Step 2: Synthesis of compound m3-12a-3

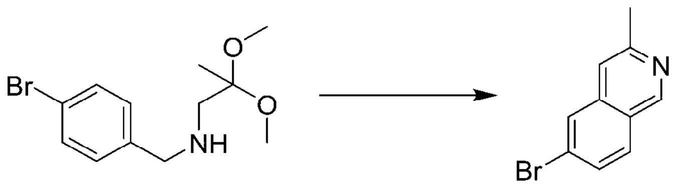

**m3-12a-2** **m3-12a-3**

At room temperature, chlorosulfonic acid (90 mL) was added to a 250 mL single-neck flask. Atmosphere in the single-neck flask was replaced with nitrogen three times, with nitrogen protection. A resulting system was cooled down to -10°C in an ice bath. Raw material m3-12a-2 (45.0 g, 156.15 mmol, 1.0 eq.) was added dropwise. After the addition, a resulting mixture was heated to 100°C and stirred for 10 minutes. Upon new product spot formation and complete consumption of the raw materials as monitored by LC-MS, the reaction was stopped. A resulting reaction solution was cooled down to room temperature and quenched by pouring ice water thereto. 10% aqueous sodium hydroxide solution was added dropwise thereto to adjust a pH value of a resulting mixture to 9. Ethyl acetate was added thereto and a resulting mixture was subjected to extraction. An organic phase was separated, washed with water and saturated aqueous sodium chloride solution, dried over anhydrous sodium sulfate, and concentrated under reduced pressure to dryness to obtain a crude product. The crude product was purified by silica gel column chromatography (PE : EA (volume ratio) = 100 : 1 to 20 : 1) to yield the product m3-12a-3 (8.32 g, with a yield of 24%, and a purity of 96.12%) as a yellow solid.

Step 3: Synthesis of compound m3-12a-4

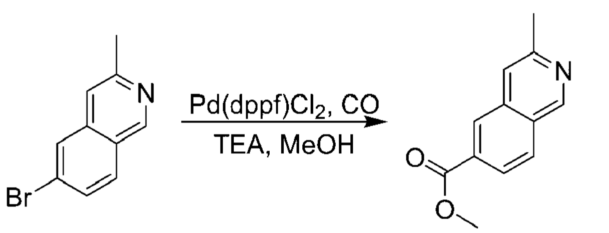


**m3-12a-3** **m3-12a-4**

At room temperature, to a 250 mL single-neck flask filled with raw material m3-12a-3 (7.50 g, 33.77 mmol, 1.0 eq.) dissolved in MeOH (75 mL), $Pd(dppf)Cl_2$ (1.24 g, 1.67 mmol, 0.05 eq.) and triethylamine (TEA) (14.08 mL, 101.31 mmol,

3.0 eq.) were added. Carbon monoxide was introduced thereto and atmosphere in the single-neck flask was replaced with carbon monoxide three times. In the protection of a carbon monoxide balloon, a resulting reaction mixture was heated to 80°C and was stirred overnight. Upon product formation as monitored by LC-MS, the reaction was stopped. A resulting reaction solution was cooled to room temperature and concentrated under reduced pressure to dryness to obtain a crude product. The crude product was purified by silica gel column chromatography (PE : EA (volume ratio) = 100 : 1 to 20 : 1) to yield product m3-12a-4 (6.02 g, with a yield of 89%, and a purity of 98.44%) as a yellow solid. Step 4: Synthesis of compound m3-12a-5

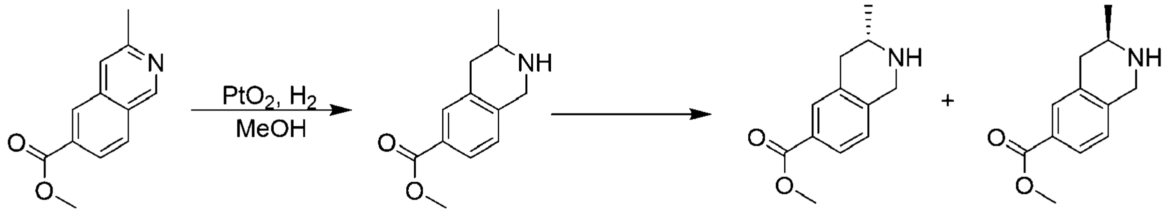


**m3-12a-4** **m3-12a-5** **m3-12a-5a** **m3-12a-5b**

At room temperature, raw material m3-12a-4 (5.90 g, 29.32 mmol) and methanol (60 mL) were added to a 250 mL single-neck flask, and a resulting mixture was stirred for dissolution. Platinum dioxide (0.67 g, 2.93 mmol, 0.1 eq.) was added thereto and atmosphere in the single-neck flask was replaced three times using a hydrogen balloon. A resulting reaction solution was reacted at 25°C overnight. Upon product formation and complete consumption of the raw materials as monitored by LC-MS, the reaction was stopped. A resulting reaction solution was subjected to suction filtration under reduced pressure. A resulting mother liquor was concentrated under reduced pressure to dryness to yield 5.9 g of a racemate, i.e., a crude product. The crude product was purified by preparative normal phase chiral chromatography (CHIRALPAK AD-10) to yield products m3-12a-5a (2.20 g, with a yield of 37%) and m3-12a-5b (2.80 g, with a yield of 47%), both as yellow solids.

Step 5: Synthesis of compound m3-12a-6

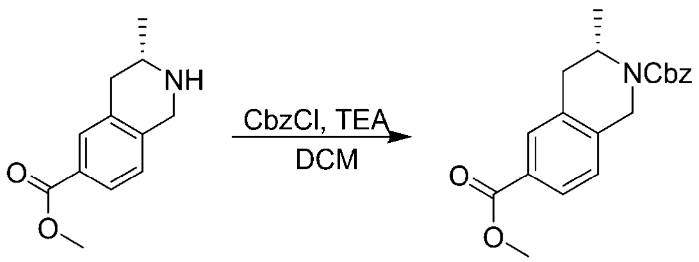


**m3-12a-5a** **m3-12a-6**

At room temperature, raw material m3-12a-5a (2.10 g, 10.23 mmol, 1.0 eq.) was added to a 250 mL single-neck flask, and dichloromethane (21 mL) was added thereto for dissolution. Triethylamine (3.11 g, 30.69 mmol, 3.0 eq.) was added thereto. Atmosphere in the single-neck flask was replaced with nitrogen three times, with nitrogen protection. In an ice bath, benzyl chloroformate (3.49 g, 20.46 mmol, 2.0 eq.) was added dropwise thereto. After the addition, a resulting mixture was stirred at room temperature for 1 hour. Upon new product spot formation and complete consumption of the raw materials as monitored by LC-MS, the reaction was stopped. The reaction was quenched by adding ice water thereto, and a resulting reaction mixture was then subjected to extraction and liquid layering. A resulting organic phase was separated, washed with water, dried over anhydrous sodium sulfate and concentrated under reduced pressure to dryness to obtain a crude product. The crude product was purified by silica gel column chromatography (PE : EA (volume ratio) = 100 : 1 to 50 : 1) to yield product m3-12a-6 (2.40 g, with a yield of 69%, and a purity of 100%) as a whitish solid.

Step 6: Synthesis of compound m3-12a-7

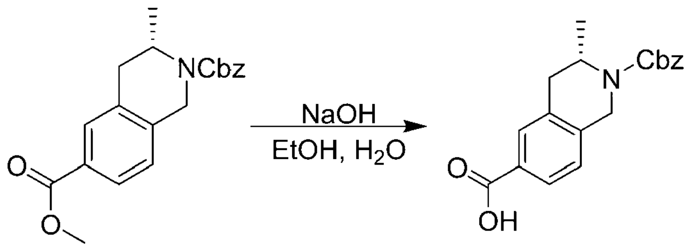


m3-12a-6 m3-12a-7

At room temperature, raw material m3-12a-6 (2.40 g, 7.07 mmol, 1.0 eq.) was added to a 100 mL single-neck flask. Methanol (24 mL) was added thereto for dissolution. An aqueous solution (12 mL) of sodium hydroxide (0.85 g, 21.22 mmol, 3.0 eq.) was added dropwise thereto and a resulting mixture was stirred at room temperature overnight. Upon new product spot formation and complete consumption of the raw materials as monitored by LC-MS, the reaction was stopped. 2 mol/L aqueous hydrochloric acid solution was added dropwise thereto until a resulting reaction mixture was adjusted to pH = 5. Ethyl acetate was added thereto and a resulting mixture was subjected to extraction. An organic phase was separated, washed with water and saturated aqueous sodium chloride solution, dried over anhydrous sodium sulfate, and concentrated under reduced pressure to dryness to yield product m3-12a-7 (2.12 g, with a yield of 92%, and a purity of 98.41%) as a whitish solid.

Step 7: Synthesis of compound m3-12a-8

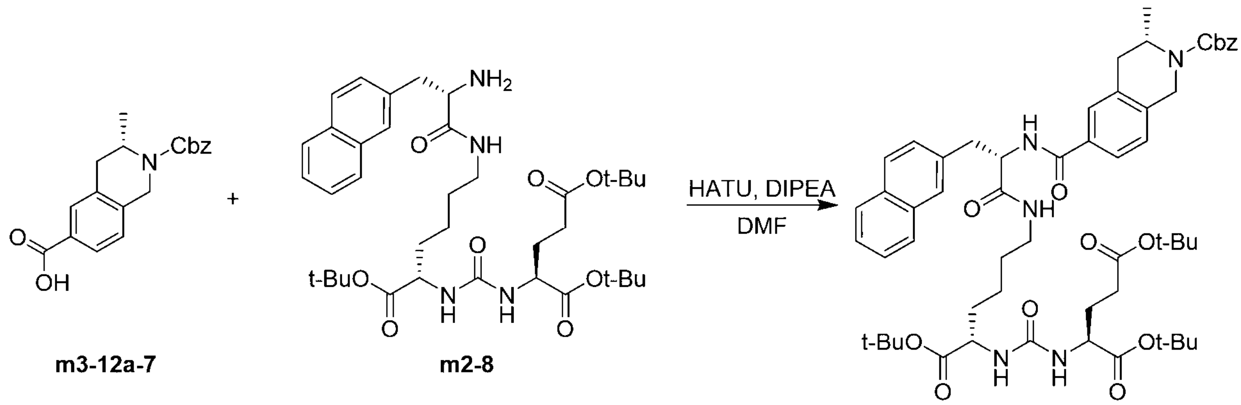


m3-12a-7 m2-8 m3-12a-8

At room temperature, raw material m2-8 (3.30 g, 4.82 mmol, 1.0 eq.) was added to a 100 mL single-neck flask. Anhydrous N,N-dimethylformamide (33 mL) was added thereto for dissolution. m3-12a-7 (2.04 g, 6.26 mmol, 1.3 eq.), HATU (2.75 g, 7.23 mmol, 1.5 eq.) and DIPEA (1.25 g, 9.64 mmol, 2.0 eq.) were added thereto. Atmosphere in the single-neck flask was replaced three times, with nitrogen protection. A resulting mixture was stirred at room temperature overnight. Upon new product spot formation and complete consumption of the raw materials as monitored by LC-MS, the reaction was stopped. The reaction was quenched by pouring ice water thereto and ethyl acetate was added thereto and a resulting mixture was subjected to extraction and liquid layering. An organic phase was separated, washed with water, dried over anhydrous sodium sulfate, and concentrated under reduced pressure to dryness to obtain a crude product. The crude product was purified by silica gel column chromatography (DCM : MeOH (volume ratio) = 100 : 1 to 50 : 1) to yield product m3-12a-8 (3.00 g, with a yield of 63%, and a purity of 92.63%) as a yellow liquid.

Step 8: Synthesis of compound m3-12a-9

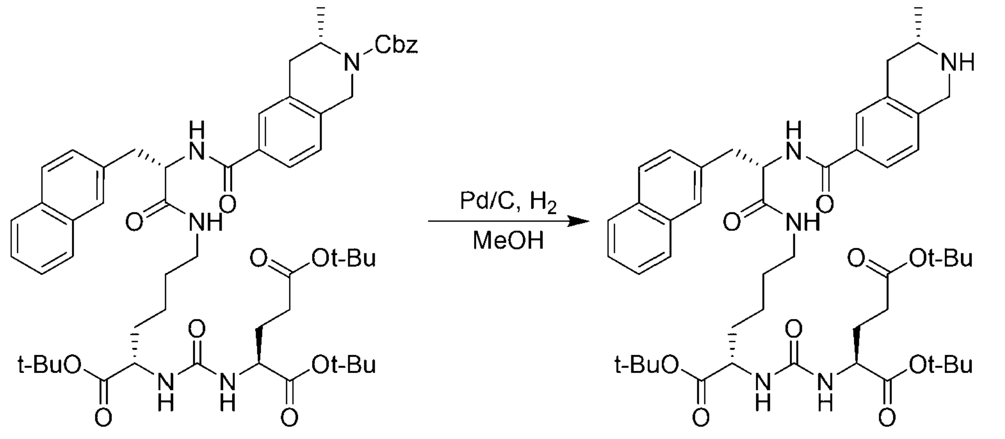


**m3-12a-8** **m3-12a-9**

At room temperature, raw material m3-12a-8 (2.90 g, 2.92 mmol, 1.0 eq.) was added to a 100 mL single-neck flask and methanol (30 mL) was added thereto for dissolution. 10% (mass percentage) palladium on carbon (0.31 g, 0.29 mmol, 0.1 eq.) was added thereto, and atmosphere in the single-neck flask was replaced with hydrogen using a hydrogen gas balloon, with hydrogen protection. A resulting mixture was stirred at room temperature overnight. Upon new product spot formation and complete consumption of the raw materials as monitored by LC-MS, the reaction was stopped. A resulting reaction solution was subjected to suction filtration under reduced pressure and a resulting mother liquor was concentrated to dryness to obtain a crude product. The crude product was purified by silica gel column chromatography (DCM : MeOH (volume ratio) = 50 : 1 to 30 : 1) to yield product m3-12a-9 (1.94 g, with a yield of 77%, and a purity of 100%) as a yellow solid.

Step 9: Synthesis of compound m3-12a-10

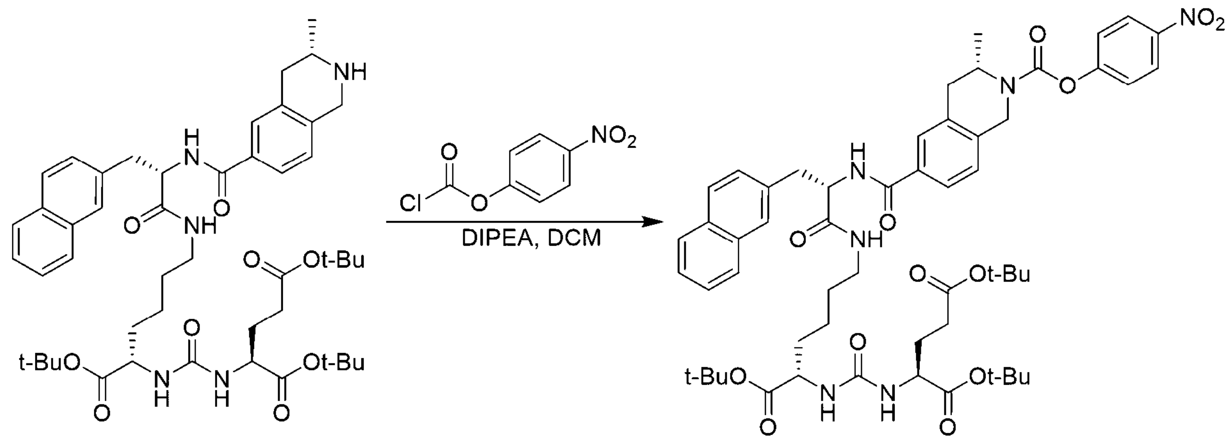


**m3-12a-9** **m3-12a-10**

At room temperature, raw material m3-12a-9 (1.90 g, 2.21 mmol, 1.0 eq.) was added to a 100 mL single-neck flask. Anhydrous dichloromethane (20 mL) was added thereto for dissolution. DIPEA (0.57 g, 4.43 mmol, 2.0 eq.) was added thereto. Atmosphere in the single-neck flask was replaced with nitrogen three times, with nitrogen protection. In an ice bath, p-nitrophenyl chloroformate (0.67 g, 3.32 mmol, 1.5 eq.) was added dropwise thereto. After the addition, a resulting mixture was stirred at room temperature for 1 hour. Upon new product spot formation and complete consumption of the raw materials as monitored by LC-MS, the reaction was stopped. The reaction was quenched by pouring ice water thereto, and dichloromethane was added thereto and a resulting mixture was subjected to extraction and liquid layering. An organic phase was separated, washed with water, dried over anhydrous sodium sulfate, and concentrated under reduced pressure to dryness to yield product m3-12a-10 (2.20 g, with a yield of 97%, and a purity of 88.38%) as a yellow liquid.

Step 10: Synthesis of compound m3-12a-11

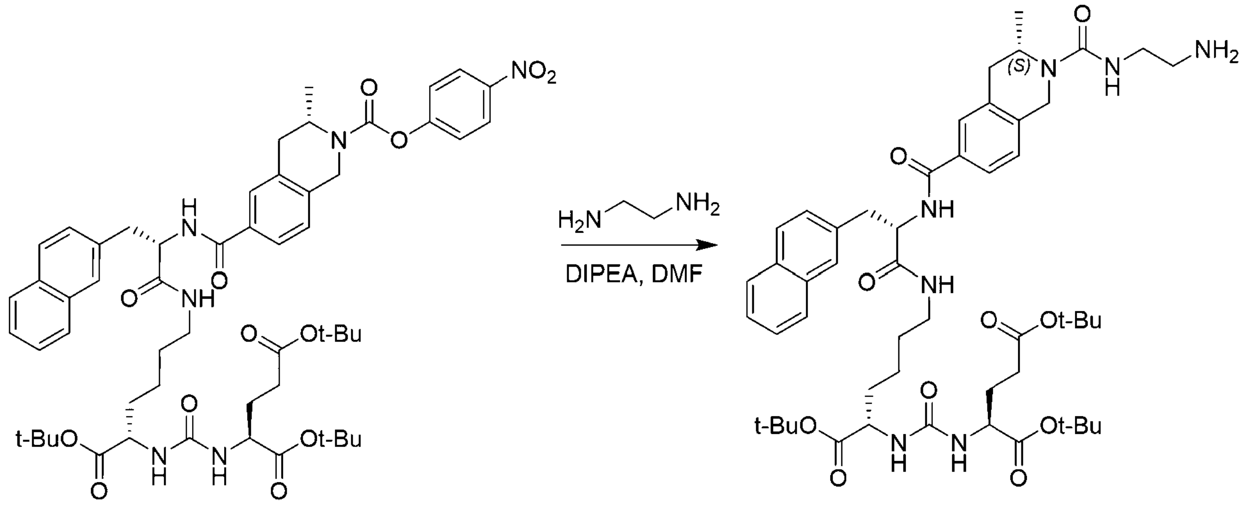


m3-12a-10

m3-12a-11

At room temperature, raw material m3-12a-10 (2.20 g, 2.15 mmol, 1.0 eq.) was added to a 100 mL single-neck flask and anhydrous N,N-dimethylformamide (22 mL) was added thereto for dissolution. Ethylenediamine (1.57 g, 21.50 mmol, 10.0 eq.) and DIPEA (0.56 g, 4.30 mmol, 2.0 eq.) were added thereto, and atmosphere in the single-neck flask was replaced with nitrogen three times, with nitrogen protection. A resulting mixture was heated to 60°C and stirred for 1 hour. Upon new product spot formation and complete consumption of the raw materials as monitored by LC-MS, the reaction was stopped. A resulting reaction solution was cooled down to room temperature and the reaction was quenched by pouring ice water thereto. Ethyl acetate was added and a resulting mixture was subjected to extraction. An organic phase was separated, washed with water, dried over anhydrous sodium sulfate, and concentrated under reduced pressure to dryness to obtain a crude product. The crude product was purified by silica gel column chromatography (DCM : MeOH (volume ratio) = 50 : 1 to 10 : 1) to yield product m3-12a-11 (1.90 g, with a yield of 94%, and a purity of 92.07%) as a yellow solid.

Step 11: Synthesis of compound m3-12a-12

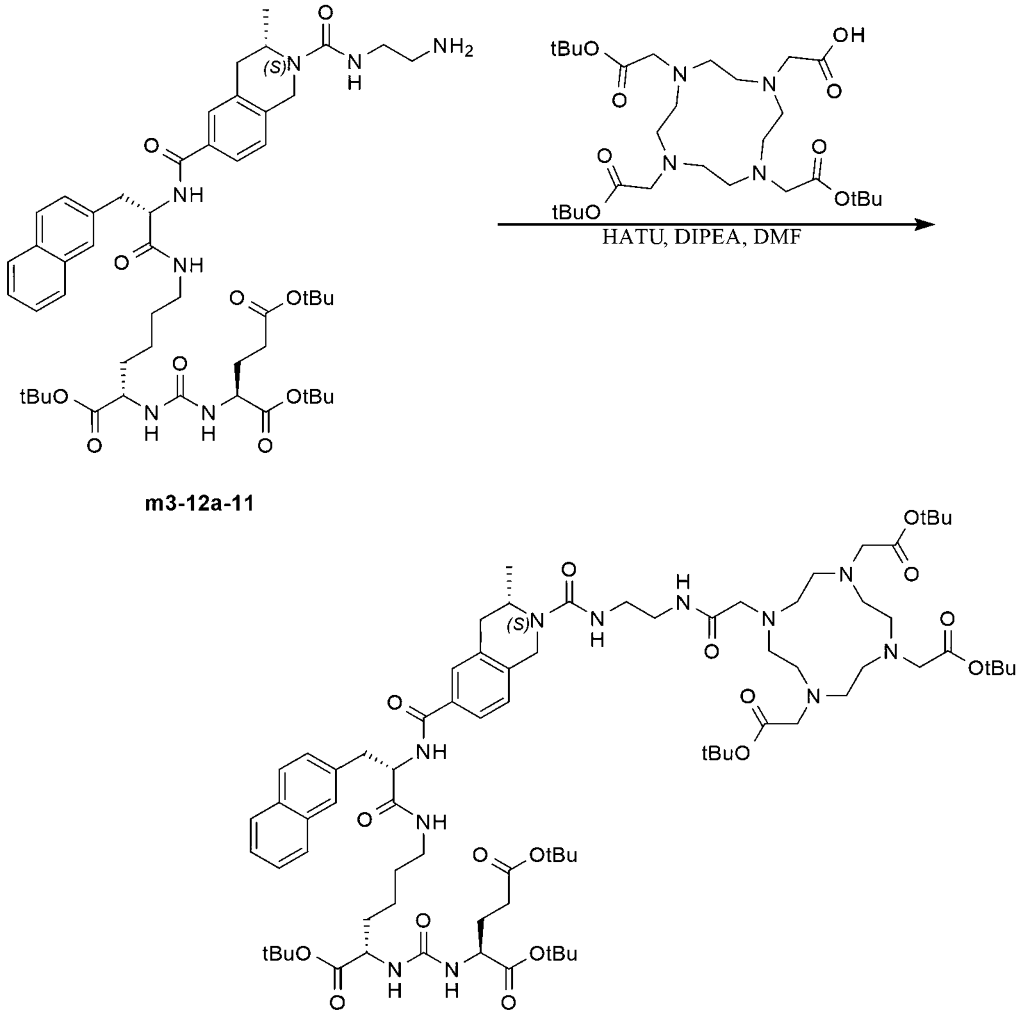


m3-12a-11

m3-12a-12

At room temperature, raw material m3-12a-11 (900 mg, 0.95 mmol, 1.0 eq.) was added to a 100 mL single-neck flask. Anhydrous N,N-dimethylformamide (10 mL) was added thereto for dissolution. Tri-tert-butyl 1,4,7,10-tetraazacyclododecane-1,4,7,10-tetraacetic acid (819 mg, 1.43 mmol, 1.5 eq.), HATU (544 mg, 1.43 mmol, 1.5 eq.) and DIPEA (246 mg, 1.91 mmol, 2.0 eq.) were added thereto, and atmosphere in the single-neck flask was replaced with nitrogen three times, with nitrogen protection. The mixture was stirred at room temperature overnight. Upon new product spot formation and complete consumption of the raw materials as monitored by LC-MS, the reaction was stopped. The reaction was quenched by pouring ice water thereto, and ethyl acetate was added thereto and a resulting mixture was subjected to extraction and liquid layering. An organic phase was separated, washed with water, dried over anhydrous sodium sulfate, and concentrated under reduced pressure to dryness to obtain a crude product. The crude product was purified by silica gel column chromatography (DCM : MeOH (volume ratio) = 5 : 1 to 2 : 1) to yield crude product m3-12a-12, followed by preparative high-performance liquid phase chromatography ((Welch Xtimate C18, 21.2 × 250 mm, 5 μm)) to yield m3-12a-12 (700 mg, with a yield of 49%) as a white solid.

Step 12: Synthesis of compound m3-12a

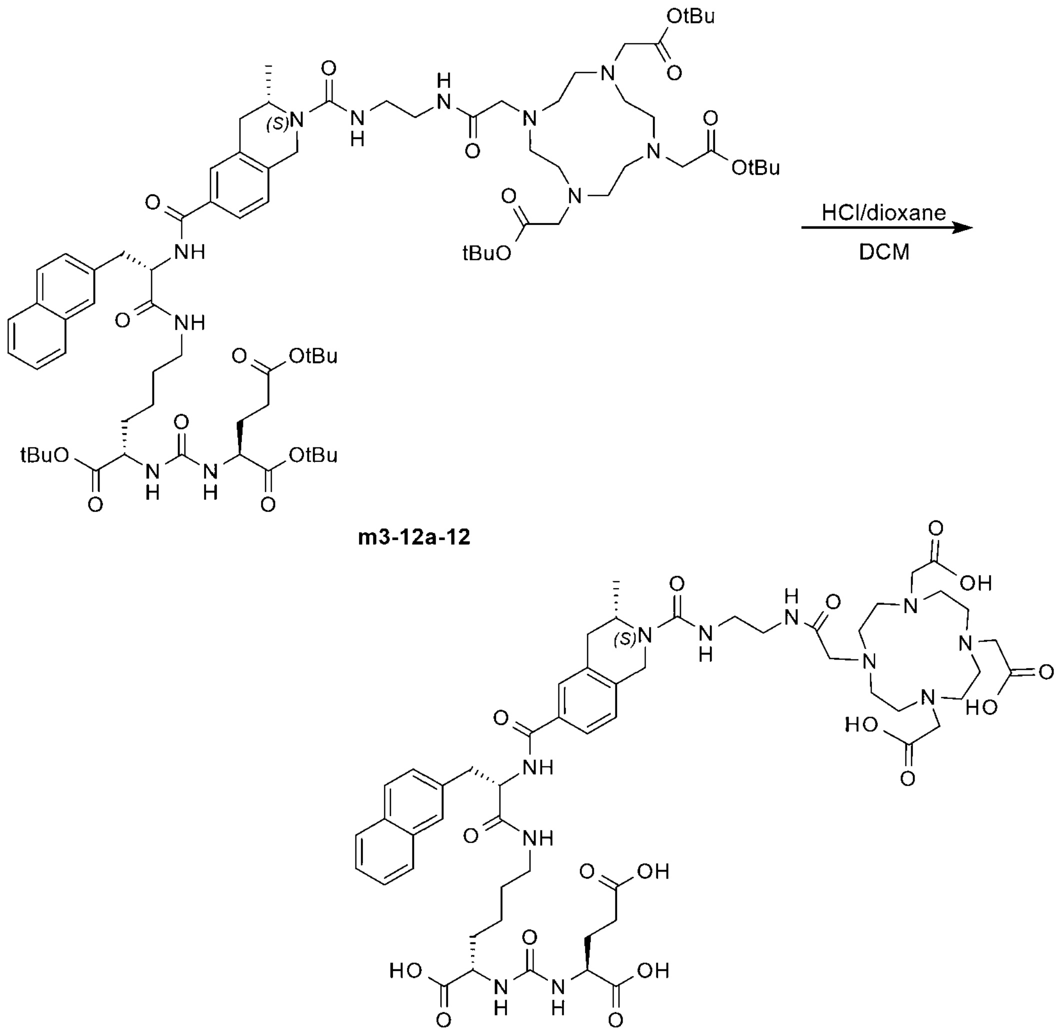


m3-12a-12

m3-12a

At room temperature, raw material m3-12a-12 (350 mg, 0.23 mmol, 1.0 eq.) was added to a 100 mL single-neck flask and dichloromethane (10 mL) was then added thereto for dissolution. A solution of hydrogen chloride in dioxane (10 mL, 4 mol/L) was added dropwise and under nitrogen protection, and a resulting mixture was stirred at room temperature for 8 hours. Upon new product spot formation and complete consumption of the raw materials as monitored by LC-MS, the reaction was stopped. A resulting reaction solution was concentrated under reduced pressure and then subjected to preparative high-performance liquid phase chromatography (Welch Xtimate C18, 21.2 × 250 mm, 5 μm) to yield m3-12a (210 mg, with a yield of 49%, and a purity of 95.65%) as a white solid. The purity was determined to be up to standard.

The LC-MS, HPLC and $^1$H-NMR spectra of m3-12-a are shown in FIG. 16, FIG. 17A, and FIG. 18, respectively.

**Example 7 Biological activity test**

1. LogP value

The Log P values of the test compounds and control compounds in 1-octanol/PBS (pH 1.0) were determined using the shake flask method. The results are shown in Tables 1-3.

1.1 Experimental reagents:

100 mM Sodium phosphate buffer (pH = 1.0), and n-octanol.

1.2 Experimental method:

(1) To a 96-well deep-well plate, 15 μL of a stock solution (30 mM) of each test compound or control compound prepared from DMSO was added. Blank dimethyl sulfoxide was then added thereto to bring the total volume to 45 μL, yielding a working concentration of 10 mM for each test compound or control compound.
(2) 15 μL of the 10 mM working solutions was sequentially transferred into respective 96-well deep-well plates (LogP plate). To each vial of the LogP plate, 800 μL of saturated n-octanol was added, followed by adding 200 μL of saturated phosphate buffer. The LogP plate was sealed with a 96-well plate cover, transferred to an Eppendorf Thermomixer Comfort plate shaker, and shaken at 2000 rpm at 25°C for 2 h.
(3) The samples were centrifuged at 3220 g for 30 min at 25°C for phase separation. Using a pipette and syringe, about 100 μL of each phase (the n-octanol phase and the buffer phase) was transferred to a new 96-well plate. 5 μL of the n-octanol sample was transferred to a new 96-well plate, followed by adding 495 μL of a mixture of acetonitrile and $H_2O$ (acetonitrile : $H_2O$ = 1 : 1) to obtain a 100-fold diluted n-octanol sample. The sample was eddied at 1000 rpm for 5 min.
(4) 50 μL of the 100-fold diluted n-octanol sample was transferred to a new 96-well plate, followed by adding 450 μL of a mixture of acetonitrile and $H_2O$ (acetonitrile: $H_2O$ = 1 : 1) containing an internal standard, yielding a 1000-fold diluted n-octanol sample. The sample was eddied at 1000 rpm for 5 min.
(5) 50 μL of the buffer sample was transferred to a new 96-well plate, followed by adding 450 μL of a mixture of acetonitrile and $H_2O$ (acetonitrile : $H_2O$ = 1 : 1) containing an internal standard, yielding a 10-fold diluted buffer sample. The sample was eddied at 1000 rpm for 5 min.
(6) The samples were analyzed by LC-MS/MS.

1.3 Data analysis:

The LogP for the compound was calculated according to the following equation:

$$LogP = Log[(AR_{Oct} \times DF)/(AR_{Buffer} \times DF);$$

$AR_{Oct}$ refers to the peak area ratio of the test compound in the n-octanol phase;
$AR_{Buffer}$ refers to the peak area ratio of the test compound in the buffer phase; and
DF refers to dilution fold.

Table 1. Measured LogP values of test compounds and control compounds

| Compound | LogP |
|---|---|
| Nicardipine | 1.29 |
| Progesterone | 3.85 |
| PSMA-617 | < -2.67 |
| m3-12a | < -3.52 |
| m3-12-3 | < -2.82 |

When the test compound was not detected in the octanol phase (below the limit of detection), the limit of detection value was used as the peak area for cut-off value calculation.

Table 2. Measured LogP values of test compounds and control compounds

| Compound | LogP |
|---|---|
| Nicardipine | 1.46 |
| Progesterone | 3.71 |
| PSMA-617 | < -4.02 |
| m3-12 | < -4.08 |

When the test compound was not detected in the octanol phase (below the limit of detection), the limit of detection value was used as the peak area for cut-off value calculation.

Table 3. Measured LogP values of test compounds and control compounds

| Compound | LogP |
|---|---|
| Nicardipine | 1.35 |
| Progesterone | 3.79 |
| PSMA-617 | < -3.55 |
| m3-19 | < -2.65 |
| m3-12-1 | < -3.39 |

When the test compound was not detected in the octanol phase (below the limit of detection), the limit of detection value was used as the peak area for cut-off value calculation.

The LogP test results across three batches recorded in Tables 1-3 are shown below:

PSMA-617 (< -2.67) vs m3-12a (< -3.52), m3-12-3 (< -2.82);

PSMA-617 (< -4.02) vs m3-12 (< -4.08);

PSMA-617 (< -3.55) vs m3-19 (< -2.65), m3-12-1 (< -3.39).

The test results for the compounds of the present disclosure in each batch are at the same level as those for PSMA-617 (i.e., within three times the systematic error), indicating that they achieve logP properties similar to PSMA-617. This provides a basis for proceeding with subsequent biological tests.

2. Enzymatic affinity

The binding of the test compounds and control compounds to PSMA was determined using a fluorometric assay. The results are shown in Tables 4-7.

2.1 Experimental reagents:

PSMA protein, NAAG substrate, OPA.

2.2 Experimental method:

(1) To a 384-well dilution plate, 60 μL of a 10 μM solution of a test compound or a control compound was added. A resulting solution was then serially diluted with DMSO at a ratio of 1 : 3 to 10 concentration points.
(2) 0.1 μL of the solutions with different concentrations were transferred to a 384-well assay plate, with two wells replicating for per concentration.
(3) 5 μL of the PSMA enzyme working solution (10 nM) was added to the 384-well plate. The plate was centrifuged at 1000 rpm for 1 minute and incubated at 25°C for 10 min.
(4) The reaction was initiated by adding 5 μL of the NAAG substrate working solution (10 μM). The plate was centrifuged at 1000 rpm for 1 minute and incubated at 25°C for 60 min.
(5) The reaction was initiated by adding 5 μL of the OPA working solution (4 mg/mL). The plate was centrifuged at 1000 rpm for 1 minute and incubated at 25°C for 15 min.
(6) Fluorescence signals were read using a BMG plate reader at Ex 330 nm and Em 450 nm.

2.3 Data analysis:

Percentage inhibition (%) for compound wells = 100*(Average of high concentration control group-compound well value)/(Average of high concentration control group-Average of low concentration control group),
High concentration control group: DMSO and enzyme;
Low concentration control group: DMSO and buffer;

$$S/B = \text{Average of high concentration control group/Average of low concentration control group;}$$

CV% (low concentration control group) = 100*(SD of low concentration control group/Average of low concentration control group);

CV% (high concentration control group) = 100*(SD of high concentration control group/Average of high concentration control group);

Z' = 1-3* (SD of low concentration control group + SD of high concentration control group)/(Average of high concentration control group-Average of low concentration control group);

the $IC_{50}$ values were fitted using a nonlinear regression equation with XLfit 5.5.0:

$$Y = \text{Bottom} + (\text{Top-Bottom})/(1 + 10\text{^}((\text{LogIC}_{50}\text{-X}) * \text{HillSlope})),$$

where X: Logarithm of the compound concentration;
Y: Percentage inhibition (%);
Top and Bottom refer to fluorescence value of the high concentration control group and fluorescence value of the low concentration control group, respectively (expressed in the same unit as Y);
log$IC_{50}$: in the same logarithmic unit as X; and
HillSlope: slope factor or slope.

Table 4. Test results of the inhibition of test compounds and control compounds against PSMA

| Compound | 30 min | | | | | Average $IC_{50}$ of reference compound | Proportion |
|---|---|---|---|---|---|---|---|
| | Maximum inhibition % | Relative value | Absolute value | | | | |
| | | $IC_{50}$ (nM) | $IC_{50}$ (nM) | $IC_{80}$ (nM) | IC90 (nM) | | |
| PSMA-617 | 101.16 | 4.14 | 4.27 | 8.76 | 13.01 | 3.22 | 1.29 |
| PSMA-617-HG | 101.31 | 4.07 | 4.15 | 7.33 | 10.03 | | |
| m3-12 | 102.67 | 2.24 | 2.44 | 4.79 | 6.99 | | |

Table 5. Test results of the inhibition of test compounds and control compounds against PSMA

| Compound | 30 min | | | | | Average $IC_{50}$ of reference compound | Proportion |
|---|---|---|---|---|---|---|---|
| | Maximum Inhibition % | Relative value | Absolute value | | | | |
| | | $IC_{50}$ (nM) | $IC_{50}$ (nM) | $IC_{80}$ (nM) | $IC_{90}$ (nM) | | |
| PSMA-617 | 102.35 | 2.24 | 1.93 | 3.63 | 4.98 | 3.22 | 0.69 |
| m3-18 | 101.63 | 3.98 | 3.79 | 7.85 | 11.55 | | |

Table 6. Test results of the inhibition of test compounds and control compounds against PSMA

| Compound | Bottom | Top | $IC_{50}$ (nM) | Slope |
|---|---|---|---|---|
| PSMA-617 | 10.1 | 97.8 | 1.59 | 2.31 |
| PSMA-617-HG | 4.53 | 97.4 | 2.14 | 2.10 |
| m3-12a | 7.18 | 98.7 | 1.73 | 2.26 |
| m3-12-3 | 3.84 | 99.5 | 2.28 | 1.50 |

Table 7. Test results of the inhibition of test compounds and control compounds against PSMA

| Compound | Bottom | Top | $IC_{50}$ (nM) | Slope |
|---|---|---|---|---|
| PSMA-617 | -8.48 | 94.4 | 2.45 | 2.14 |
| PSMA-617-HG | -6.71 | 94.5 | 2.84 | 2.04 |
| m3-19 | -6.74 | 96.9 | 2.00 | 1.81 |
| m3-12-1 | -8.37 | 94.2 | 2.10 | 2.15 |

The enzymatic experimental results of the compound of the present disclosure and PSMA-617 are as follows:

PSMA-617 (4.14 nM) vs m3-12 (2.24 nM);

PSMA-617 (2.24 nM) vs m3-18 (3.98 nM);

PSMA-617 (1.59 nM) vs m3-12a (1.73 nM), m3-12-3 (2.28 nM);

PSMA-617 (2.45 nM) vs m3-19 (2.00 nM), m3-12-1 (2.10 nM).

The results from the aforementioned four batches demonstrate that the compounds of the present disclosure exhibit an enzymatic affinity for PSMA that is at a comparable level to that of the control compound PSMA-617, with all data falling within three times the systematic error. This provides a foundation for the subsequent screening of compounds with superior uptake and internalization properties.

3. Cellular uptake

Cellular uptake experiments were carried out for the test compounds and control compounds using LNCap cells. The results are shown in Tables 8-11.

3.1 Experimental reagents:

Phenol red-free RPMI 1640 medium, 10% FBS, Glu-490 probe, PBS buffer, 0.25% trypsin without EDTA.

3.2 Experimental method:

(1) Cells were resuspended in phenol red-free RPMI 1640 medium containing 10% FBS in a 48-well plate and then cultured at 37°C with 5% $CO_2$ for 24 h.
(2) Test compounds were added to the cells and the plate was incubated at 4°C for 30 min.
(3) Glu-490 was added to the cells to a final concentration of 0.3 μM and the plate was incubated at 4°C for 1 h.
(4) The cells were rinsed with pre-chilled PBS at 4°C.
(5) The cells were collected using 0.25% trypsin without EDTA, and centrifuged at 400 g for 5 min, and the supernatant was discarded.
(6) The cells were resuspended in 100 μL of PBS without washing, and the sample was detected using a CytoFLEXS flow cytometer (Beckman). Analysis was performed using ModFit software.

3.3 Data analysis:

H = Ave (DMSO);
L = Ave (without Glu490);
SD (H) = STDEV (DMSO);
SD (L) = STDEV (without Glu490);

$$CV\% (DMSO) = 100* (SD_H/Ave_H);$$

$$CV\% (without Glu490) = 100*(SD_L/Ave_L);$$

$$Z' = 1-3*(SD_H + SD_L)/(Ave_H - Ave_L);$$

Percentage Inhibition% = (sample-Ave_L)/(Ave_H-Ave_L);
the $IC_{50}$ values were fitted using a nonlinear regression equation:

$$Y = Bottom + (Top-Bottom)/(1 + 10^((LogIC50-X) * HillSlope));$$

X: Concentration,
Y: Inhibition rate,
Top and Bottom refer to the fluorescence values corresponding to H = Ave (DMSO) and L = Ave (without Glu490), respectively,
HillSlope: Slope factor or slope.

Table 8. Uptake results of test compounds and control compounds in LNCap cells

| Compound | Initial concentration (nM) | Dilution fold | Minimum inhibition% | Maximum inhibition% | Slope | R_$IC_{50}$ (nM) | A_$IC_{50}$ (nM) |
|---|---|---|---|---|---|---|---|
| PSMA-617 | 10000 | 4 | 6.00 | 76.30 | 0.37 | 9.42 | 0.36 |
| m3-19 | 10000 | 4 | 5.11 | 77.75 | 0.31 | 6.69 | 32.13 |
| m3-12-1 | 10000 | 4 | -26.87 | 79.94 | 0.43 | 289.27 | 442.11 |

Table 9. Uptake results of test compounds and control compounds in LNCap cells

| Compound | Concentration (nM) | Inhibition%_ 1 | Inhibition%_ 2 | Inhibition%_average |
|---|---|---|---|---|
| PSMA-617 | 2500 | 77.48 | 68.06 | 72.77 |
| m3-12a | 2500 | 75.77 | 67.69 | 71.73 |
| m3-12-3 | 2500 | 62.12 | 58.73 | 60.42 |

Table 10. Uptake results of test compounds and control compounds in LNCap cells

| Compound | Initial concentration (nM) | Dilution fold | Minimum inhibition % | Maximum inhibition % | Slope | R_ $IC_{50}$ (nM) | A_$IC_{50}$ (nM) |
|---|---|---|---|---|---|---|---|
| PSMA-617 | 10000 | 5 | 8.90 | 88.84 | 0.47 | 4.16 | 7.15 |
| m3-12 | 10000 | 5 | 23.47 | 85.61 | 0.54 | 5.00 | 4.19 |
| m3-12a | 10000 | 5 | -21.83 | 93.35 | 0.38 | 5.82 | 35.00 |
| m3-12-3 | 10000 | 5 | -6.63 | 78.96 | 0.51 | 490.80 | 1370.6 8 |

Table 11. Uptake results of test compounds and control compounds in LNCap cells

| Compound | Parallel 1 | Parallel 2 | 4 h uptake |
|---|---|---|---|
| PSMA-617 | 4.35% | 5.98% | 5.17%±1.15% |
| m3-12 | 6.20% | 9.52% | 7.86%±2.35% |

The uptake rate results at a single concentration of 2.5 μM, as tested by the FACS method, are as follows: PSMA-617 (72.77%) vs m3-12a (71.73%), m3-12-3 (60.42%).

The results demonstrate a significantly lower uptake rate for m3-12-3 compared with the control compound PSMA-617, whereas m3-12a exhibits a comparable uptake level to PSMA-617. The $IC_{50}$ of compound uptake rate was then calculated using multiple concentration points to accurately distinguish differences in uptake rate among compounds.

The $IC_{50}$ results of the uptake rate of the compounds of the present disclosure at multiple concentration points, tested using the FACS method, are as follows:

PSMA-617 (9.42 nM) vs m3-19 (6.69 nM), m3-12-1 (289.27 nM);
PSMA-617 (4.16 nM) vs m3-12 (5.00 nM), m3-12a (5.82 nM), m3-12-3 (490.80 nM).

The 4 h uptake rate results, tested by the LC-MS/MS method, are as follows:
PSMA-617 (5.17%) vs m3-12 (7.86%)

The $IC_{50}$ results of the uptake rate of the compounds of the present disclosure at multiple concentration points, tested using the FACS method, demonstrate that m3-19, m3-12, and m3-12a are at a comparable level to that of the control compound PSMA-617 ($IC_{50}$ values within a 3-fold range). The uptake rate $IC_{50}$ values for m3-12-1 and m3-12-3 are larger by one order of magnitude higher, compared with PSMA-617. The uptake potency of m3-19, m3-12, and m3-12a in LnCap prostate cancer cells reaches the level of the control compound PSMA-617. Furthermore, as tested by the LC-MS/MS method, the uptake rate result of m3-12 is also at a comparable level to that of the control compound PSMA-617. Therefore, the subsequent focus was on evaluating whether the downstream internalization rates of m3-19, m3-12, and m3-12a were superior to that of the control compound.

4. Cellular internalization

Cellular internalization experiments of the test compounds and control compounds were carried out using LNCap cells. The results are shown in Tables 12-14.

4.1 Experimental reagents:

HHBS buffer, Phenol red-free RPMI 1640 medium, 10% FBS, PBS buffer, 0.25% trypsin without EDTA.

4.2 Experimental method:

LNCap cells in the logarithmic growth phase were taken, digested and centrifuged, and resuspended in HHBS buffer ($1 \times 10^6$ cells/mL). 200 μL of the cell suspension was then separately placed into each EP tube. The drug (final concentration: 1 μM) and the antibody (200×) were separately added to the tubes, followed by co-incubation at 37°C for 2 h. After incubation, the cells were washed by adding pre-chilled HHBS buffer and centrifuged. An acid wash solution pre-chilled at 4°C was then added for washing and centrifugation. The cells were then fixed with paraformaldehyde at 4°C for 30 min, centrifuged, and washed once with HHBS buffer. After centrifugation, the cells were resuspended in HHBS buffer for flow cytometry.

4.3 Data processing:

$$\text{Cellular internalization\%} = [1-((A2-A0)/(A1-A0))] \times 100\%,$$

A0: Background values of cells without antibody or compound,
A1: Fluorescence values after incubation with antibody only,
A2: Fluorescence values after co-incubation with both antibody and compound.

Table 12. Internalization results of test compounds and control compounds in LNCap cells

| Sample name | Geometric mean (Alexa Fluor 488-A :: null) | | Internalization rate | Average |
|---|---|---|---|---|
| Blank parallel 1 | 49.4 | 43 | / | |
| Blank parallel 2 | 36.6 | | / | |
| BC parallel 1 | 939 | 1066.5 | 12.46% | 0.00% |
| BC parallel 2 | 1194 | | -12.46% | |
| PSMA-617 parallel 1 | 490 | | 56.33% | 56.86% |
| PSMA-617 parallel 2 | 479 | | 57.40% | |
| m3-12a parallel 1 | 394 | | 65.71% | 67.22% |
| m3-12a parallel 2 | 363 | | 68.73% | |
| m3-12-3 parallel 1 | 425 | | 62.68% | 60.04% |
| m3-12-3 parallel 2 | 479 | | 57.40% | |

Table 13. Internalization results of test compounds and control compounds in LNCap cells

| Sample name | Geometric mean (Alexa Fluor 488-A :: null) | | Internalization rate | Average |
|---|---|---|---|---|
| Blank parallel 1 | 57.4 | 53.7 | / | |
| Blank parallel 2 | 50 | | / | |
| BC parallel 1 | 1602 | 1590 | -0.78% | 0.00% |
| BC parallel 2 | 1578 | | 0.78% | |
| PSMA-617 parallel 1 | 746 | | 54.94% | 58.29% |
| PSMA-617 parallel 2 | 643 | | 61.64% | |
| m3-12-1 parallel 1 | 672 | | 59.75% | 59.30% |
| m3-12-1 parallel 2 | 686 | | 58.84% | |
| m3-19 parallel 1 | 510 | | 70.30% | 71.11% |
| m3-19 parallel 2 | 485 | | 71.93% | |

Table 14. Internalization results of test compounds and control compounds in LNCap cells

| Sample name | Geometric mean (Alexa Fluor 488-A :: null) | Internalization rate | Average |
|---|---|---|---|
| BC 2 h parallel 1 | 30.9 | / | / |
| BC 2 h parallel 2 | 33.5 | / | / |
| BC 24 h | 47 | / | / |
| PSMA-617 2 h parallel 1 | 20.2 | 34.63% | 39.25% |
| PSMA-617 2 h parallel 2 | 18.8 | 43.88% | |
| PSMA-617 24 h parallel 1 | 45.6 | 2.98% | -0.32% |
| PSMA-617 24 h parallel 2 | 48.7 | -3.62% | |
| m3-12 2 h parallel 1 | 20 | 35.28% | 37.64% |
| m3-12 2 h parallel 2 | 20.1 | 40.00% | |
| m3-12 24 h parallel 1 | 45 | 4.26% | 2.77% |
| m3-12 24 h parallel 2 | 46.4 | 1.28% | |

Result comparisons of the internalization rate experiment between the compounds of the present application and the control compound PSMA-617 are shown as follows:

PSMA-617 (56.86%) vs m3-12a (67.22%), m3-12-3 (60.04%);
PSMA-617 (58.29%) vs m3-12-1 (59.30%), m3-19 (71.11%);
PSMA-617 (39.25%) vs m3-12 (37.64%).

Comparison of internalization rates between the compounds of the present disclosure across multiple batches and the control compound PSMA-617 demonstrates that m3-12a, and m3-19 exhibit a clear higher trend relative to the control compound, whereas m3-12-3, m3-12-1, and m3-12 exhibit comparable internalization rate relative to the control compound.

5. Plasma stability

The stability of compounds PSMA-617, m3-12, m3-19, m3-12-1, m3-12-3, and m3-12a was investigated in mouse and human plasma. The results are shown in Tables 15-18.

5.1 Experimental reagents:

Plasma information:

| Matrix type | Anticoagulant | Source |
|---|---|---|
| Mouse | Heparin sodium | Hunan Everpro Medical Co., Ltd., China |
| Human | Heparin sodium | Zibo Municipal Hospital |

| Species | Cat. No. | Lot. No. | Description | Supplier |
|---|---|---|---|---|
| Human | CMS-S50 | F03010 | Pooled | SHQY |
| Species | Cat. No. | Lot. No. | Description | Supplier |
| Mouse | 0191E1.11 | 23B007 | Pooled | IPHASE |

5.2 Experimental method:

An appropriate amount of blank plasma was added to an EP tube. A working solution of the test substance (final concentration: 0.5 μM) was then added thereto and incubated, followed by vortex mixing. The samples were incubated in a water bath at 37°C with two paralleling groups, with incubation time points of 0, 2, 4, and 24 h. After incubation, sample preprocessing was performed: 20 μL of the sample was collected from the incubation system, and 250 μL of an internal standard precipitant was added; a resulting mixture was vortex-mixed, followed by adding 100 μL of ultrapure water. The samples were centrifuged (5500 g) for 10 min, and 250 μL of a resulting supernatant was transferred to a 96-well plate, vortex-mixed, and analyzed by LC-MS/MS.

5.3 Data processing:

$$\text{Remaining original drug\%} = At/A0 \times 100\%$$

At: Amount of the test substance at the incubation time point;
A0: Amount of the test substance in 0 h sample; and

$$T1/2 = 0.693/k.$$

Table 15. Stability results of compounds PSMA-617 and m3-12 in mouse and human plasma

| Compound | Mouse | | Human | |
|---|---|---|---|---|
| | Remaining amount (%) | t1/2 (min) | Remaining amount (%) | t1/2 (min) |
| Propantheline | 0.175 | 40.5 | 0.0440 | 31.5 |
| PSMA-617 | 105 | > 48* | 100 | > 48* |
| m3-12 | 112 | > 48* | 96.8 | > 48* |
| *After 24 h of incubation in the matrix, not less than 75% of the compound is remained, which by default indicates $t_{1/2}$ > 48 h. This means that at 24 h, 75% of the compound is remained, and at 48 hours, 50% of the compound is remained ($t_{1/2}$). | | | | |

Table 16. Stability results of compounds PSMA-617, m3-12a and m3-12-3 in human plasma

| Compound | Species | Test concentration (μM) | Remaining amount (%)§ | | | | | | T1/2 (min) |
|---|---|---|---|---|---|---|---|---|---|
| | | | 0 min | 15 min | 30 min | 45 min | 60 min | 120 min | |
| Propantheline | Human | 5 | 100. 00 | 77.8 5 | 61.72 | 47.97 | 37.82 | 11.24 | 38.08 |
| PSMA-617 | Human | 5 | 100. 00 | 94.4 1 | 90.70 | 100.49 | 85.38 | 82.48 | > 372.68* |
| m3-12a | Human | 5 | 100. 00 | 96.2 2 | 91.87 | 103.85 | 97.82 | 95.35 | > 372.68* |
| m3-12-3 | Human | 5 | 100. 00 | 101. 07 | 100.79 | 95.67 | 100.1 5 | 103.53 | > 372.68* |
| Symbol § represents that: remaining amounts (%) of < 1% were not used for t1/2 calculation. Symbol * represents that: t1/2 is reported as > 372.68 when the remaining amount (%) of the compound at 120 min is > 80%. | | | | | | | | | |

Table 17. Stability results of compounds PSMA-617, m3-12a, and m3-12-3 in mouse plasma

| Compound | Species | Test concentration (μM) | Remaining amount (%)§ | | | | | | T1/2 (min) |
|---|---|---|---|---|---|---|---|---|---|
| | | | 0 min | 15 min | 30 min | 45 min | 60 min | 120 min | |
| Propantheline | Mouse | 5 | 100. 00 | 75.0 7 | 59.46 | 49.00 | 34.90 | 12.77 | 40.57 |
| PSMA-617 | Mouse | 5 | 100. 00 | 98.1 4 | 97.45 | 95.34 | 94.63 | 85.51 | > 372.68* |
| m3-12a | Mouse | 5 | 100. 00 | 103. 42 | 103.0 0 | 98.53 | 92.85 | 101.63 | > 372.68* |
| m3-12-3 | Mouse | 5 | 100. 00 | 104. 61 | 102.1 2 | 105.85 | 101.06 | 95.24 | > 372.68* |
| Symbol § represents that: remaining amounts (%) of < 1% were not used for t1/2 calculation. Symbol * represents that: t1/2 is reported as > 372.68 when the remaining amount (%) of the compound at 120 min is > 80%. | | | | | | | | | |

Table 18. Stability results of compounds PSMA-617, m3-19 and m3-12-1 in human plasma

| Compound | Mouse | | Human | |
|---|---|---|---|---|
| | Remaining amount (%) | t1/2 (h) | Remaining amount (%) | t1/2 (h) |
| Propantheline | 0.0405 | 0.361 | 0.0319 | 0.339 |
| PSMA-617 | 95.2 | > 48* | 85.7 | > 48* |
| m3-19 | 90.9 | > 48* | 88.3 | > 48* |
| m3-12-1 | 103 | > 48* | 96.2 | > 48* |
| * After 24 h of incubation in the matrix, not less than 75% of the compound is remained, which by default indicates $t_{1/2}$ > 48 h. This means that at 24 h, 75% of the compound is remained, and at 48 hours, 50% of the compound is remained ($t_{1/2}$). | | | | |

The results of the protein stability tests in plasma are as follows:

PSMA-617 > 48 h (mice) or > 48 h (human) vs m3-12 > 48 h (mouse) or > 48 h (human);
PSMA-617 > 372.68 min (mice) or > 372.68 min (human) vs m3-12a > 372.68 min (mice) or > 372.68 min (human), m3-12-3 > 372.68 min (mice) or > 372.68 min (human);
PSMA-617 > 48 h (mice) or > 48 h (human) vs m3-19 > 48 h (mice) or > 48h (human), m3-12-1 > 48 h (mice) or > 48 h (human).

Based on the plasma protein stability experiment results presented above, the compounds of the present disclosure exhibit stability in plasma comparable to the control compound. The plasma protein binding rate of the compounds of the present disclosure was further evaluated.

6. Plasma protein binding rate

The binding rates of compounds PSMA-617, m3-12, m3-19, m3-12-1, m3-12a and m3-12-3 to mouse and human plasma proteins were studied. Results are shown in Tables 19-22. In the experiment:

$$f_u = \frac{C_R}{C_D} \times 100\%, \quad f_b = (1 - f_u) \times 100\%, \quad Recovery\ rate = \frac{C_R + C_D}{C_I} \times 100\%.$$

6.1 Experimental reagents:

| Matrix type | | | Anticoagulant | |
|---|---|---|---|---|
| Mouse plasma | | | EDTA-2K | |
| Human plasma | | | EDTA-2K | |
| Species | Cat. No. | Lot. No. | Strain and gender | Supplier |
| Mouse | 0184E1.11 | B0124H0009 | Mixed gender | IPHASE |
| Species | Cat. No. | Lot. No. | Strain and gender | Supplier |
| Human | CMS-S50 | F03010 | Pooled | SHQY |

6.2 Experimental method:

The test compound was added to plasma pre-warmed to 37°C to achieve a final concentration of 0.5 μM. Pre-treated dialysis membranes were assembled into a dialysis plate. 120 μL of the test compound prepared in plasma was added to the donor side of the dialysis membrane in each dialysis well, and 120 μL of 0.002% Tween-PB was added to the receptor side. A 20 μL sample of the test compound at the final concentration was taken and added to a 96-well sample plate to obtain the T0 sample, and the T0 sample was stored in a refrigerator at -20°C. The remaining sample was incubated with shaking at 37°C for 20 h. After incubation, 20 μL of samples were taken from the post-dialysis receptor side (sample B), post-dialysis donor side (sample A), and stability sample (20 h stability sample), respectively. Corresponding volumes of blank plasma or 0.002% Tween-PB were added to the sample B, sample A, T0 sample, and 20 h stability sample, respectively, to achieve a plasma-to-buffer volume ratio of 1 : 1 in each sample well. To all sample wells of the test substance group, 250 μL of precipitant containing internal standard was added, followed by adding 80 μL of ultrapure water. The mixture was mixed uniformly and then centrifuged, and 250 μL of a resulting supernatant was collected for a second centrifugation prior to injection and analysis.

6.3 Data analysis:

The percentage of unbound compound in plasma and the recovery rate were calculated according to the following equations:

Plasma protein binding rate (%) = 1-Percentage of unbound compound;

$$\text{Percentage of unbound compound (\%)} = CB/CA;$$

$$\text{Recovery rate (\%)} = (CB + CA)/CT0;$$

where CB is the concentration of the compound in the receptor solution after equilibrium dialysis; CA is the concentration of the compound in the donor plasma after equilibrium dialysis; and CT0 is the initial concentration of the compound in plasma.

Table 19. Protein binding rate of compounds PSMA-617 and m3-12 in mouse and human plasma

| Compound | Concentration (μM) | Species | Results | | | |
|---|---|---|---|---|---|---|
| | | | $F_u$ (%) | $F_b$ (%) | Recovery rate (%) | Average stability (%) |
| Warfarin | 2 | Mouse | 7.70 | 92.3 | 99.1 | NA |
| PSMA-617 | 0.5 | Mouse | 78.7 | 21.3 | 89.6 | 91.7 |
| m3-12 | 0.5 | Mouse | 90.5 | 9.46 | 99.1 | 90.9 |
| Warfarin | 2 | Human | 0.609 | 99.4 | 94.8 | NA |
| PSMA-617 | 0.5 | Human | 14.0 | 86.0 | 91.7 | 105 |
| m3-12 | 0.5 | Human | 19.9 | 80.1 | 91.4 | 101 |

Table 20. Human plasma protein binding rate of compounds PSMA-617, m3-12a, and m3-12-3 determined by equilibrium dialysis

| Compound | Species | Test concentration (μM) | Fu% | Binding% | Remaining amount% | Recovery% |
|---|---|---|---|---|---|---|
| Warfarin | Human | 1 | 0.56 | 99.44 | 100.59 | 95.73 |
| PSMA-617 | Human | 1 | 30.61 | 69.39 | 93.26 | 74.05 |
| m3-12a | Human | 1 | 18.33 | 81.67 | 98.50 | 83.83 |
| m3-12-3 | Human | 1 | 33.72 | 66.28 | 101.71 | 89.85 |

Table 21. Mouse plasma protein binding rate of compounds PSMA-617, m3-12a and m3-12-3 determined by equilibrium dialysis

| Compound | Species | Test concentration (μM) | Fu% | Binding% | Remaining amount% | Recovery% |
|---|---|---|---|---|---|---|
| Warfarin | Mouse | 1 | 8.16 | 91.84 | 101.58 | 87.44 |
| PSMA-617 | Mouse | 1 | 74.44 | 25.56 | 94.50 | 85.88 |
| m3-12a | Mouse | 1 | 52.06 | 47.94 | 83.33 | 83.63 |
| m3-12-3 | Mouse | 1 | 61.13 | 38.87 | 91.65 | 87.07 |

Table 22. Protein binding rate of compounds PSMA-617, m3-19 and m3-12-1 in mouse and human plasma

| Compound | Concentration (μM) | Species | Results | | | |
|---|---|---|---|---|---|---|
| | | | $F_u$ (%) | $F_b$ (%) | Recovery rate (%) | Average stability (%) |
| Warfarin | 2 | Mouse | 16.5 | 83.5 | 89.5 | NA |
| PSMA-617 | 0.5 | Mouse | 82.1 | 17.9 | 87.0 | 86.2 |
| m3-19 | 0.5 | Mouse | 42.5 | 57.5 | 90.7 | 93.5 |
| m3-12-1 | 0.5 | Mouse | 71.1 | 28.9 | 97.7 | 92.2 |
| Warfarin | 2 | Human | 0.558 | 99.4 | 90.0 | NA |
| PSMA-617 | 0.5 | Human | 14.8 | 85.2 | 90.4 | 97.5 |
| m3-19 | 0.5 | Human | 9.95 | 90.1 | 93.0 | 81.9 |

(continued)

| Compound | Concentration (μM) | Species | Results | | | |
|---|---|---|---|---|---|---|
| | | | $F_u$ (%) | $F_b$ (%) | Recovery rate (%) | Average stability (%) |
| m3-12-1 | 0.5 | Human | 5.03 | 95.0 | 95.5 | 87.5 |

The plasma protein binding rates serve as reference for subsequent *in vivo* and *in vitro* metabolism experiments, with the human plasma protein binding rate being the primary reference standard. The experimental results are as follows:

PSMA-617 (86.0%) vs m3-12 (80.1%);
PSMA-617 (69.39%) vs m3-12a (81.67%), m3-12-3 (66.28%);
PSMA-617 (85.2%) vs m3-19 (90.1%), m3-12-1 (95.0%).

The results show that the compounds of the present disclosure exhibited plasma protein binding rates comparable to the control compound, and all fall within the acceptable range of 60% to 90%. The next step was to evaluate whether the compounds demonstrate superior performance in liver microsomal stability assays.

7. Liver microsomal stability

The stability of compounds PSMA-617, m3-12, m3-19, m3-12-1, m3-12-3, and m3-12a was investigated in mouse, rat, and human liver microsomes. The results are shown in Tables 23-24. Among the parameters, CLint (mic) represents intrinsic clearance in microsomes, R2 represents linear correlation coefficient, and T1/2 represents half-life.

7.1 Experimental reagents:

Liver microsome information

| Species | Cat. No. | Lot. No. | Strain and gender | Supplier |
|---|---|---|---|---|
| Human | 452117 | 38300 | Male and female | Gentest |
| Rat | 452501 | 1260001 | Male Sprague Dawley | Gentest |
| Mouse | LMCD-100P | LMCD-2311v211 | Male CD-1 | Milestone |

7.2 Experimental method:

Liver microsome (LM) solution (0.5587 mg/mL) was added to a 96-well plate (culture plate), followed by adding NADPH (final concentration: 1 mM) or PBS (negative control). The plate was mixed at 800 rpm for 10 s and then pre-warmed at 37°C for 10 min. The reaction was then initiated by adding the test compound (final concentration: 2 μM) to the culture plate. At incubation time points (0.5, 15, 30, 45, and 60 min), 50 μL of the solution was transferred from the incubation plate to a sample plate filled with 200 μL of pre-chilled methanol containing internal standard to terminate the reaction. The sample plate was centrifuged at 3220 g for 40 min. 100 μL of the supernatant was transferred to an analysis plate containing an appropriate volume of $H_2O$ for LC-MS/MS analysis.

7.3 Data analysis:

(1) The slope k of the straight line (ln(remaining compound percentage) vs culture time) was calculated using Microsoft Excel;
(2) The *in vitro* $t_{1/2}$ and *in vitro* Clint were calculated using the following equations: T1/2 (min) = 0.693/k;

Clint (μL/min/mg protein) = (Incubation volume in μL/Protein content in mg in the system) × (0.693/$t_{1/2}$).

Table 23. Stability results of compounds PSMA-617 and m3-12 in mouse, rat, and human liver microsomes

| Species \ Compound | | Midazolam | PSMA-617 | m3-12 |
|---|---|---|---|---|
| Mouse | Remaining amount% (T = 60 min) | 3.23 | 101 | 88.2 |
| | R2 | 0.9999 | 0.0451 | 0.7514 |
| | T1/2 (min) | 3.03 | > 120 | > 120 |
| | *In vitro* CLint (mic) | 1.14 | 0.000800 | 0.00520 |
| Rat | Remaining amount% (T = 60 min) | 4.24 | 90.6 | 100 |
| | R2 | 0.9999 | 0.1219 | 0.0206 |
| | T1/2 (min) | 3.30 | > 120 | > 120 |
| | *In vitro* CLint (mic) | 1.05 | 0.00200 | 0.000200 |
| Human | Remaining amount% (T = 60 min) | 11.8 | 59.8 | 51.3 |
| | R2 | 0.9930 | 0.9986 | 0.9966 |
| | T1/2 (min) | 4.94 | 80.6 | 60.8 |
| | *In vitro* CLint (mic) | 0.701 | 0.0172 | 0.0228 |

Table 24. Metabolic stability results of compounds PSMA-617 and m3-12 conjugated with nuclide $^{175}$Lu in mouse, rat, and human liver microsomes

| Compound | Species | Test concentration (μM) | +/cofactor | Remaining amount (%)§ | | | | | t1/2 (min) | CLint (μL/min/m g protein) |
|---|---|---|---|---|---|---|---|---|---|---|
| | | | | 0.5 min | 15 min | 30 min | 45 min | 60 min | | |
| Verapamil | Human | 2 | + | 100. 00 | 17.5 3 | 7.41 | 4.1 3 | 2.76 | 11.99 | 115.57 |
| | | | - | 100. 00 | 101. 17 | 97.4 3 | 97. 63 | 101. 74 | | |
| Verapamil | Rat | 2 | + | 100. 00 | 6.43 | 1.33 | 0.4 9 | 0.22 | 4.74 | 292.39 |
| | | | - | 100. 00 | 99.0 9 | 91.2 3 | 93. 69 | 101. 07 | | |
| Verapamil | Mouse | 2 | + | 100. 00 | 7.82 | 2.07 | 0.7 2 | 0.32 | 5.28 | 262.37 |
| | | | - | 100. 00 | 92.4 7 | 93.0 5 | 94. 33 | 89.2 6 | | |
| $^{175}$Lu-PSMA -617 | Human | 2 | + | 100. 00 | 94.3 1 | 95.8 7 | 94. 40 | 106. 52 | > 184.7 8 | < 7.50* |
| | | | - | 100. 00 | 99.4 1 | 116. 80 | 106 .74 | 110. 50 | | |
| $^{175}$Lu-PSMA -617 | Rat | 2 | + | 100. 00 | 104. 37 | 98.2 4 | 98. 99 | 94.0 0 | > 184.7 8 | < 7.50* |
| | | | - | 100. 00 | 102. 80 | 107. 48 | 105 .79 | 92.5 0 | | |
| $^{175}$Lu-PSMA -617 | Mouse | 2 | + | 100. 00 | 95.9 4 | 97.5 9 | 89. 75 | 102. 13 | > 184.7 8 | < 7.50* |
| | | | - | 100. 00 | 87.3 7 | 90.9 2 | 98. 57 | 97.3 1 | | |
| $^{175}$Lu-m3-12 | Human | 2 | + | 100. 00 | 105. 03 | 107. 04 | 106 .31 | 98.8 9 | > 184.7 | < 7.50* |
| | | | - | 100. 00 | 105. 00 | 103. 28 | 103 .23 | 97.4 0 | 8 | |
| $^{175}$Lu-m3-12 | Rat | 2 | + | 100. 00 | 94.4 3 | 93.7 3 | 97. 50 | 86.9 5 | > 184.7 8 | < 7.50* |
| | | | - | 100. 00 | 94.5 7 | 97.2 4 | 89. 99 | 85.6 4 | | |
| $^{175}$Lu-m3-12 | Mouse | 2 | + | 100. 00 | 89.8 2 | 100. 91 | 90. 40 | 83.0 3 | > 184.7 8 | < 7.50* |
| | | | - | 100. 00 | 91.7 1 | 94.3 3 | 86. 71 | 93.2 1 | | |

§ Remaining amounts (%) of < 1% were not used for t1/2 and CLint calculation. * t1/2 is reported as > 184.78 min and CLint as < 7.50 when the remaining amount (%) of the compound at 60 minutes is > 80%.

The results of liver microsomal stability assay are as follows:

PSMA-617 > (mouse) 120 min, 0.000800 mic or > (rat) 120 min, 0.00200 mic or (human) 80.6 min, 0.0172 mic;
m3-12 > (mouse) 120 min, 0.00520 mic or > (rat) > 120 min, 0.000200 mic or > (human) 60.8 min, 0.0228 mic.

By comparing the microsomal stability results of the control compound PSMA-617 and m3-12 as empty vehicles in the mouse and rat microsomal stability tests, both PSMA-617 and m3-12 showed low clearance rates (t1/2 > 120 h, CLint (μL/min/g) < 10). In the human liver microsomal stability tests, the clearance rates of both PSMA-617 and m3-12 increased slightly, but remained at the same level. The results are as follows:

PSMA-617: (human) 80.6 min, 0.0172 μL/min/g;
m3-12: 60.8 min, 0.0228 μL/min/g.

The control compound PSMA-617 and the compounds of the present disclosure as empty vehicles exhibited comparable clearance rates in mouse, rat, and human liver microsomal stability assays. Further verification was required to determine if the clearance of the cold compounds chelated with the heavy metal $^{175}$Lu changes, in order to further confirm whether to proceed to *in vivo* pharmacokinetic (PK) experiments. The results are shown as follows:

Both $^{175}$Lu-PSMA-617 and $^{175}$Lu-m3-12 exhibited low clearance rates in mouse, rat, and human liver microsomal stability tests, specifically: t1/2 > 184.78 min, CLint< 7.5 μL/min/mg. These compounds were subjected to subsequent *in vivo* experiments to evaluate their respective plasma drug concentration.

*8. In vivo* pharmacokinetics (PK)

The pharmacokinetics of $^{175}$Lu-PSMA-617 and $^{175}$Lu-m3-12 in rats were studied, and the results are shown in Tables 25-26.

8.1 Experimental materials:

Animal strain

| Species | Strain, gender | Grade | Age of animals | Weight |
|---|---|---|---|---|
| Rat | SD rat, male | SPF | 6-8 weeks | 180-220 g |

Reagent information

| Reagent name | CAS. No. | Cat. No. | Source |
|---|---|---|---|
| DMSO | 67-68-5 | A00878 | Sigma-Aldrich |
| Solutol HS | 70142-34-6 | P916874 | Macklin |
| Saline | 7647-14-5 | 2203091905 | Shimen |

8.2 Experimental method:

| Compound name | Route of administration | Dose of administration | Solvent | Number of animals | Fasting or not |
|---|---|---|---|---|---|
| $^{175}$Lu-PSMA-617 | IV | 2 mpk | 5% DMSO+10% Solutol+85% Saline | N = 3 | NO |

(continued)

| Compound name | Route of administration | Dose of administration | Solvent | Number of animals | Fasting or not |
|---|---|---|---|---|---|
| $^{175}$Lu-m3-12 | IV | 2 mpk | 5% DMSO+10% Solutol+85% Saline | N = 3 | NO |

Blood samples were collected from the jugular vein at 5 min, 15 min, 30 min, 1 h, 2 h, 4 h, 7 h, and 24 h after IV (intravenous) administration. The blood volume collected each time was 0.2 mL. The collected blood samples were transferred to microcentrifuge tubes containing EDTA-K2 anticoagulant, centrifuged at 4°C and 4000 g for 5 min, and the supernatant was collected and stored in a refrigerator at -75°C ± 15°C. Protein precipitation was adopted for sample pretreatment, and LC-MS/MS was adopted to measure and analyze the concentrations of the plasma samples. The obtained data was used to calculate the pharmacokinetic parameters using WinNonlin software. The pharmacokinetic parameters regarding IV administration include CL, t1/2, C0, AUC, MRT, Vd, and so on.

8.3 Data analysis:

Table 25. Plasma drug concentration and PK parameters of $^{175}$Lu-PSMA-617 after intravenous injection of 2 mg/kg $^{175}$Lu-PSMA-617 in SD rats

| Dose (mg/kg) | Route of administration | Blood collection point | Concentration (ng/mL) (1) | | | Average (ng/mL) | Standard deviation | Coefficient of variation (%) |
|---|---|---|---|---|---|---|---|---|
| | | | Gender/ M | Gender/ M | Gender/ M | | | |
| | | | 101-M | 102-M | 103-M | | | |
| 2 | IV | 5 min | 5550 | 5260 | 4920 | 5243 | 315 | 6.01 |
| | | 15 min | 3630 | 3550 | 3320 | 3500 | 161 | 4.60 |
| | | 30 min | 2040 | 2830 | 2590 | 2487 | 405 | 16.3 |
| | | 1 h | 1150 | 1430 | 1250 | 1277 | 142 | 11.1 |
| | | 2h | 306 | 264 | 236 | 269 | 35.2 | 13.1 |
| | | 4 h | 17.0 | BQL | BQL | NA | NA | NA |
| | | 8 h | BQL | BQL | BQL | NA | NA | NA |
| | | 24 h | BQL | BQL | BQL | NA | NA | NA |
| PK parameters | WinNonlin Parameter | Unit | 101 | 102 | 103 | Average | Standard deviation | Coefficient of variation (%) |
| tl/2 | HL_Lambda_z | h | 0.491 | 0.434 | 0.431 | 0.452 | 0.0339 | 7.50 |
| Kel | Lambda_z | 1/h | 1.41 | 1.60 | 1.61 | 1.54 | 0.111 | 7.19 |
| Vdss | Vss_obs | L/kg | 0.342 | 0.303 | 0.326 | 0.324 | 0.0195 | 6.01 |
| MRTinf | MRTINF_obs | h | 0.658 | 0.621 | 0.608 | 0.629 | 0.0260 | 4.13 |
| Cl | Cl_obs | mL/min/kg | 8.65 | 8.14 | 8.94 | 8.58 | 0.404 | 4.71 |
| AUC0-t | AUClast | h*ng/mL | 3839 | 3930 | 3583 | 3784 | 180 | 4.75 |
| AUC0-inf | AUCINF_obs | h*ng/mL | 3851 | 4095 | 3730 | 3892 | 186 | 4.78 |
| AUCO-t/AUC0-inf | | / | 0.997 | 0.960 | 0.961 | 0.972 | 0.0212 | 2.18 |
| (1) BQL: Below the lower limit of quantification (SD rat plasma 10.0 ng/mL) | | | | | | | | |

Table 26. Plasma drug concentration and PK parameters of $^{175}$Lu-m3-12 after intravenous injection of 2 mg/kg $^{175}$Lu-m3-12 in SD rats

| Dose (mg/kg) | Route of administration | Blood collection point | Concentration (ng/mL) (1) | | | Average (ng/mL) | Standard deviation | Coefficient of variation (%) |
|---|---|---|---|---|---|---|---|---|
| | | | Gender/ M | Gender/ M | Gender /M | | | |
| | | | 201-M | 202-M | 203-M | | | |
| 2 | IV | 5 min | 10300 | 8900 | 9100 | 9433 | 757 | 8.03 |
| | | 15 min | 6120 | 4580 | 5470 | 5390 | 773 | 14.3 |
| | | 30 min | 4430 | 3130 | 3440 | 3667 | 679 | 18.5 |
| | | 1 h | 2330 | 1790 | 2230 | 2117 | 287 | 13.6 |
| | | 2 h | 658 | 436 | 533 | 542 | 111 | 20.5 |
| | | 4 h | 38.7 | 22.6 | 44.0 | 35.1 | 11.1 | 31.8 |
| | | 8 h | BQL | BQL | BQL | NA | NA | NA |
| | | 24 h | BQL | BQL | BQL | NA | NA | NA |
| PK parameters | WinNonlin Parameter | Unit | 201 | 202 | 203 | Average | Standard deviation | Coefficient of variation (%) |
| t1/2 | HL_Lambda_z | h | 0.514 | 0.475 | 0.542 | 0.510 | 0.0341 | 6.68 |
| Kel | Lambda_z | 1/h | 1.35 | 1.46 | 1.28 | 1.36 | 0.0922 | 6.77 |
| Vdss | Vss_obs | L/kg | 0.183 | 0.222 | 0.212 | 0.206 | 0.0204 | 9.88 |
| MRTinf | MRTINF_obs | h | 0.695 | 0.644 | 0.702 | 0.680 | 0.0314 | 4.62 |
| Cl | Cl_obs | mL/min/kg | 4.40 | 5.76 | 5.05 | 5.07 | 0.680 | 13.4 |
| AUC0-t | AUClast | h*ng/mL | 7554 | 5776 | 6572 | 6634 | 890 | 13.4 |
| AUC0-inf | AUCINF_obs | h*ng/mL | 7582 | 5792 | 6606 | 6660 | 896 | 13.5 |
| AUC0-t/AUC0-inf | | / | 0.996 | 0.997 | 0.995 | 0.996 | 0.00127 | 0.128 |
| (1) BQL: Below the lower limit of quantification (SD rat plasma 10.0 ng/mL) | | | | | | | | |

*In vivo* pharmacokinetic (PK) tests of the cold compounds $^{175}$Lu-PSMA-617 and $^{175}$Lu-m3-12 showed that the $t_{1/2}$ of $^{175}$Lu-PSMA-617 and $^{175}$Lu-m3-12 were 0.452 h and 0.510 h, respectively, and their AUCs were 3784 h*ng/mL and 6634 h*ng/mL, respectively. This suggests that the *in vivo* clearance of compound $^{175}$Lu-m3-12 of the present disclosure is lower than that of the control compound $^{175}$Lu-PSMA-617, and the compound of the present disclosure is expected to have better tissue distribution properties.

The above descriptions are merely several exemplary embodiments of the present disclosure and are not intended to limit the present disclosure in any form. Although the present disclosure has been disclosed as above by way of preferred embodiments, it is not intended to limit the present disclosure. Any equivalent or similar embodiments obtained by those skilled in the art by making some changes or modifications to the above-disclosed technical content without departing from the scope of the technical solutions of the present disclosure are within the scope of the present disclosure.

## Claims

1. A prostate-specific membrane antigen (PSMA)-targeting ligand compound having a structure as shown in formula I, or a stereoisomer, a tautomer, a hydrate, a solvate, a pharmaceutically acceptable salt or ester thereof,

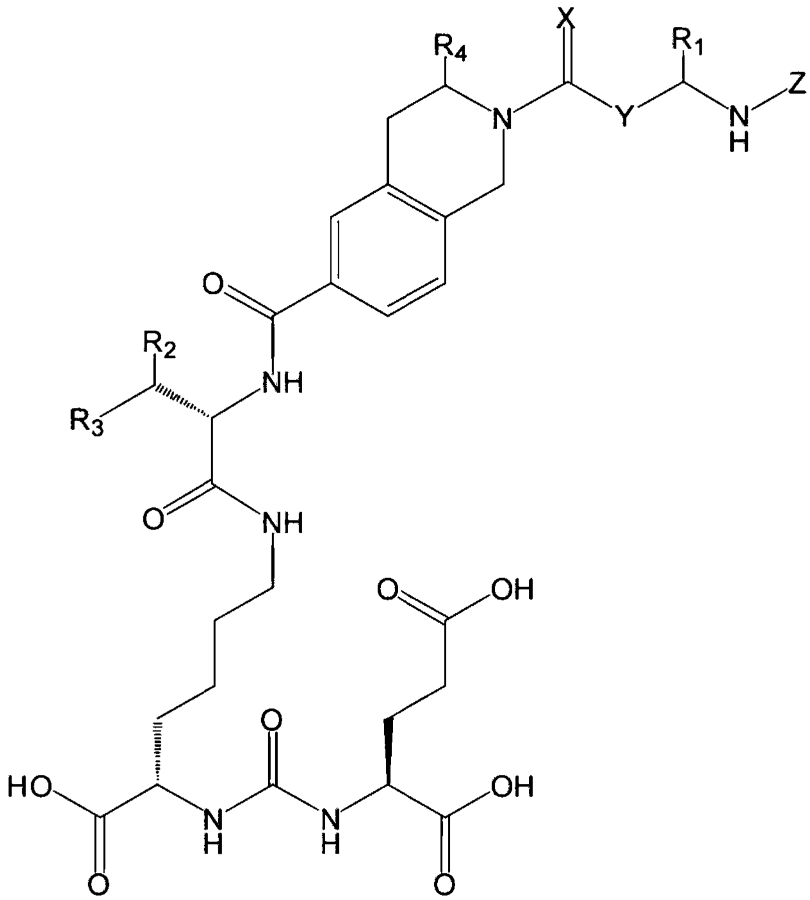

I

wherein

$R_1$, $R_2$ and $R_4$ are each independently selected from the group consisting of H and optionally substituted linear or branched C1-C5 alkyl, wherein a substituent in the optionally substituted linear or branched C1-C5 alkyl is selected from the group consisting of deuterium, carboxyl, linear or branched C1-C5 alkylacyl, linear or branched C1-C5 alkyl ester group, halogen, hydroxyl, linear or branched C1-C5 alkylthio, cyano, linear or branched C1-C5 alkoxy, amino, nitro, phenylsulfonamido, linear or branched C1-C5 alkylamido, 6- to 14-membered aryl, 5- to 14-membered heteroaryl, 6- to 14-membered aryloxy, 5- to 14-membered heteroaryloxy, linear or branched C2-C6 alkenyl, and linear or branched C2-C6 alkynyl;
$R_3$ is selected from the group consisting of optionally substituted 6- to 14-membered aryl and optionally substituted 5- to 14-membered heteroaryl, wherein a substituent in the optionally substituted 6- to 14-membered aryl or the optionally substituted 5- to 14-membered heteroaryl is selected from the group consisting of deuterium, carboxyl, halogen, linear or branched C1-C5 alkyl substituted with halogen, linear or branched C1-C5 alkylacyl, linear or branched C1-C5 alkyl ester group, hydroxyl, linear or branched C1-C5 alkylthio, cyano, linear or branched C1-C5 alkoxy, amino, nitro, phenylsulfonamido, linear or branched C1-C5 alkylamido, linear or branched C2-C6 alkenyl, and linear or branched C2-C6 alkynyl;
X is sulfur atom or oxygen atom;
Y is selected from a chemical bond and $-NH-(CH_2)m-$, wherein m is an integer selected from 0-18; and
Z is a radioactive metal ion chelating group.

2. The PSMA-targeting ligand compound or the stereoisomer, the tautomer, the hydrate, the solvate, the pharmaceutically acceptable salt or ester thereof as claimed in claim 1, wherein $R_1$ is H, carboxyl-substituted methyl, or carboxyl-substituted ethyl;

preferably, $R_2$ is H, methyl, or ethyl;
preferably, $R_3$ is selected from optionally substituted phenyl, and optionally substituted naphthyl, wherein a

substituent in the optionally substituted phenyl or the optionally substituted naphthyl is selected from the group consisting of halogen, such as F-, Cl- or Br-, trifluoromethyl, difluoromethyl, and monofluoromethyl;
preferably, $R_4$ is H, methyl, or ethyl;
preferably, Y is a chemical bond or $-NH-CH_2-$;
preferably, m is 0, 1, or 2;
preferably, Z is the radioactive metal ion chelating group formed from a chelating agent selected from the group consisting of: 1,4,7,10-tetraazacyclododecane-1,4,7,10-tetraacetic acid (DOTA), 1,4,7-triazacyclononane-1,4,7-triacetic acid (NOTA), 1,4,7-triazacyclononane triacetic acid modified with an amino acid (NOTA-AA), diethylenetriamine pentaacetic acid (DTPA), 1,4,7-triazacyclononane-1,4-diacetic acid (NODA), 2-(4,7-bis(carboxymethyl)-1,4,7-triazonon-1-yl)glutaric acid (NODAGA), 1,4,7,10-tetraazacyclododecane-1,4,7,10-tetramethylene phosphonic acid (DOTP), 1,4,8,11-tetraazacyclotetradecane-1,4,8,11-tetraacetic acid (TETA), diacetylbis(N4-methyl-3-thiosemicarbazone) (ATSM), pyruvaldehyde bis(N4,N4-dimethylthiosemicarbazone) (PTSM), ethylenediamine tetraacetic acid (EDTA), trisodium ethylenediamine disuccinate (EC), N,N'-bis(2-hydroxybenzyl)-ethylenediamine-N,N-diacetic acid (HBED), N,N'-bis(2-hydroxybenzyl)ethylenediamine-N,N'-diacetic acid ketone (HBEDCC), monobenzylamino diacetic acid (SBAD), N,N'-bis(3-aminopropyl)ethylenediamine (BAPEN), deferoxamine mesylate (Df), N'-{5-[acetyl(hydroxyl)amino]pentyl}-N-[5-({4-[5-aminopentyl)(hydroxyl)amino]-4-oxobutanoyl}amino)pentyl]-N-hydroxylsuccinamide (DFO), 1,4,7-triazacyclononane (TACN), 1,4,7-triazacyclononane-1,7-diacetic acid/1,4,7-triazacyclononane-1,4,7-triacetic acid maleimide (NO2A/NOTAM), 1,4,7,10-tetraazacyclododecane-1,7-diacetic acid (CB-DO2A), 1,4,7,10-tetraazacyclododecane (Cyclen), sodium 2,2',2"-(1,4,7,10-tetraazacyclododecane-1,4,7-triyl)triacetate (DO3A), 1,4,7,10-tetraazacyclododecane-1,4,7-triacetic acid-10-methylphosphonic acid (DO3AP), 6-hydrazinonicotinoyl succinimide ester hydrochloride (HYNIC), S-acetylmercaptoacetyltriserine (MAS3), mercaptoacetyltriglycine (MAG3), isonitrile, and derivatives thereof.

**3.** The PSMA-targeting ligand compound or the stereoisomer, the tautomer, the hydrate, the solvate, the pharmaceutically acceptable salt or ester thereof as claimed in claim 1 or 2, wherein $R_3$ is selected from the group consisting of the following groups:

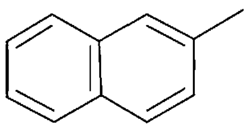 , 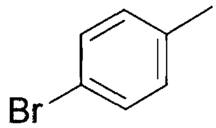 , and 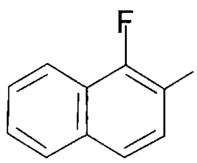 ;

preferably, Z is

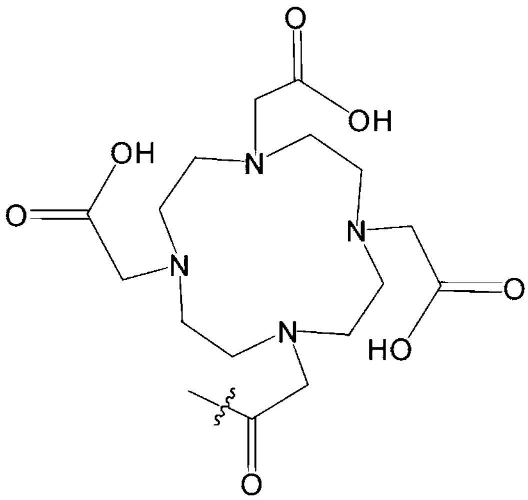 .

**4.** The PSMA-targeting ligand compound or the stereoisomer, the tautomer, the hydrate, the solvate, the pharmaceutically acceptable salt or ester thereof as claimed in any one of claims 1 to 3, wherein the PSMA-targeting ligand compound or the stereoisomer thereof is selected from the group consisting of the following compounds:

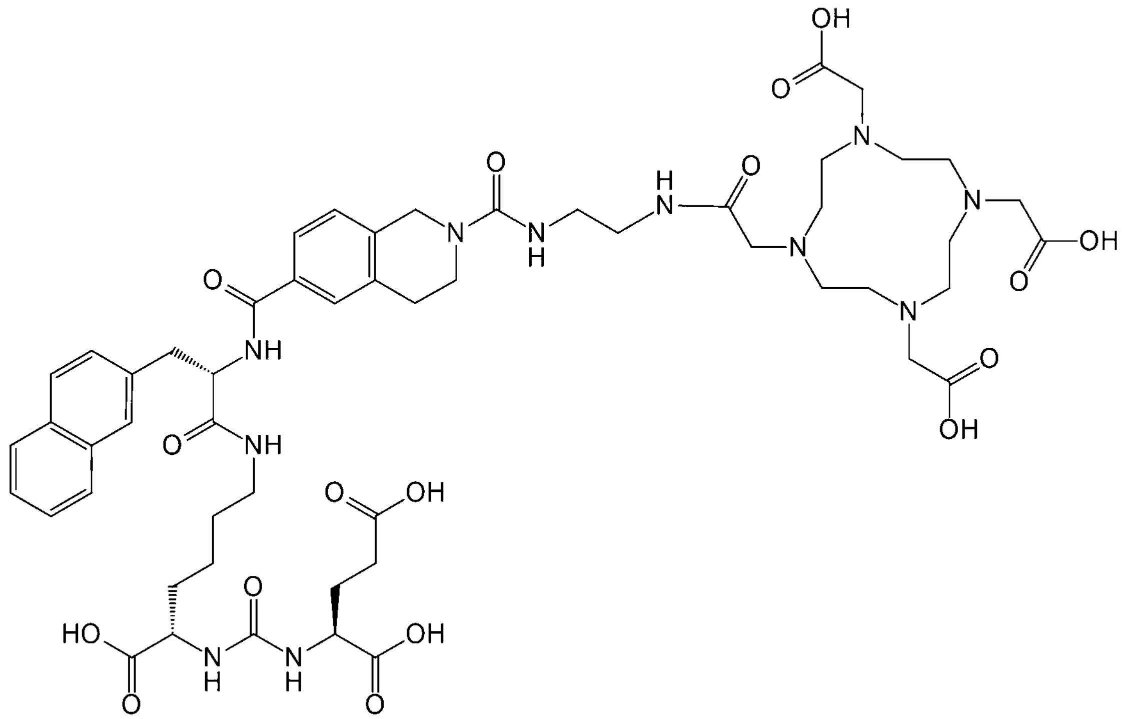

m3-12;

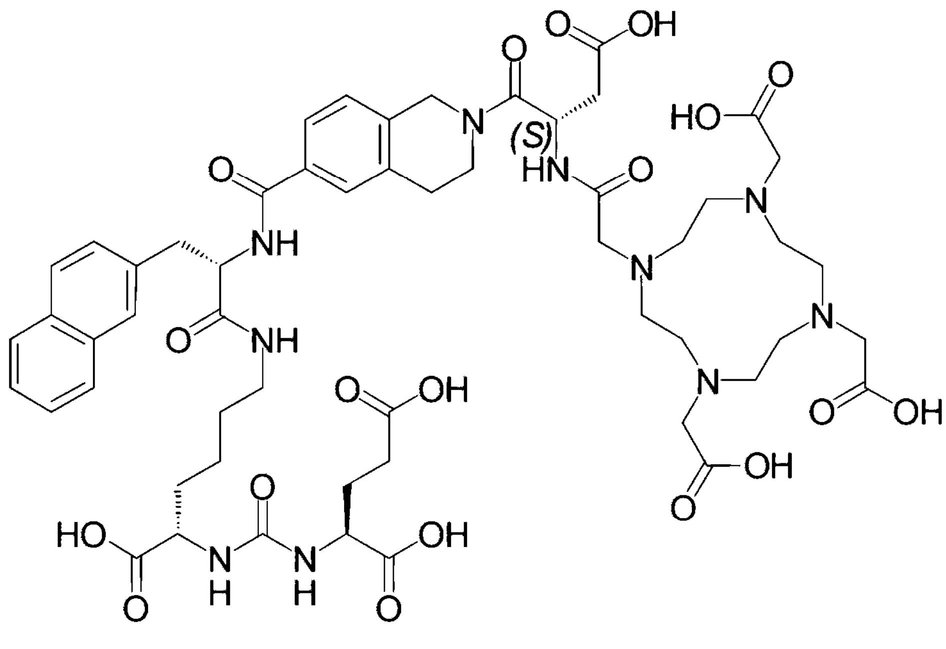

m3-18;

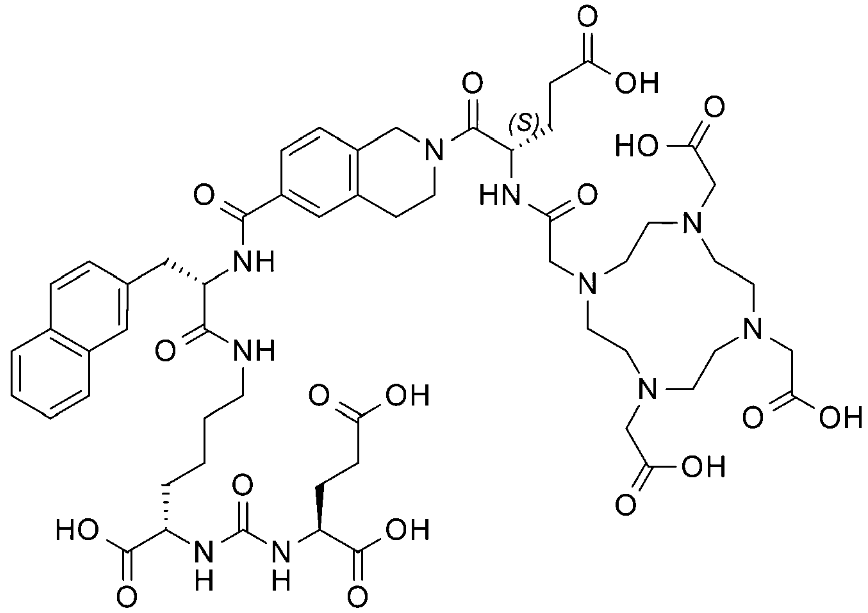

m3-19;

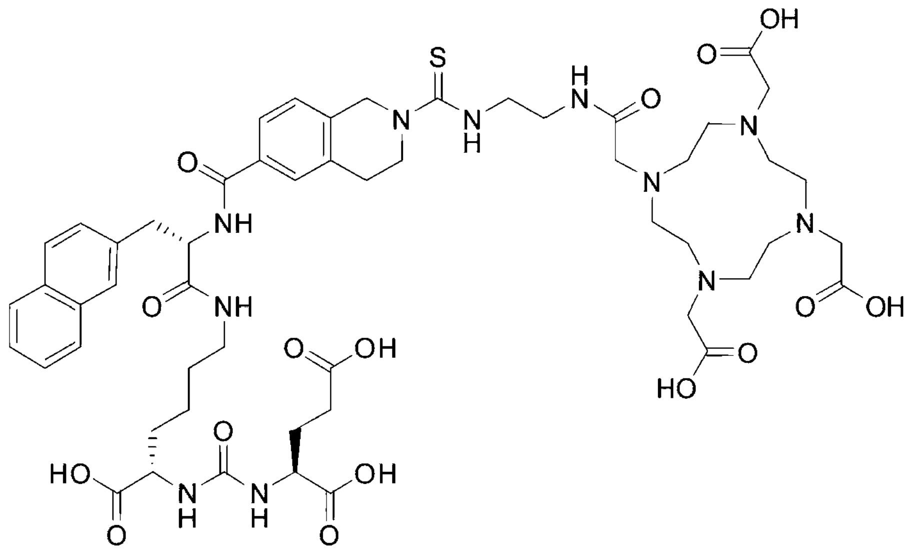

m3-12-1;

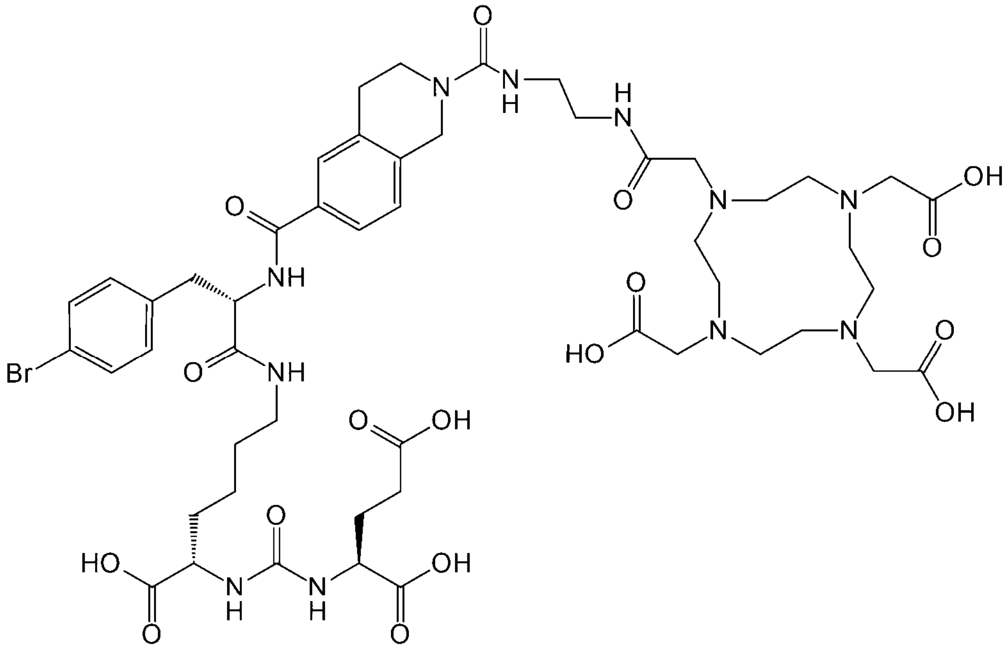

m3-12-3;

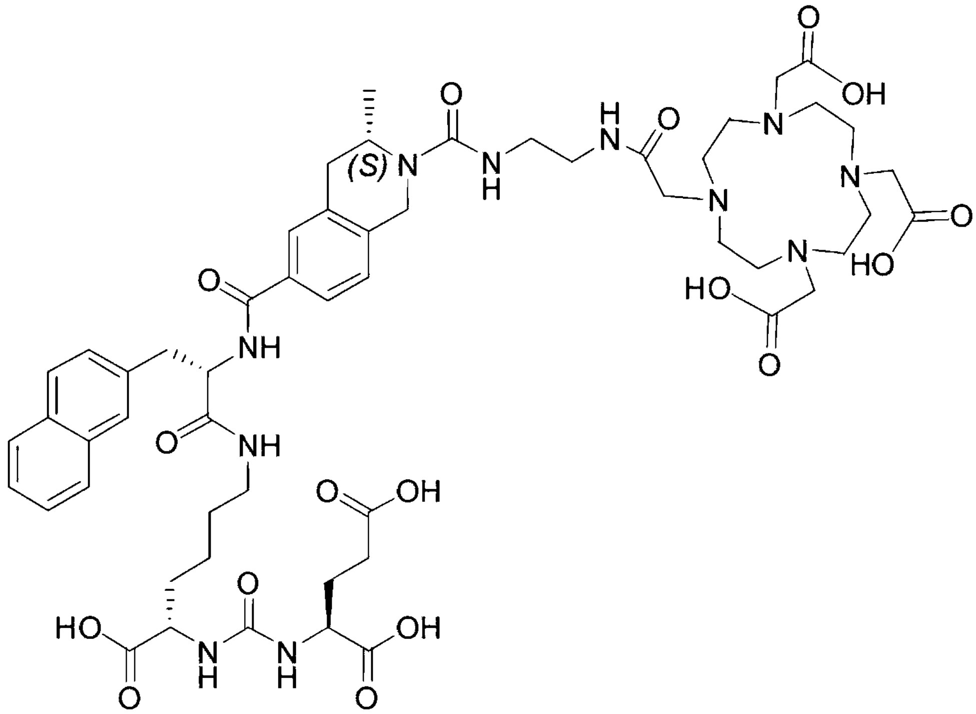

m3-12-a;

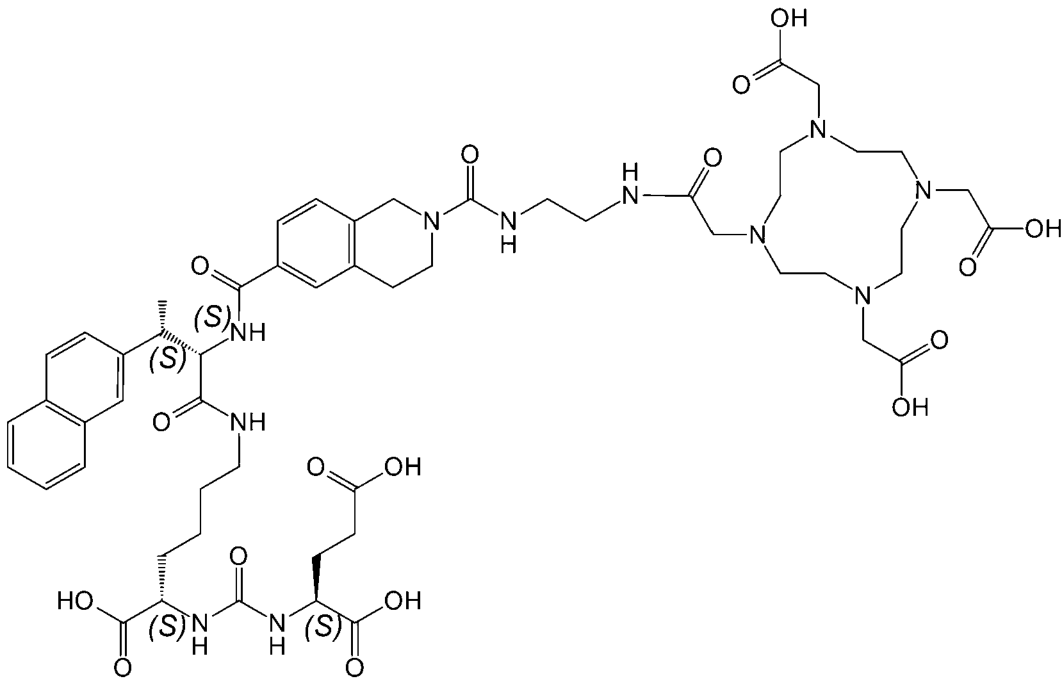

m3-12-2; and

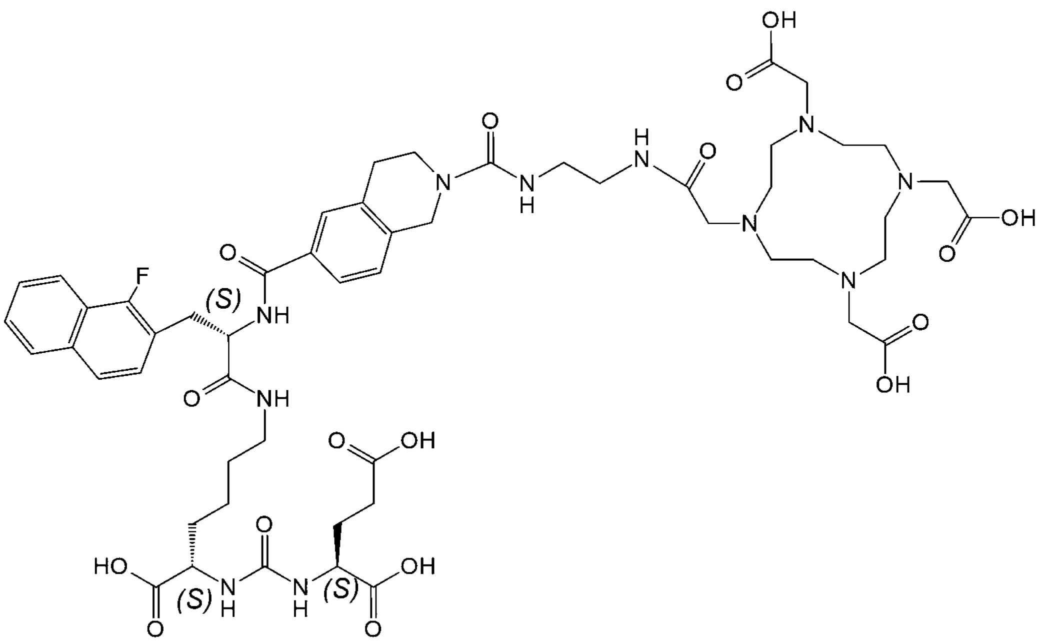

m3-12-5.

**5.** A method for preparing the PSMA-targeting ligand compound or the stereoisomer, the tautomer, the hydrate, the solvate, the pharmaceutically acceptable salt or ester thereof as claimed in any one of claims 1 to 4, the method comprising:

(1) reacting a compound as shown in formula II with a compound as shown in formula III in an organic solvent containing a condensing reagent, and an organic amine to produce a compound as shown in formula IV;

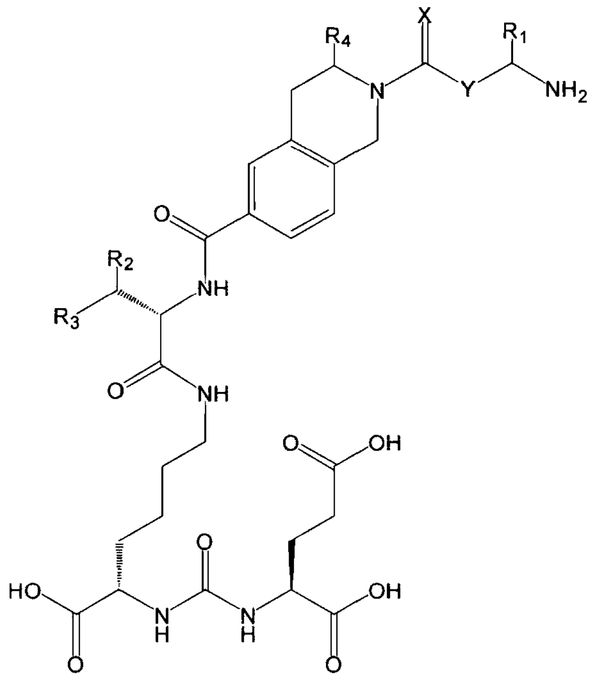

II,

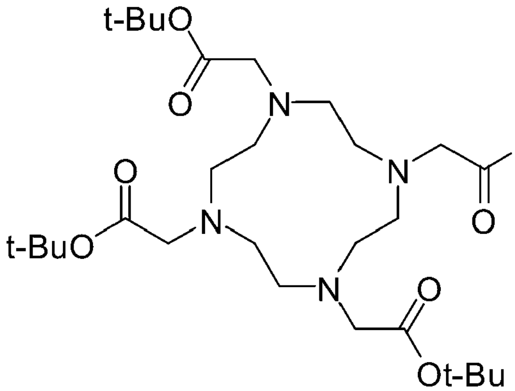

E

III,

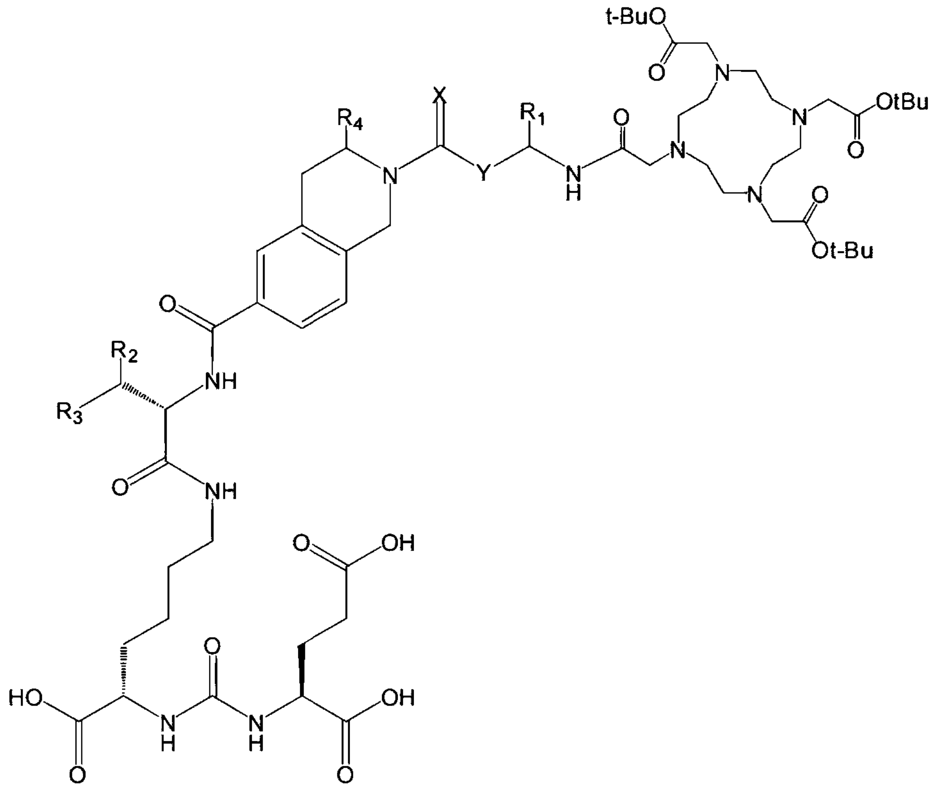

IV;

and

(2) reacting the compound as shown in formula IV in an organic solvent containing a deprotection reagent to produce the PSMA-targeting ligand compound as shown in formula I.

**6.** The method as claimed in claim 5, wherein the condensing reagent is at least one selected from the group consisting of 2-(7-azobenzotriazole)-N,N,N',N'-tetramethyluronium hexafluorophosphate (HATU), N,N'-dicyclohexylcarbodiimide (DCC), 1-(3-dimethylaminopropyl)-3-ethylcarbodiimide (EDCI), (benzotriazol-1-yl-oxy)tripyrrolidnophosphonium hexafluorophosphate (ByBOP), and 1-propylphosphonic acid cyclic anhydride (T3P);

preferably, the organic amine is at least one selected from the group consisting of N,N-diisopropylethylamine (DIEA), and triethylamine ($Et_3N$);
preferably, the deprotection reagent is at least one selected from the group consisting of trifluoroacetic acid (TFA), a solution of hydrogen chloride in dioxane, a solution of hydrogen chloride in methanol, and a solution of hydrogen chloride in ethyl acetate; and
preferably, the organic solvent is at least one selected from the group consisting of N,N-dimethylformamide (DMF), tetrahydrofuran (THF), and dichloromethane (DCM).

**7.** A chelate comprising:

the PSMA-targeting ligand compound or the stereoisomer, the tautomer, the hydrate, the solvate, the pharmaceutically acceptable salt or ester thereof as claimed in any one of claims 1 to 4; and
a radioactive metal ion chelated thereto.

**8.** The chelate as claimed in claim 7, wherein a radioactive metal is one or more selected from the group consisting of $^{177}Lu$, $^{121}I$, $^{131}I$, $^{211}At$, $^{111}In$, $^{153}Sm$, $^{186}Re$, $^{188}Re$, $^{67}Cu$, $^{212}Pb$, $^{225}Ac$, $^{213}Bi$, $^{212}Bi$, $^{212}Pb$, and $^{68}Ga$; preferably, the radioactive metal is $^{177}Lu$.

**9.** A pharmaceutical composition for use in treatment of a tumor, the pharmaceutical composition comprising the chelate as claimed in claim 7 or 8, and optional at least one pharmaceutically acceptable carrier;

preferably, the tumor is at least one selected from the group consisting of prostate cancer, renal carcinoma, and gastric cancer;
preferably, the prostate cancer is at least one selected from the group consisting of castration-resistant prostate cancer, metastatic castration-resistant prostate cancer, and PSMA-positive prostate cancer.

**10.** A reagent composition for use in diagnosis of a tumor, the reagent composition comprising the chelate as claimed in claim 7 or 8, and optional at least one diagnostically acceptable carrier;

preferably, the tumor is at least one selected from the group consisting of prostate cancer, renal carcinoma, and gastric cancer;
preferably, the prostate cancer is at least one selected from the group consisting of castration-resistant prostate cancer, metastatic castration-resistant prostate cancer, and PSMA-positive prostate cancer.

**11.** A method for preparing a PSMA inhibitor, comprising using the PSMA-targeting ligand compound or the stereoisomer, the tautomer, the hydrate, the solvate, the pharmaceutically acceptable salt or ester thereof as claimed in any one of claims 1 to 4.

**12.** A method for preparing a medicament for treatment of a tumor, comprising: using the chelate as claimed in claim 7 or 8;

preferably, the tumor is at least one selected from the group consisting of prostate cancer, renal carcinoma, and gastric cancer;
preferably, the prostate cancer is at least one selected from the group consisting of castration-resistant prostate cancer, metastatic castration-resistant prostate cancer, and PSMA-positive prostate cancer.

**13.** A method for preparing a reagent for diagnosis of a tumor, comprising:

using the chelate as claimed in claim 7 or 8;
preferably, the tumor is at least one selected from the group consisting of prostate cancer, renal carcinoma, and gastric cancer;
preferably, the prostate cancer is at least one selected from the group consisting of castration-resistant prostate cancer, metastatic castration-resistant prostate cancer, and PSMA-positive prostate cancer.

**14.** The method as claimed in claim 13, wherein the reagent for the diagnosis of the tumor is a tumor imaging agent.

**15.** A tumor imaging agent, comprising the chelate as claimed in claim 7 or 8.

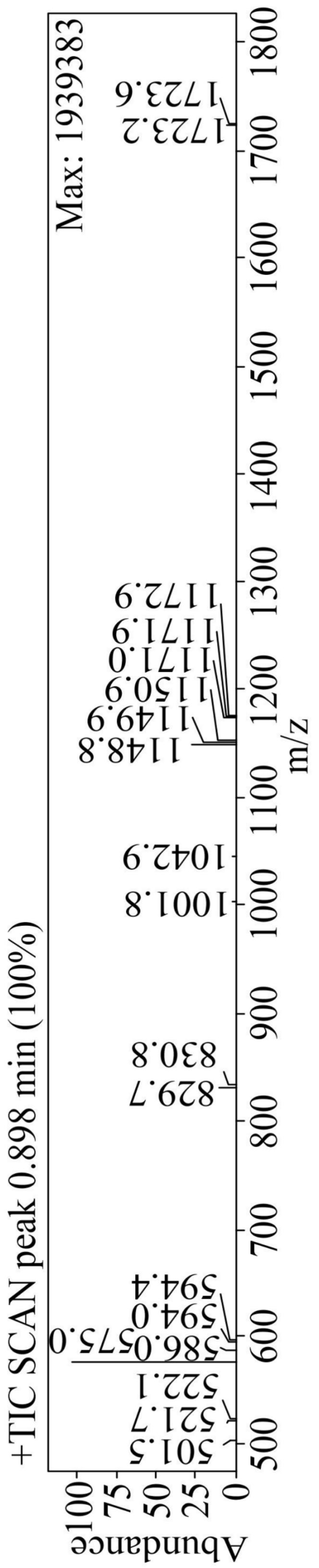
+TIC SCAN peak 0.898 min (100%)
Max: 1939383
Abundance
100
75
50
25
0
501.5
521.7
522.1
575.0
586.0
594.0
594.4
829.7
830.8
1001.8
1042.9
1148.8
1149.9
1150.9
1171.0
1171.9
1172.9
1723.2
1723.6
500
600
700
800
900
1000
1100
1200
1300
1400
1500
1600
1700
1800
m/z

FIG. 1

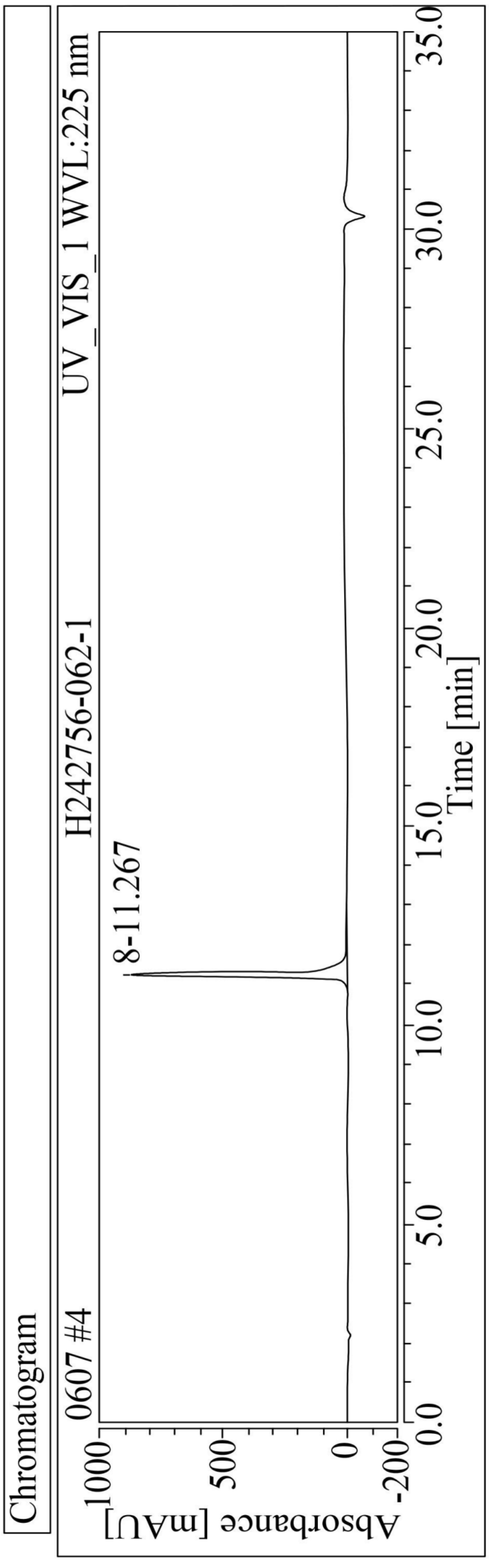
Chromatogram
0607 #4
H242756-062-1
UV_VIS_1 WVL:225 nm
8-11.267
Absorbance [mAU]
1000
500
0
-200
0.0
5.0
10.0
15.0
20.0
25.0
30.0
35.0
Time [min]

FIG. 2A

Integration Results

| NO. | Peak Name | Retention Time min | Area mAU*min | Height mAU | RelativeArea % | Resolution USP | Plates USP | Asymmetry USP |
|---|---|---|---|---|---|---|---|---|
| 1 | | 8.982 | 0.023 | 0.319 | 0.02 | 6.34 | 108003 | 1.11 |
| 2 | | 9.800 | 0.022 | 0.193 | 0.02 | 1.84 | 69379 | n.a. |
| 3 | | 10.083 | 0.096 | 0.887 | 0.09 | 2.44 | 64344 | 1.14 |
| 4 | | 10.255 | 0.006 | 0.081 | 0.01 | n.a. | n.a. | n.a. |
| 5 | | 10.498 | 0.113 | 0.962 | 0.11 | 1.15 | 54065 | n.a. |
| 6 | | 10.715 | 0.064 | 0.569 | 0.06 | 1.05 | 47923 | n.a. |
| 7 | | 10.940 | 0.584 | 4.054 | 0.58 | 1.61 | 35937 | n.a. |
| 8 | | 11.267 | 98.026 | 880.597 | 97.10 | 1.40 | 65763 | 1.05 |
| 9 | | 11.482 | 0.893 | 9.384 | 0.88 | 1.39 | 120865 | n.a. |
| 10 | | 11.750 | 0.821 | 5.103 | 0.81 | 0.20 | 34411 | n.a. |
| 11 | | 11.918 | 0.180 | 1.468 | 0.18 | 0.86 | 1162 | n.a. |
| 12 | | 12.638 | 0.123 | 0.739 | 0.12 | n.a. | 34534 | 0.93 |
| Total: | | | 100.949 | 904.356 | 100.00 | | | |

FIG. 2B

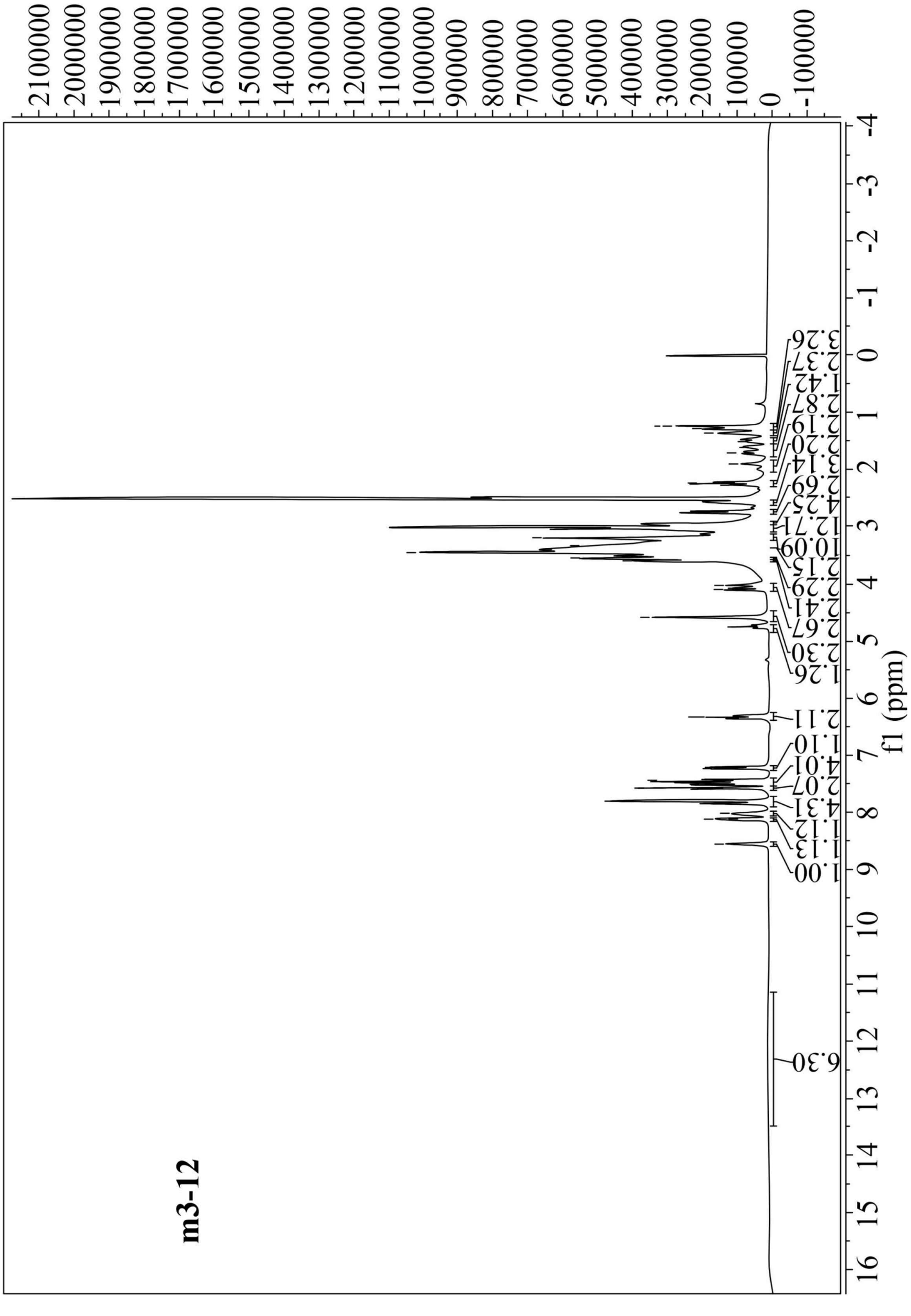


FIG. 3

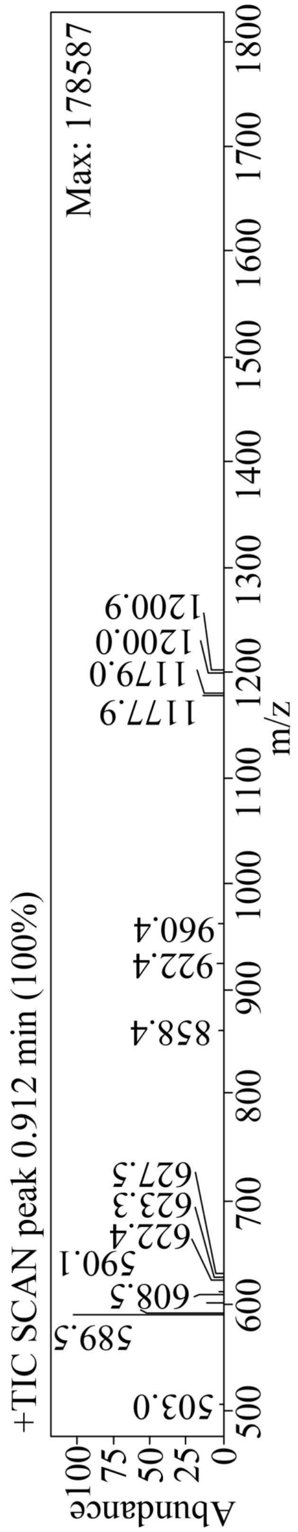
+TIC SCAN peak 0.912 min (100%)
Max: 178587
Abundance
m/z
503.0
589.5
608.5
590.1
622.4
623.3
627.5
858.4
922.4
960.4
1177.9
1179.0
1200.0
1200.9

FIG. 4

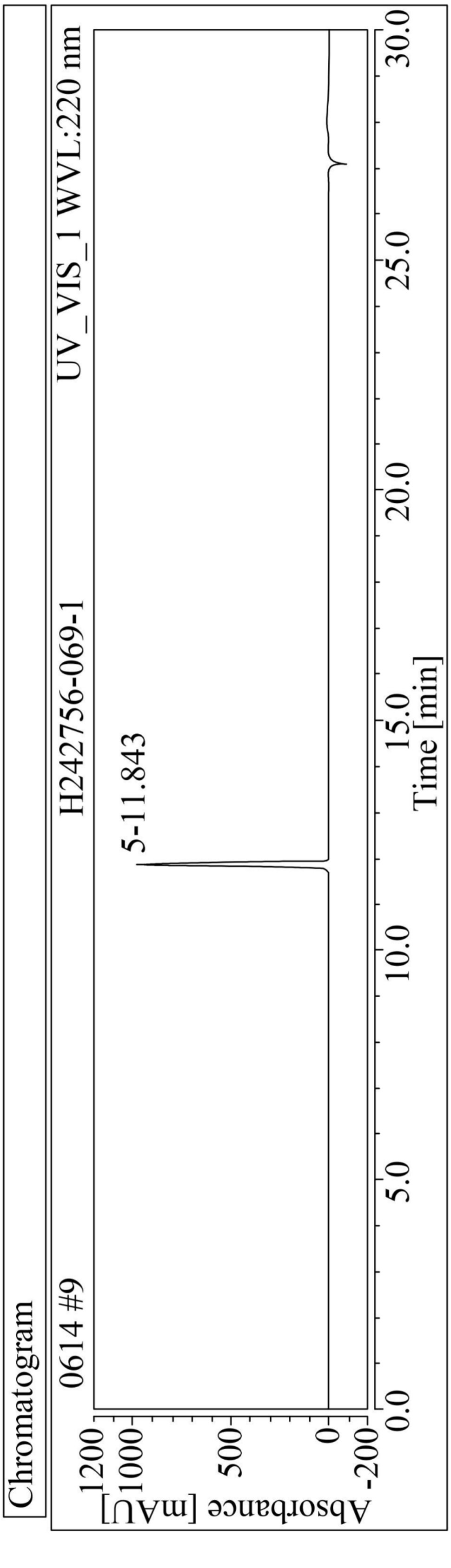
Chromatogram
0614 #9
H242756-069-1
UV_VIS_1 WVL:220 nm
5-11.843
Absorbance [mAU]
1200
1000
500
0
-200
0.0
5.0
10.0
15.0
20.0
25.0
30.0
Time [min]

FIG. 5A

| Integration Results | | | | | | | | |
|---|---|---|---|---|---|---|---|---|
| NO. | Peak Name | Retention Time min | Area mAU*min | Height mAU | RelativeArea % | Resolution USP | Plates USP | Asymmetry USP |
| 1 | | 10.642 | 0.006 | 0.083 | 0.01 | 2.96 | 191325 | 0.93 |
| 2 | | 10.912 | 0.003 | 0.056 | 0.00 | 2.05 | 263385 | 0.91 |
| 3 | | 11.100 | 0.584 | 7.943 | 1.02 | 1.74 | 202702 | n.a. |
| 4 | | 11.267 | 0.033 | 0.492 | 0.06 | 6.25 | 233714 | n.a. |
| 5 | | 11.843 | 56.143 | 953.561 | 98.15 | 2.90 | 269924 | 1.07 |
| 6 | | 12.063 | 0.042 | 0.847 | 0.07 | 0.46 | 639882 | n.a. |
| 7 | | 12.130 | 0.037 | 0.543 | 0.06 | 0.89 | 44221 | n.a. |
| 8 | | 12.292 | 0.017 | 0.292 | 0.03 | 0.64 | 140348 | n.a. |
| 9 | | 12.377 | 0.029 | 0.374 | 0.05 | 1.03 | 139696 | n.a. |
| 10 | | 12.492 | 0.019 | 0.353 | 0.03 | 0.95 | 309030 | n.a. |
| 11 | | 12.575 | 0.019 | 0.355 | 0.03 | 2.79 | 342620 | n.a. |
| 12 | | 12.842 | 0.016 | 0.313 | 0.03 | 0.92 | 237658 | n.a. |
| 13 | | 12.935 | 0.135 | 2.105 | 0.24 | 1.64 | 284919 | n.a. |
| 14 | | 13.123 | 0.033 | 0.422 | 0.06 | 0.71 | 156669 | n.a. |
| 15 | | 13.223 | 0.021 | 0.307 | 0.04 | 0.96 | 122860 | n.a. |
| 16 | | 13.367 | 0.008 | 0.092 | 0.01 | 0.85 | 129259 | n.a. |
| 17 | | 13.498 | 0.014 | 0.136 | 0.02 | 9.13 | 112925 | n.a. |
| 18 | | 14.625 | 0.006 | 0.109 | 0.01 | 12.01 | 461698 | 0.89 |
| 19 | | 15.745 | 0.012 | 0.186 | 0.02 | 1.65 | 393360 | 1.00 |
| 20 | | 15.897 | 0.024 | 0.440 | 0.04 | n.a. | 585714 | 1.16 |
| Total: | | | 57.200 | 969.009 | 100.00 | | | |

**FIG. 5B**

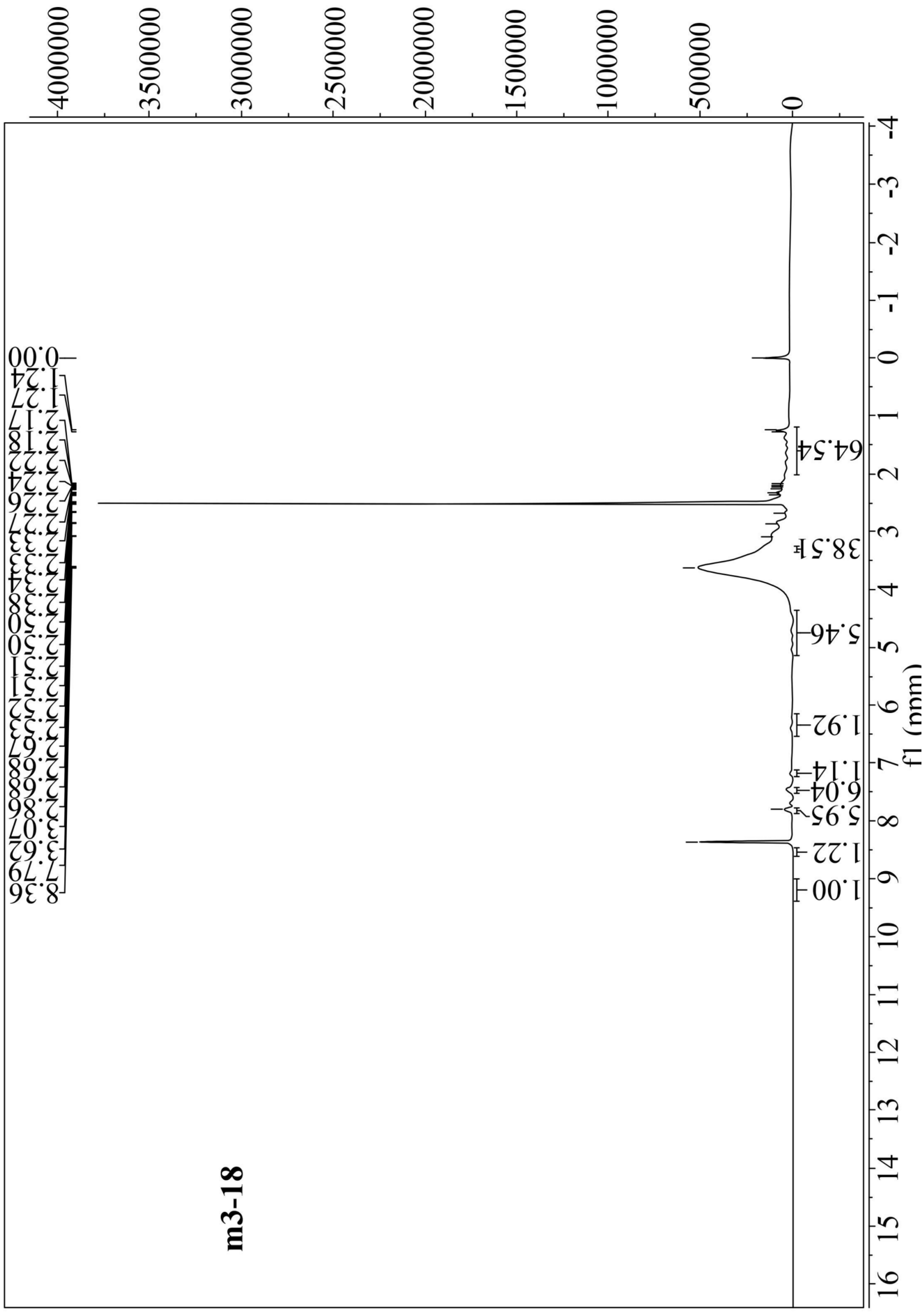
m3-18
f1 (ppm)
0.00
1.24
1.27
2.17
2.18
2.22
2.24
2.26
2.27
2.33
2.33
2.34
2.38
2.50
2.50
2.51
2.51
2.52
2.53
2.67
2.68
2.68
2.86
3.07
3.62
7.79
8.36
64.54
38.51
5.46
1.92
1.14
6.04
5.95
1.22
1.00

FIG. 6

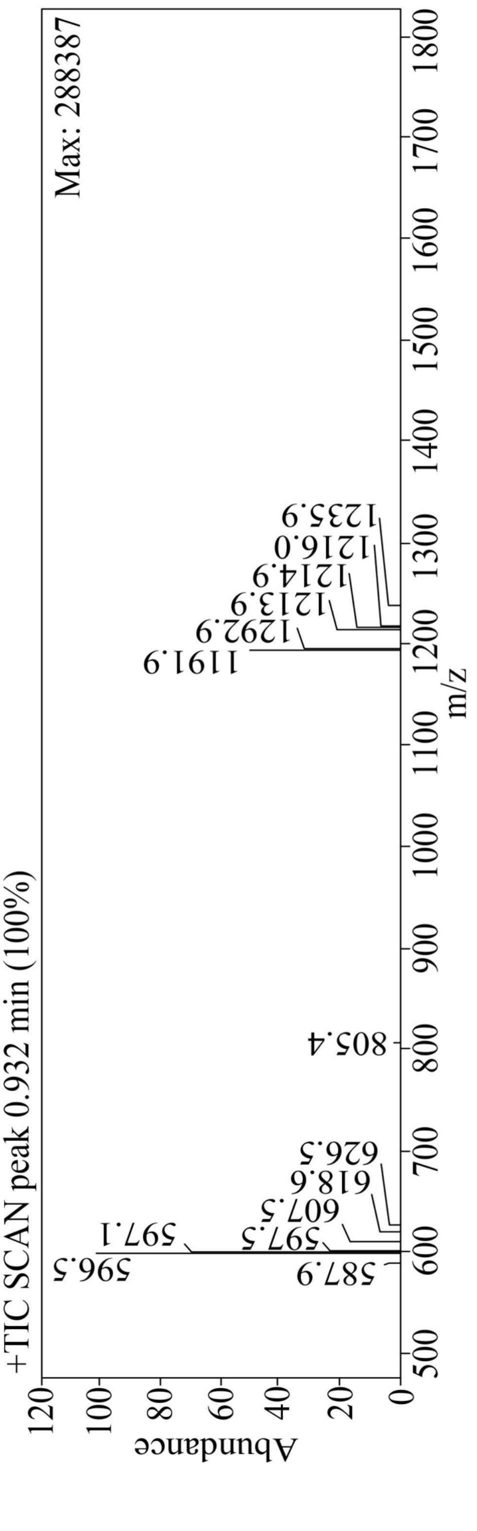
+TIC SCAN peak 0.932 min (100%)
Max: 288387
Abundance
m/z
596.5
597.1
587.9
597.5
607.5
618.6
626.5
805.4
1191.9
1292.9
1213.9
1214.9
1216.0
1235.9

FIG. 7

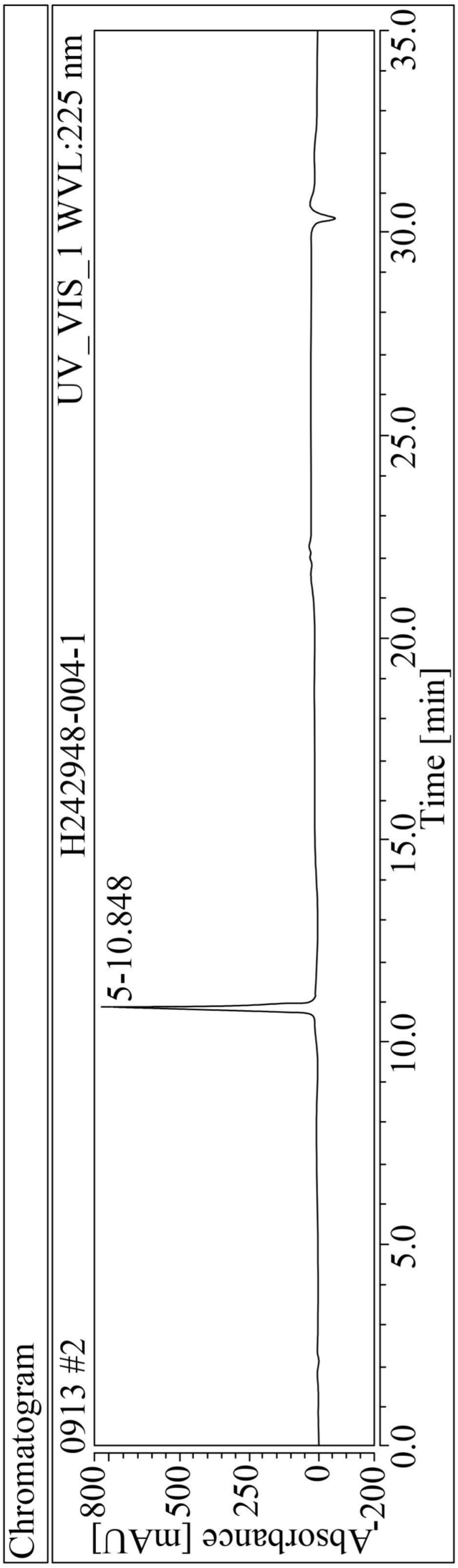
Chromatogram
0913 #2
H242948-004-1
UV_VIS_1 WVL:225 nm
5-10.848
Absorbance [mAU]
800
500
250
0
-200
0.0
5.0
10.0
15.0
20.0
25.0
30.0
35.0
Time [min]

FIG. 8A

| Integration Results | | | | | | | | |
|---|---|---|---|---|---|---|---|---|
| NO. | Peak Name | Retention Time min | Area mAU*min | Height mAU | RelativeArea % | Resolution USP | Plates USP | Asymmetry USP |
| 1 | | 8.922 | 0.398 | 4.070 | 0.47 | 5.48 | 51805 | 1.22 |
| 2 | | 9.750 | 0.375 | 3.924 | 0.44 | 2.70 | 71573 | 1.12 |
| 3 | | 10.280 | 0.352 | 2.355 | 0.42 | 0.94 | 27773 | n.a. |
| 4 | | 10.558 | 0.964 | 4.740 | 1.14 | 1.13 | 15115 | n.a. |
| 5 | | 10.846 | 80.612 | 738.109 | 95.68 | 2.40 | 23962 | 1.06 |
| 6 | | 11.075 | 0.649 | 6.777 | 0.77 | 0.59 | 6826812 | n.a. |
| 7 | | 11.265 | 0.267 | 2.043 | 0.32 | 0.33 | 5180 | n.a. |
| 8 | | 11.430 | 0.166 | 1.557 | 0.20 | 0.44 | 16186 | n.a. |
| 9 | | 11.557 | 0.322 | 2.376 | 0.38 | 4.31 | 44893 | n.a. |
| 10 | | 12.537 | 0.149 | 0.607 | 0.18 | n.a. | 44856 | 1.41 |
| Total: | | | 84.254 | 766.557 | 100.00 | | | |

**FIG. 8B**

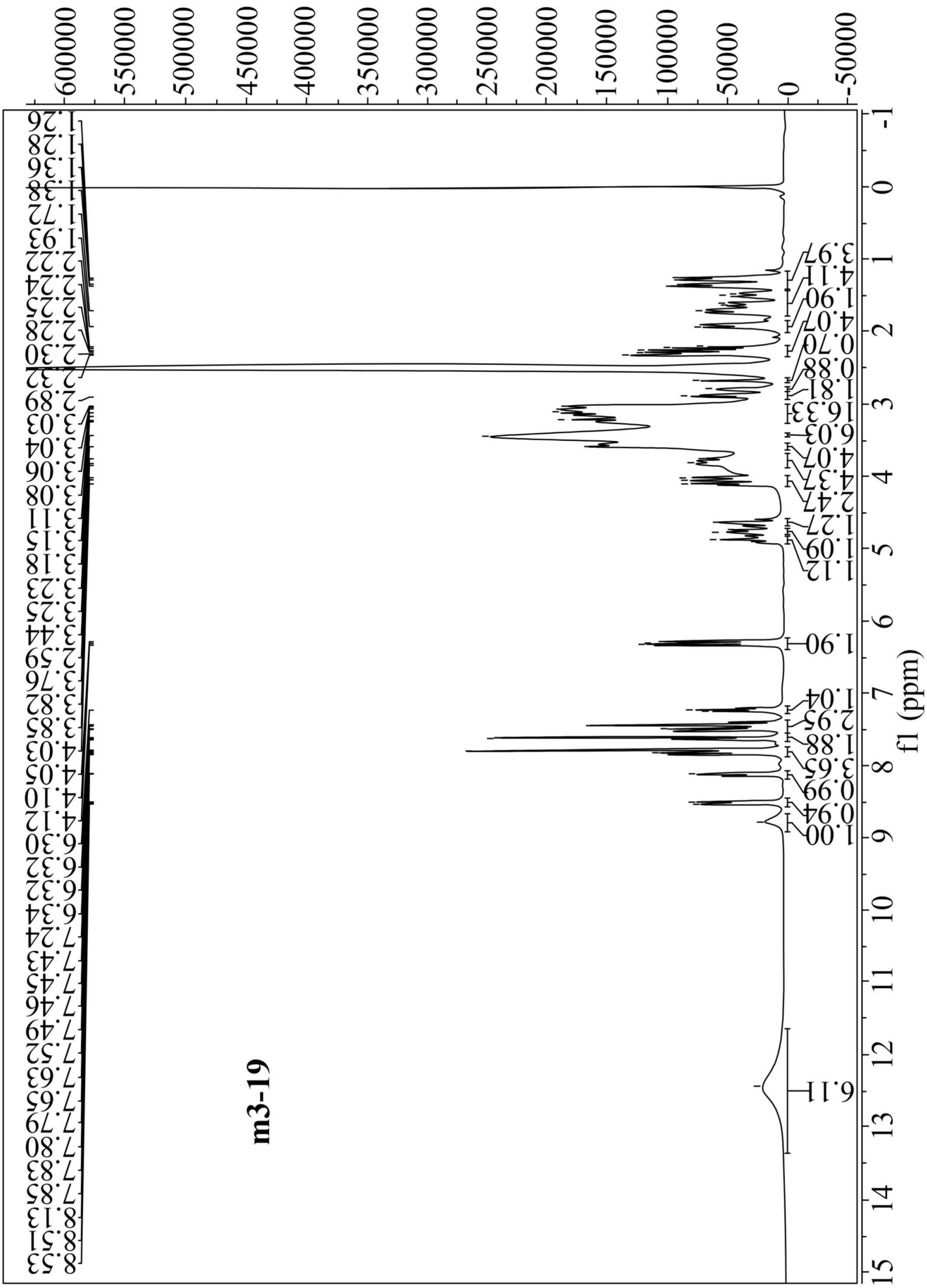


FIG. 9

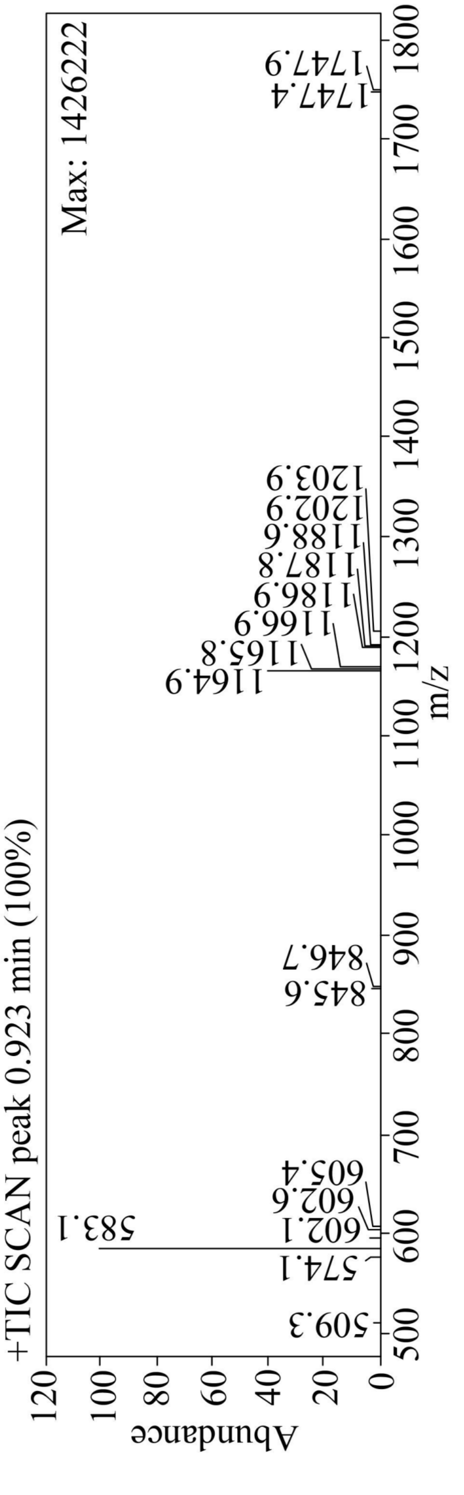
+TIC SCAN peak 0.923 min (100%)
Max: 1426222
Abundance
m/z
509.3
574.1
583.1
602.1
602.6
605.4
845.6
846.7
1164.9
1165.8
1166.9
1186.9
1187.8
1188.6
1202.9
1203.9
1747.4
1747.9

FIG. 10

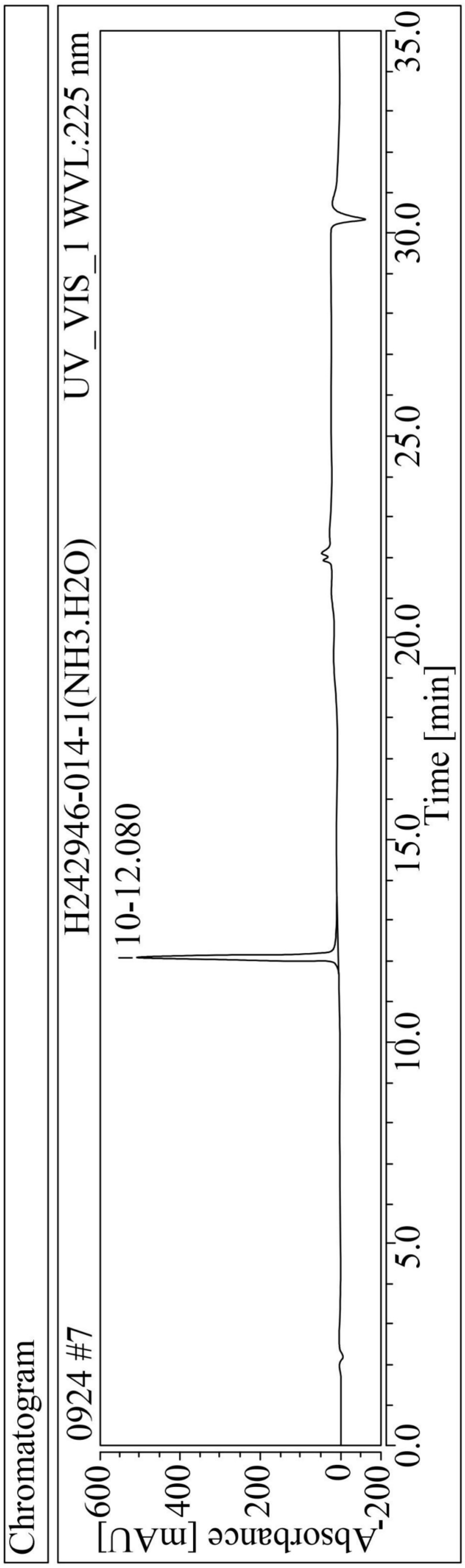
Chromatogram
0924 #7
H242946-014-1(NH3.H2O)
UV_VIS_1 WVL:225 nm
10-12.080
Absorbance [mAU]
600
400
200
0
-200
Time [min]
0.0
5.0
10.0
15.0
20.0
25.0
30.0
35.0

FIG. 11A

| Integration Results | | | | | | | | |
|---|---|---|---|---|---|---|---|---|
| NO. | Peak Name | Retention Time min | Area mAU*min | Height mAU | RelativeArea % | Resolution USP | Plates USP | Asymmetry USP |
| 1 | | 8.358 | 0.177 | 2.918 | 0.28 | 6.79 | 134560 | 1.24 |
| 2 | | 9.288 | 0.019 | 0.134 | 0.03 | 2.57 | 41274 | 1.24 |
| 3 | | 9.590 | 0.002 | 0.052 | 0.00 | n.a. | n.a. | n.a. |
| 4 | | 9.710 | 0.158 | 1.532 | 0.25 | 5.52 | 72093 | 0.79 |
| 5 | | 10.497 | 0.028 | 0.158 | 0.04 | 0.17 | 89477 | 1.68 |
| 6 | | 10.870 | 0.059 | 0.745 | 0.09 | 0.07 | 101 | n.a. |
| 7 | | 11.018 | 2.834 | 27.280 | 4.47 | 1.53 | 72509 | 1.05 |
| 8 | | 11.277 | 0.161 | 1.464 | 0.25 | 1.16 | 66810 | n.a. |
| 9 | | 11.498 | 0.050 | 0.339 | 0.08 | 2.99 | 49213 | n.a. |
| 10 | | 12.080 | 58.359 | 503.831 | 92.03 | 2.64 | 71013 | 1.03 |
| 11 | | 12.700 | 0.267 | 1.481 | 0.42 | 0.63 | 31168 | n.a. |
| 12 | | 12.863 | 0.139 | 1.091 | 0.22 | 4.78 | 51267 | n.a. |
| 13 | | 13.840 | 0.056 | 0.484 | 0.09 | 4.37 | 93535 | 1.21 |
| 14 | | 14.723 | 0.034 | 0.328 | 0.05 | 0.76 | 69570 | n.a. |
| 15 | | 14.973 | 0.303 | 0.903 | 0.48 | 4.17 | 19078 | n.a. |
| 16 | | 16.238 | 0.032 | 0.252 | 0.05 | 7.59 | 140043 | 1.28 |
| 17 | | 17.918 | 0.160 | 0.997 | 0.25 | 9.17 | 70988 | 1.00 |
| 18 | | 20.228 | 0.572 | 3.782 | 0.90 | n.a. | 118528 | 1.09 |
| Total: | | | 63.410 | 547.772 | 100.00 | | | |

**FIG. 11B**

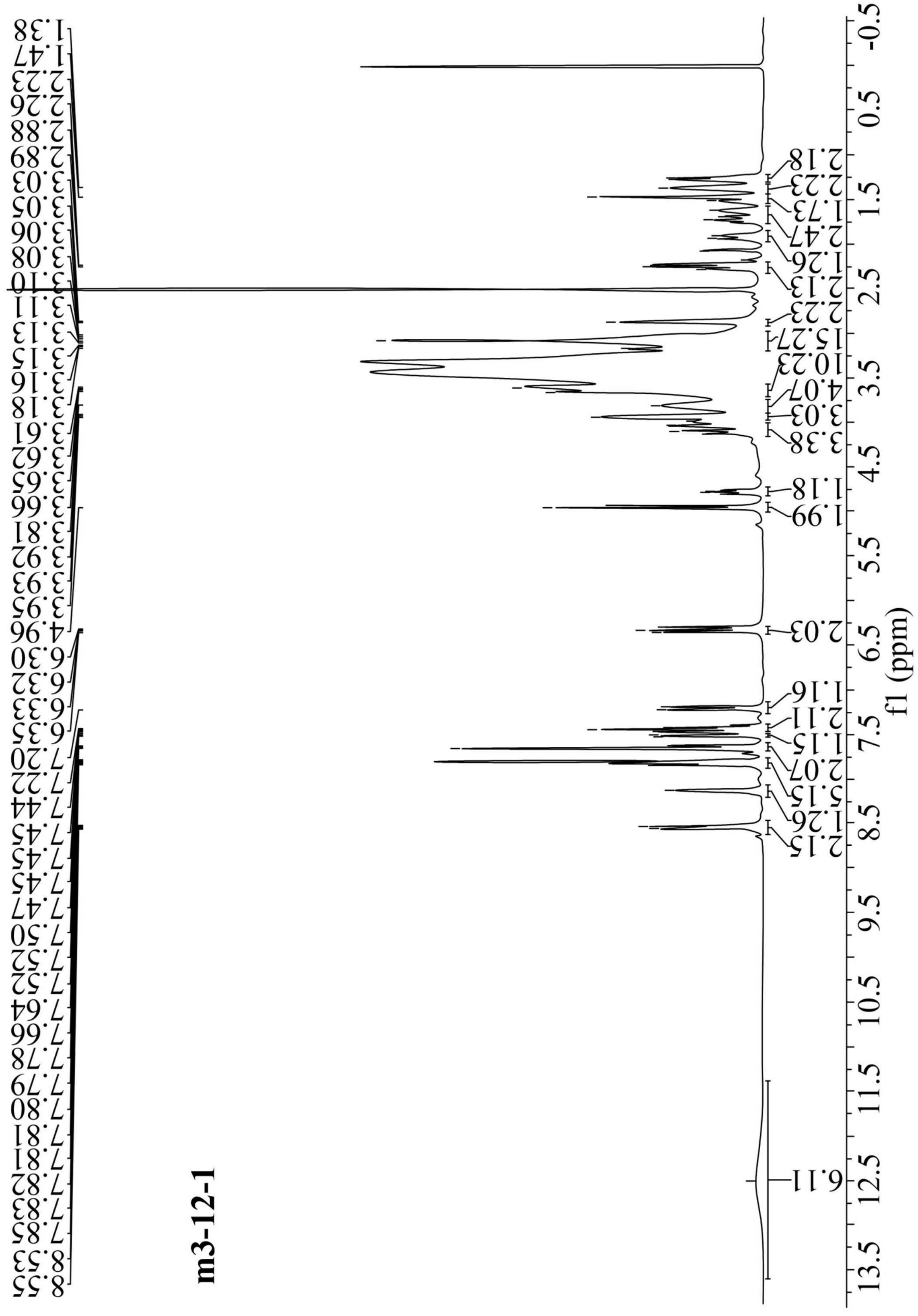
8.55
8.53
7.85
7.83
7.82
7.81
7.81
7.80
7.79
7.78
7.66
7.64
7.52
7.50
7.47
7.45
7.45
7.44
7.22
7.20
6.35
6.33
6.32
6.30
4.96
3.95
3.93
3.92
3.81
3.66
3.65
3.62
3.61
3.18
3.16
3.15
3.13
3.11
3.10
3.08
3.06
3.05
3.03
2.89
2.88
2.26
2.23
1.47
1.38
m3-12-1
6.11
2.15
1.26
5.15
2.07
1.15
2.11
1.16
2.03
1.99
1.18
3.38
3.03
4.07
10.23
15.27
2.23
2.13
1.26
2.47
1.73
2.23
2.18
13.5
12.5
11.5
10.5
9.5
8.5
7.5
6.5
5.5
4.5
3.5
2.5
1.5
0.5
-0.5
f1 (ppm)

FIG. 12

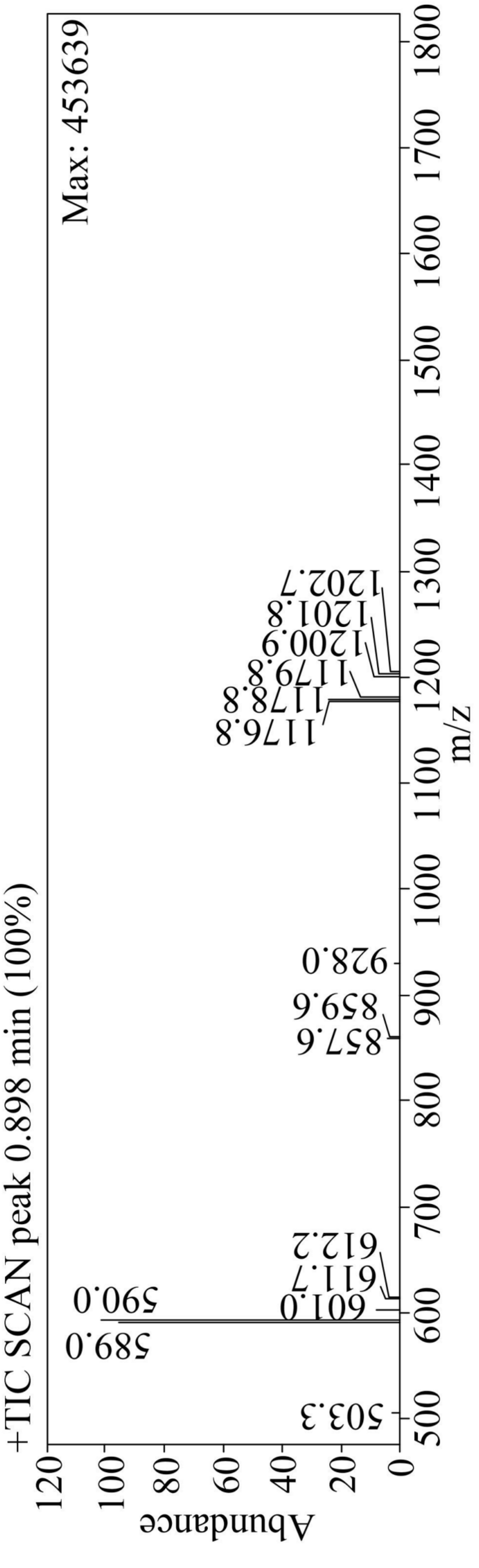
+TIC SCAN peak 0.898 min (100%)
Max: 453639
Abundance
120
100
80
60
40
20
0
m/z
500
600
700
800
900
1000
1100
1200
1300
1400
1500
1600
1700
1800
503.3
589.0
590.0
601.0
611.7
612.2
857.6
859.6
928.0
1176.8
1178.8
1179.8
1200.9
1201.8
1202.7

FIG. 13

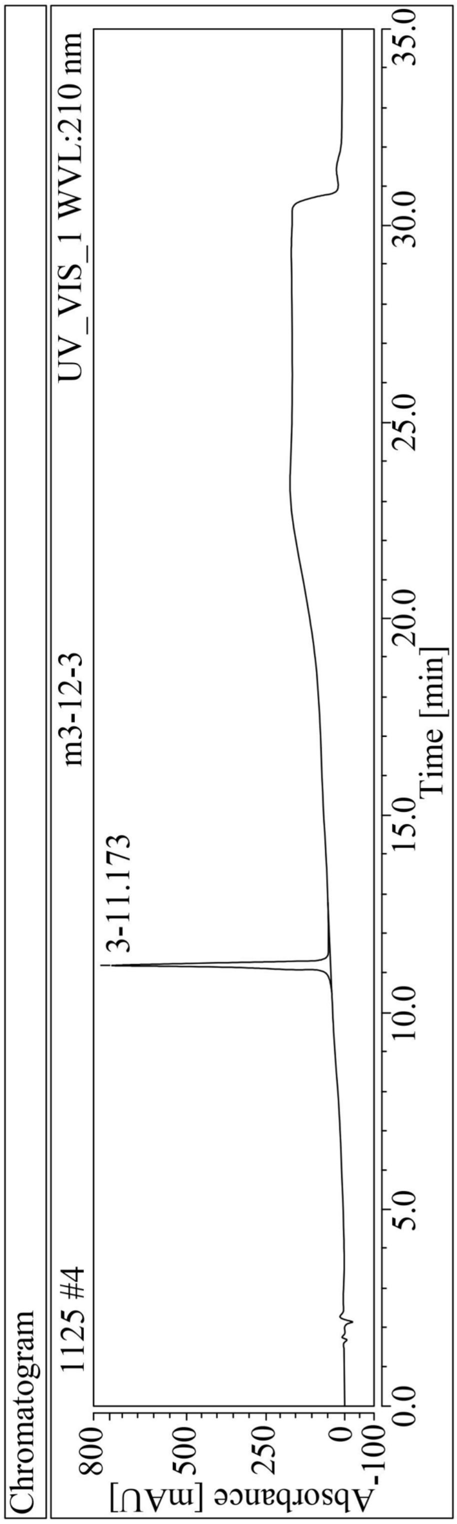

Chromatogram
1125 #4
m3-12-3
UV_VIS_1 WVL:210 nm
3-11.173
Absorbance [mAU]
800
500
250
0
-100
0.0
5.0
10.0
15.0
20.0
25.0
30.0
35.0
Time [min]


FIG. 14A

| Integration Results | | | | | | | | |
|---|---|---|---|---|---|---|---|---|
| NO. | Peak Name | Retention Time min | Area mAU*min | Height mAU | RelativeArea % | Resolution USP | Plates USP | Asymmetry USP |
| 1 | | 10.471 | 0.039 | 0.411 | 0.05 | 2.15 | 71347 | 0.97 |
| 2 | | 10.830 | 0.314 | 2.786 | 0.40 | 1.97 | 60698 | n.a. |
| 3 | | 11.173 | 78.317 | 709.915 | 99.20 | 1.97 | 66938 | 1.01 |
| 4 | | 11.683 | 0.278 | 0.978 | 0.35 | n.a. | 18465 | n.a. |
| Total: | | | 78.949 | 714.090 | 100.00 | | | |

**FIG. 14B**

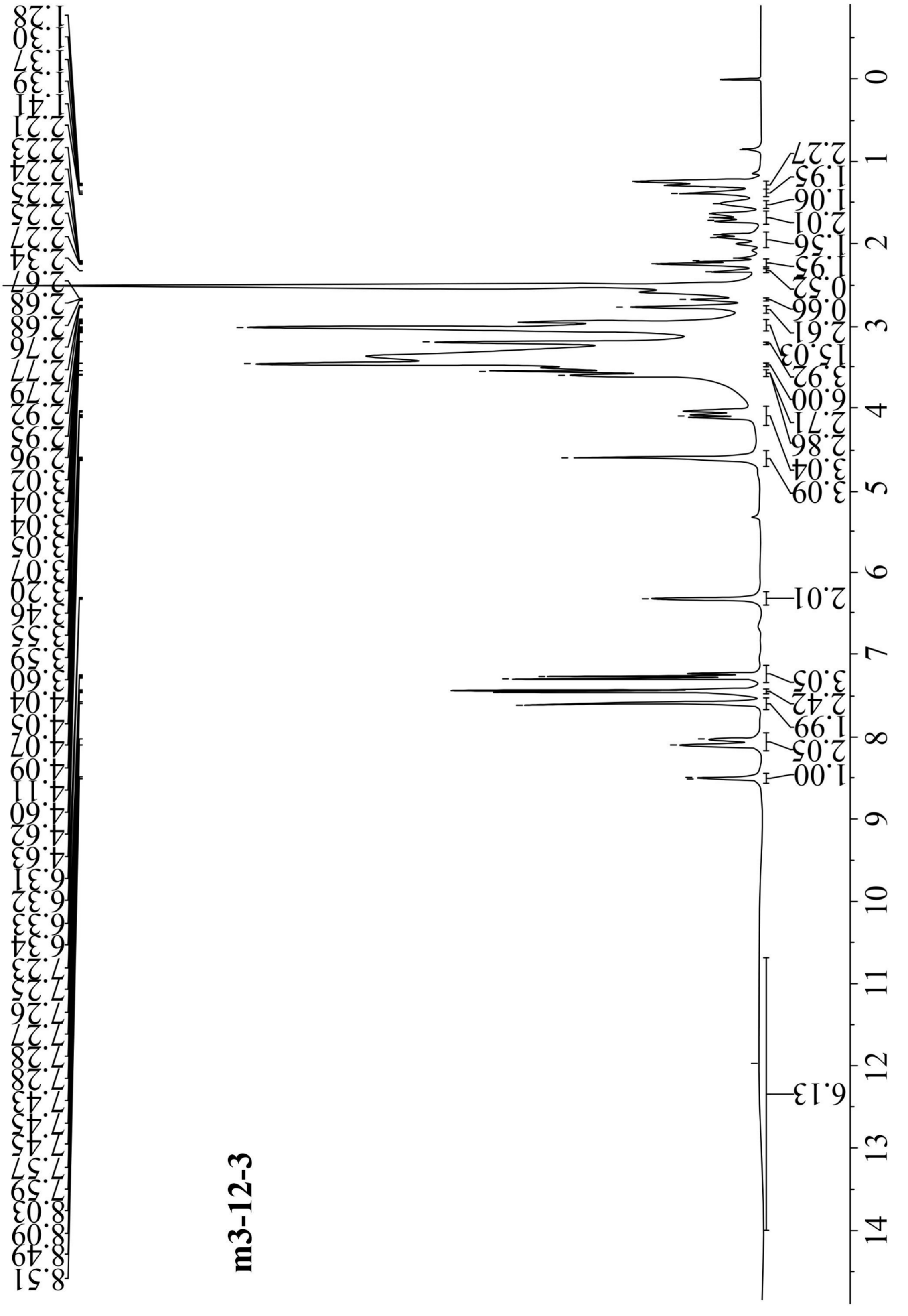


FIG. 15

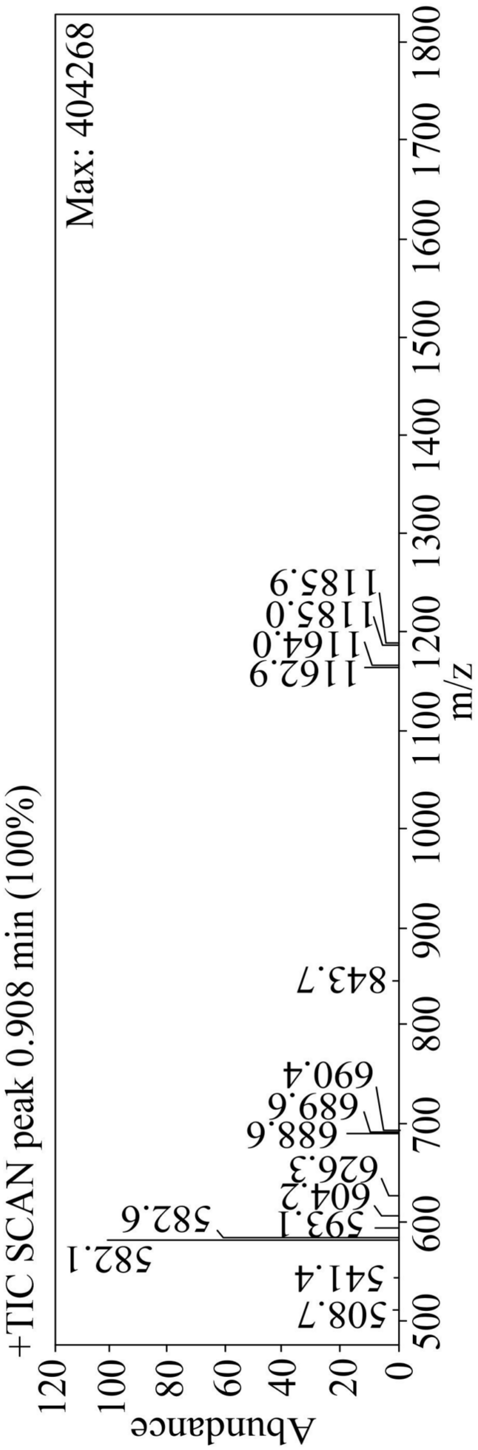
+TIC SCAN peak 0.908 min (100%)
Max: 404268
Abundance
120
100
80
60
40
20
0
500
600
700
800
900
1000
1100
1200
1300
1400
1500
1600
1700
1800
m/z
508.7
541.4
582.1
582.6
593.1
604.2
626.3
688.6
689.6
690.4
843.7
1162.9
1164.0
1185.0
1185.9

FIG. 16

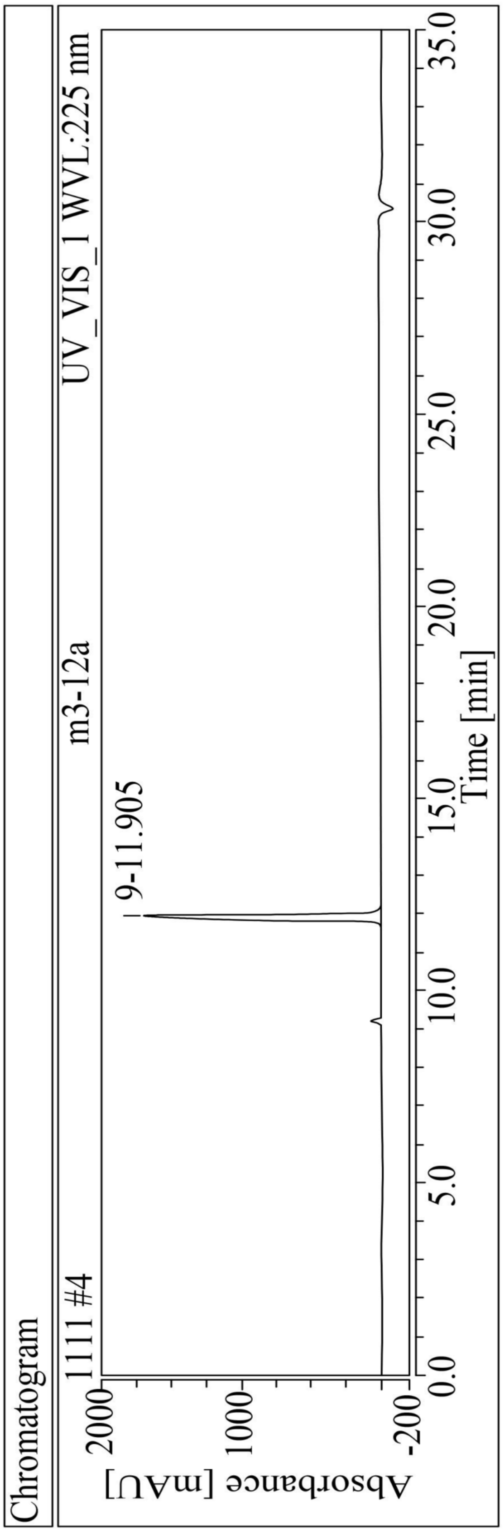
Chromatogram
1111 #4
m3-12a
UV_VIS_1 WVL:225 nm
9-11.905
Absorbance [mAU]
2000
1000
-200
Time [min]
0.0
5.0
10.0
15.0
20.0
25.0
30.0
35.0

FIG. 17A

| Integration Results | | | | | | | | |
|---|---|---|---|---|---|---|---|---|
| NO. | Peak Name | Retention Time min | Area mAU*min | Height mAU | RelativeArea % | Resolution USP | Plates USP | Asymmetry USP |
| 1 | | 4.458 | 0.199 | 3.173 | 0.10 | 46.31 | 43665 | 1.39 |
| 2 | | 9.180 | 5.984 | 77.775 | 2.89 | 4.68 | 95871 | 1.14 |
| 3 | | 9.960 | 0.415 | 4.491 | 0.20 | 0.15 | 34320 | n.a. |
| 4 | | 10.052 | 0.267 | 2.909 | 0.13 | 0.42 | 1650 | n.a. |
| 5 | | 10.347 | 0.594 | 2.047 | 0.29 | 2.14 | 9598 | n.a. |
| 6 | | 11.137 | 0.143 | 1.478 | 0.07 | 0.36 | 19859 | n.a. |
| 7 | | 11.247 | 0.217 | 1.849 | 0.10 | 0.61 | 22366 | n.a. |
| 8 | | 11.405 | 0.384 | 2.518 | 0.19 | 2.50 | 42660 | n.a. |
| 9 | | 11.905 | 198.031 | 1723.475 | 95.65 | 2.32 | 71061 | 1.00 |
| 10 | | 12.380 | 0.432 | 3.087 | 0.21 | 3.26 | 45861 | n.a. |
| 11 | | 13.277 | 0.212 | 0.924 | 0.10 | 0.77 | 27623 | n.a. |
| 12 | | 13.487 | 0.167 | 1.222 | 0.08 | n.a. | 56590 | n.a. |
| Total: | | | 207.045 | 1824.948 | 100.00 | | | |

**FIG. 17B**

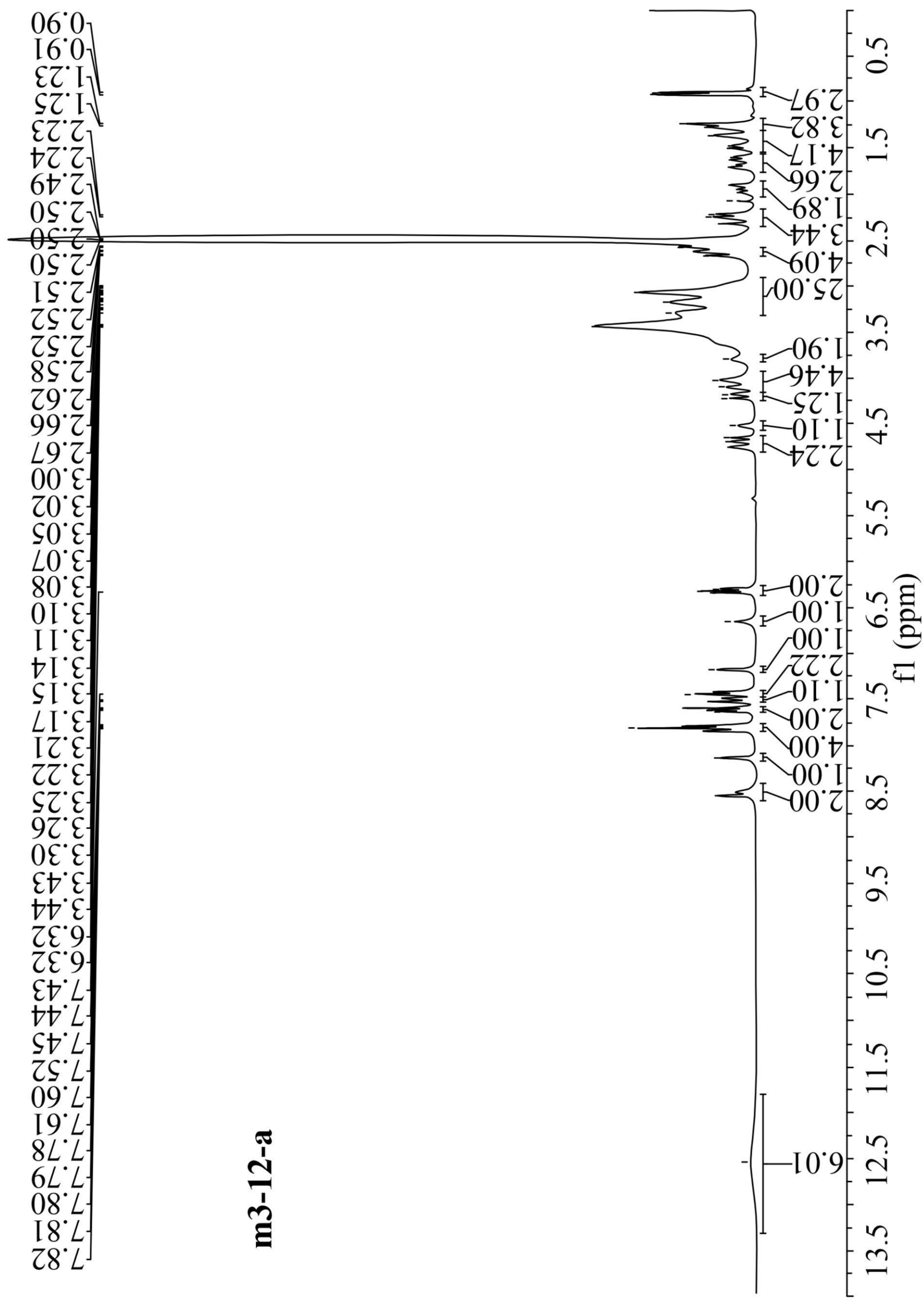

0.90
0.91
1.23
1.25
2.23
2.24
2.49
2.50
2.50
2.50
2.51
2.52
2.52
2.58
2.62
2.66
2.67
3.00
3.02
3.05
3.07
3.08
3.10
3.11
3.14
3.15
3.17
3.21
3.22
3.25
3.26
3.30
3.43
3.44
6.32
6.32
7.43
7.44
7.45
7.52
7.60
7.61
7.78
7.79
7.80
7.81
7.82
2.97
3.82
4.17
2.66
1.89
3.44
4.09
25.00
1.90
4.46
1.25
1.10
2.24
2.00
1.00
1.00
2.22
1.10
2.00
4.00
1.00
2.00
6.01
0.5
1.5
2.5
3.5
4.5
5.5
6.5
7.5
8.5
9.5
10.5
11.5
12.5
13.5
f1 (ppm)


m3-12-a

**FIG. 18**

Europäisches Patentamt
European Patent Office
Office européen des brevets

# EUROPEAN SEARCH REPORT

Application Number
EP 25 22 5667

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 474 379 A1 (BIVISION PHARMACEUTICALS INC [CN]) 11 December 2024 (2024-12-11)<br>* the whole document *<br>* page 59; example 64 *<br>* effect examples 1,3,5;<br>page 59 *<br>* page 65, paragraph 257 *<br>* page 61, paragraphs 231,240 *<br>----- | 1-15 | INV.<br>A61K51/04<br>A61K103/30<br>C07C275/00 |
| X | EP 4 400 505 A1 (BIVISION PHARMACEUTICALS INC [CN]) 17 July 2024 (2024-07-17)<br>* the whole document *<br>* page 64, paragraph 154 *<br>* effect example 5;<br>page 66 *<br>* effect example 9;<br>page 67 *<br>----- | 1-15 | |
| X,P | WO 2025/092841 A1 (VITSGEN THERAPEUTICS INC [CN]) 8 May 2025 (2025-05-08)<br>* the whole document *<br>* claims 1-31 *<br>* page 137, paragraph 836 *<br>* page 131 - paragraph 791 *<br>----- | 1-15 | TECHNICAL FIELDS SEARCHED (IPC)<br>A61K<br>C07C |

The present search report has been drawn up for all claims

| Place of search | Date of completion of the search | Examiner |
|---|---|---|
| The Hague | 19 May 2026 | Sciortino, Flavien |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................................................................
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 25 22 5667

This annex lists the patent family members relating to the patent documents cited in the above-mentioned European search report. The members are as contained in the European Patent Office EDP file on
The European Patent Office is in no way liable for these particulars which are merely given for the purpose of information.

19-05-2026

| Patent document cited in search report | Publication date | Patent family member(s) | Publication date |
|---|---|---|---|
| EP 4474379 A1 | 11-12-2024 | CN 116514735 A | 01-08-2023 |
| | | CN 119930516 A | 06-05-2025 |
| | | EP 4474379 A1 | 11-12-2024 |
| | | JP 2025505467 A | 26-02-2025 |
| | | US 2025108139 A1 | 03-04-2025 |
| | | WO 2023143612 A1 | 03-08-2023 |
| EP 4400505 A1 | 17-07-2024 | CN 115745903 A | 07-03-2023 |
| | | CN 118852043 A | 29-10-2024 |
| | | CN 118852044 A | 29-10-2024 |
| | | EP 4400505 A1 | 17-07-2024 |
| | | JP 2024532521 A | 05-09-2024 |
| | | US 2024366813 A1 | 07-11-2024 |
| | | US 2025319194 A1 | 16-10-2025 |
| | | WO 2023030509 A1 | 09-03-2023 |
| WO 2025092841 A1 | 08-05-2025 | CN 119954773 A | 09-05-2025 |
| | | CN 121909185 A | 21-04-2026 |
| | | WO 2025092841 A1 | 08-05-2025 |

EPO FORM P0459

For more details about this annex : see Official Journal of the European Patent Office, No. 12/82